# EUROPÄISCHE PATENTSCHRIFT

(11) **EP 0 621 901 B1**
(45) Veröffentlichungstag und Bekanntmachung des Hinweises auf die Patenterteilung: **15.04.1998**
(21) Anmeldenummer: 92923795.6
(22) Anmeldetag: 25.11.1992
(51) Int. Cl.: C12Q 1/02, C12N 15/12, C12N 5/10, C12Q 1/66

(54) **VERFAHREN ZUM SCREENEN VON SUBSTANZEN MIT MODULIERENDER WIRKUNG AUF EINEN REZEPTORABHÄNGIGEN ZELLULÄREN SIGNALÜBERTRAGUNGSWEG**
PROCESS FOR SCREENING SUBSTANCES CAPABLE OF MODULATING A RECEPTOR-DEPENDENT CELLULAR SIGNAL TRANSDUCTION PATHWAY
PROCEDE DE TRIAGE DE SUBSTANCES A EFFET MODULATEUR D'UNE VOIE CELLULAIRE DE TRANSMISSION DE SIGNAUX DEPENDANT D'UN RECEPTEUR

(30) Priorität: 25.11.1991 DE 4138621
(43) Veröffentlichungstag der Anmeldung: 02.11.1994
(73) Patentinhaber: BOEHRINGER INGELHEIM INTERNATIONAL GmbH, 55218 Ingelheim am Rhein (DE)
(72) Erfinder: CZERNILOFSKY, Armin, Peter, A-2500 Baden (AT); HIMMLER, Adolf, A-1236 Wien (AT); STRATOWA, Christian, A-1010 Wien (AT); WEYER, Ulrike, A-1070 Wien (AT); LAMCHE, Herbert, A-2534 Alland (AT); SCHÄFER, Renate, A-1140 Wien (AT)
(86) Internationale Anmeldenummer: EP9202718
(87) Internationale Veröffentlichungsnummer: WO9311257

(56) Entgegenhaltungen:
- EP-A- 0 325 849
- WO-A-91/15602
- PROCEEDINGS OF THE NATIONAL ACADEMY OF SCIENCES OF USA. Bd. 88, Nr. 8, 15. April 1991, WASHINGTON US Seiten 3135 - 3139 MONTMAYEUR J.-P. ET AL. in der Anmeldung erw hnt
- MOLECULAR ENDOCRINOLOGY Bd. 5, Nr. 7, Juli 1991, BALTIMORE,USA Seiten 881 - 889 VAN OBBERGHEN-SCHILLING E. ET AL.
- SCIENCE Bd. 250, 5. Oktober 1990, LANCASTER, PA US Seiten 121 - 123 KING K. ET AL.
- CELL. Bd. 56, 10. Februar 1989, CAMBRIDGE, NA US Seiten 487 - 493 ASHKENAZI A. ET AL.

## Beschreibung

Die vorliegende Erfindung bezieht sich auf ein Verfahren zur Bestimmung der modulierenden Wirkung von Substanzen auf einen rezeptorabhängigen Signalübertragungsweg in menschlichen oder tierischen Zellen.

Herkömmliche Tests zum Auffinden pharmakologisch wirksamer Substanzen sind häufig Assays, in denen eine Substanz daraufhin untersucht wird, inwieweit sie einen an einen Rezeptor gebundenen (markierten) Liganden verdrängen kann (Radioligandentests). Mit solchen Tests können nur solche Substanzen identifiziert werden, die die Bindung von bekannten Liganden Rezeptor-Bindungsstellen beeinflussen. Diese Tests erfassen somit nur die Bindung der Substanz, nicht eine funktionelle Antwort der Zelle und können daher nicht unterscheiden, ob die bindende Substanz agonistische oder antagonistische Wirkung hat. Für Radioligandentests werden relativ große Mengen an Rezeptoren benötigt und häufig rezeptorhältige Membranfraktionen verwendet, die aus tierischem Gewebe isoliert werden. Diese Gewebe können aus mehreren Zellarten bestehen, die verschiedene oder auch heterologe Rezeptoren enthalten. Trotz der großen Bedeutung solcher Substrate kann die heterologe Zusammensetzung bzw.- im Falle der Untersuchung von Drogen auf ihre pharmakologische Wirkung im Menschen - der Spezies-Unterschied Tier-Mensch und der sich daraus ergebende Unterschied in den Bindungseigenschaften von Liganden an den menschlichen Rezeptor und an tierische Rezeptoren Probleme bei der Interpretation der Ergebnisse hervorrufen.

Viele Transmembran-Signalübertragungssysteme bestehen aus den folgenden Membran-gebundenen Komponenten: a) einem Zelloberflächenrezeptor; b) einem Guanin-Nukleotid-bindenden und GTP spaltenden regulatorischen Protein, das als G-Protein bezeichnet wird und das sowohl an den Rezeptor als auch an seinen Effektor gekoppelt werden kann; c) einem sog. "Effektor", z.B. einem Ionenkanal oder Adenylatzyklasen, Guanylatzyklasen oder Phospholipasen.

Die sog. G-Protein-gekoppelten Rezeptoren vermitteln die Wirkungen von sehr unterschiedlichen extrazellulären Signalen, wie Licht, Duftstoffen, (Peptid)hormonen, Neurotransmittern, etc.; sie wurden in evolutionär so weit auseinanderliegenden Organismen, wie es Mensch und Hefe sind, identifiziert (Dohlman et al., 1991). Beinahe alle G-Protein-gekoppelten Rezeptoren weisen untereinander Ähnlichkeiten in ihrer Sequenz auf; es wird angenommen, daß allen gemeinsam ein ähnliches topologisches Motiv zugrundeliegt, das aus sieben hydrophoben (möglicherweise α-helikalen) Abschnitten besteht, die die Lipiddoppelschicht durchdringen.

Zelloberflächenrezeptoren erkennen die passenden Liganden aus einer Vielzahl von extrazellulären Stimuli. Durch die Bindung des Liganden an den Rezeptor wird eine Signalkaskade aktiviert, die mit der Aktivierung des heterotrimären G-Proteins beginnt, die Aktivierung des Rezeptors über einen längeren Zeitraum führt bei bestimmten Rezeptoren zur Desensibilisierung, die durch verschiedenartige Modifikationen des Rezeptors hervorgerufen wird. Die Wechselwirkung des G-Proteins mit dem aktivierten Rezeptor bewirkt den Austausch von Guanosindiphosphat (GDP), gebunden an die α-Untereinheit, durch Guanosintriphosphat (GTP), Dissoziation des α-GTP-Komplexes vom β-γ-Heterodimer und Hydrolyse von GTP zu GDP. Ein einziger Rezeptor kann zahlreiche G-Proteinmoleküle aktivieren, wodurch das Liganden-Bindungsereignis verstärkt wird. Die α-Untereinheit, an die GTP gebunden ist, und die freie β-γ-Untereinheit können mit den Effektoren in Wechselwirkung treten, wodurch das Signal noch weiter verstärkt wird, indem sog. "second messengers" gebildet werden. Niedermolekulare Second Messengers, wie cAMP (zyklisches AMP), ausgelöst durch Aktivierung der Adenylatzyklase cGMP (zyklisches GMP), ausgelöst durch Aktivierung der Guanylatzyklase, oder Inositol-1,4,5-Triphosphat (IP₃) und Diacylglyzerine (DAG), ausgelöst durch Aktivierung von Phospholipasen, wie Phospholipase C oder, unter Beteiligung von Hydrolasen, Phospholipase D (Billah et al., 1989), bewirken ihrerseits intrazelluläre Veränderungen. Dazu zählen die selektive Phosphorylierung von Proteinen durch Aktivierung von Proteinkinasen (z.B. PKC durch IP₃/DAG, PKA durch cAMP), die Beeinflussung der Regulation der Transkription bestimmter Gene, die Reorganisation des Zytoskeletts und die Depolarisierung der Membran. (Eine antagonistisch wirksame Substanz kann die durch eine agonistisch wirksame Substanz hervorgerufene Wechselwirkung und die dadurch bewirkte Konzentrationsänderung des Second Messengers ganz oder teilweise aufheben bzw. selbst zu einem reversen funktionellen Effekt führen.) Über dieses Signalübertragungssystem können Zellen miteinander kommunizieren und ihre Entwicklung bzw. die durch sie ausgelösten Wirkungen coordinieren. Die nichtaktivierte Form des G-Proteins wird wiederhergestellt, wenn das an die α-Untereinheit des G-Proteins gebundene GTP zu GDP hydrolysiert wird.

Die spezifischen, mit der Aktivierung von Phospholipasen, deren Reaktionsprodukt ein DAG ist und die außer durch G-Proteine auch durch andere Signalübertragungswege bewirkt wird (Berridge, 1993), bzw. der Adenylatzyklase assoziierten Signalübertragungswege werden im folgenden als "Phospholipase-Signalübertragungsweg" (oder "Phospholipase-Effektorsystem"), bzw. "Adenylatzyklase-Signalübertragungsweg" (oder "Adenylatzyklase-Effektorsystem") bezeichnet.

In Säugetieren wurden bis jetzt ca. hundert verschiedene G-Protein-gekoppelte Rezeptoren gefunden (einige davon binden denselben Liganden). Beispielsweise wurden bisher fünf verschiedene muskarinische Rezeptor-Subtypen, mehr als acht verschiedene adrenergische Rezeptoren, mindestens fünf verschiedene Serotoninrezeptoren und vier verschiedene Opsinrezeptoren identifiziert. Eine wachsende Gruppe von Rezeptoren und Rezeptor-Subtypen, die auf Purine, Bombesin, Bradykinin, Thrombin, Histamin, Dopamin, Ecosinoide, Vasopressin, Peptidhormone wie GHRH ("growth hormone releasing hormone") und Somatostatin ansprechen, wurden kloniert und charakterisiert (Dohlman et al., 1991, Simon et al., 1991; TIPS Receptor Nomenclature Supplement, 1991; Doods und von Meel, 1991; Probst et al., 1992). Unterschiedliche Formen, oder Subtypen, von Rezeptoren, die auf denselben Liganden ansprechen, können auch aufgrund der von ihnen ausgelösten intrazellulären Reaktionen voneinander unterschieden werden. Diese spezifischen Rezeptor-Subtypen können mit verschiedenen Effektorsystemen gekoppelt sein und verschiedene Ionenkanäle regeln. Da ein einziger Rezeptor-Subtyp (möglicherweise in derselben Zelle oder in verschiedenen Zellen) an mehr als einen Effektor gekoppelt sein kann und viele Rezeptor-Subtypen dieselben Effektoren aktivieren können, werden komplizierte Signalübertragungsnetzwerke ausgebildet. Darüberhinaus hat die Charakterisierung der G-Proteine bzw. der Effektoren gezeigt, daß auch sie durch große Genfamilien spezifiziert sind. Es wurden zahlreiche G-Proteine, verschiedene Typen von Adenylatzyklasen und Phospholipasen, wie Phospholipase C und A2, identifiziert. Auch die G-Protein abhängigen Ionenkanäle können in verschiedene Proteinfamilien eingeteilt werden. Welche Kriterien die Spezifität der Wechselwirkung der heterogenen Population der G-Proteine und Effektorproteine bestimmen, auf welche Weise spezifische Rezeptoren mit einer bestimmten G-Protein-Variante verbunden sind, wie sie autonome Kreisläufe bilden, auf welche Weise diese Signal-Kreisläufe miteinander interagieren und wie sie während der Zelldifferenzierung von neuem gebildet werden, ist noch nicht geklärt.

Die derart aktivierten Transkriptionsfaktoren (z.B. CREB-Protein, AP1-Protein) treten in Wechselwirkung mit den regulatorischen DNA-Elementen CRE (CRE-Element, "cAMP responsive element") bzw. TRE (TRE = "TPA responsive element"; TPA = Phorbol-12-Myristat-13-Acetat = Phorbolester), die CREB bzw. AP1 binden: viele Gene, deren Transkription durch cAMP reguliert ist (z.B. Ratten-Somatostatin, Human-α-Gonadotropin), enthalten in der 5'-flankierenden Region eine konservierte Sequenz als regulatorisches Element. Diese Sequenz ist identisch oder ähnlich dem palindromischen Octamer TGACGTCA (Montminy et al., 1990). TRE-Elemente enthalten das sehr ähnliche heptamere Motiv TGAGTCA, das sich von der CRE-Element-Konsensussequenz nur durch ein einziges Nukleotid unterscheidet (Deutsch et al., 1988). Das TRE-Motiv, oder sehr ähnliche Motive, wurde in vielen Genen identifiziert, deren Transkription durch Phorbolester aktiviert wird (Angel et al., 1987a und b; Lee et al., 1987). Umgebende DNA-Sequenzen bzw. Protein-Protein-Wechselwirkungen mit anderen Faktoren bestimmen u.a. die konkreten Regulationsphänomene an einem bestimmten Gen.

Bedingt durch die Komplexität des Netzwerkes der Signalübertragungswege kann es zu sog. "crosstalk" zwischen Signalübertragungswegen, z.B. dem Adenylatzyklase- und dem Phospholipase C-Signalübertragungsweg, kommen. Unter "crosstalk" wird das Phänomen verstanden, daß die Beeinflussung des einen Effektorsystems auch zur Beeinflussung des anderen führt (Sassone-Corsi et al., 1990; Houslay, 1991). Das Phänomen des Crosstalk wird physiologisch zur Signalintegration bzw. Signalvernetzung verwendet, um eine Redundanz der Signale herzustellen bzw. die Kommunikation der verschiedenen Signalübertragungswege zu gewährleisten. Crosstalk kann auf verschiedenen Ebenen des Signalübertragungsweges stattfinden, u.a. auf Ebene der G-Proteine. Beispielsweise kann ein Rezeptor bzw. Rezeptorsubtyp mit mehr als einer G-Protein-Variante wechselwirken, sodaß gegebenenfalls sowohl der cAMP- als auch der IP₃/DAG-Spiegel verändert werden. Eine mögliche Ursache für pathologische Veränderungen der Zelle ist die Störung dieser Wechselwirkungen, z.B. wenn ein bestimmter Rezeptor im physiologischen Sinn nicht korrekt mit einem Effektorsystem wechselwirkt.

Es besteht ein Bedarf an Assays, die es ermöglichen, für die Behandlung von pathologischen Zuständen Drogen zu finden, die spezifisch für einen bestimmten Rezeptor bzw. Rezeptorsubtyp sind und die darüberhinaus spezifisch nur einen bestimmten rezeptorabhängigen Signalübertragungsweg beeinflussen.

Es wurden bereits Assays entwickelt, die sich den Effekt zunutze machen, daß die modulierende Wirkung von Substanzen auf den rezeptorabhängigen Signalübertragungsweg über die Expression von Genen nachgewiesen werden kann:

Die WO 91/15602 beschreibt ein Verfahren zur Bestimmung der agonistischen oder antagonistischen Wirkung einer Substanz auf den nicotinischen Acetylcholinrezeptor, der ein Ionenkanal ist und dessen Aktivierung nicht zur Aktivierung der Adenylatzyklase führt.

Ein von King et al., 1990, beschriebenes Assay-System beruht auf der Beeinflussung des Signalübertragungsweges, der von den G-Protein-gekoppelten Pheromon-Rezeptoren von *Saccharomyces cerevisiae* benutzt wird, wobei in der Hefezelle die Reaktion auf die Bindung einer agonistisch wirksamen Verbindung an einen in die Hefezelle transfizierten Rezeptor kolorimetrisch gemessen wird. Dazu wurde ein modifiziertes β-Adrenorezeptorgen unter der Kontrolle des Galaktose-induzierbaren GAL1-Promotors (zwecks Erzielung hoher Expressionsraten) mit der Säugetier-G-Protein-Untereinheit Gₛα in einem Hefestamm co-transfiziert, der ein Reportergen (β-Galaktosidase) unter der Kontrolle eines auf Pheromon ansprechenden FUS1-Promotors stabil im Genom integriert enthält. Dieses System bietet die Möglichkeit zum Screenen von Substanzen, deren agonistische Wirkung β-Galaktosidase aktiviert, wobei diese Aktivierung in einem einfachen, auf Farbumschlag basierenden, automatisierbaren Assay gemessen werden kann. Dieses System weist jedoch den Nachteil auf, daß die Verwendung eines aus seinem komplexen System isolierten Humanproteins in einer Hefezelle einen direkten Rückschluß auf die Vorgänge in der humanen Zelle nicht ohne weiteres zuläßt. Außerdem erfordert dieses System in Hefezellen die Co-Transfektion einer geeigneten G-Protein-Untereinheit, die mit dem humanen Rezeptor in Wechselwirkung treten kann. Dieses System verwendet nicht das den höheren Zellen eigene Signaltransduktionssystem, die funktionelle Analyse von rezeptorwirksamen Substanzen in diesem System ist daher problematisch.

Von Montmayeur und Borelli, 1991, wurde ein Assay beschrieben, der auf der Beeinflussung des Adenylatzyklase-Signalübertragungsweges durch Aktivierung von G-Protein-gekoppelten Rezeptoren (es wurden D₂-Rezeptoren und der β-adrenergische Rezeptor verwendet) beruht. Dabei werden mit den Rezeptoren Humanzellen transformiert, die als Reportergen das Chloramphenicol-Acetyltransferase-Gen (CAT) unter der Kontrolle eines Thymidin-Kinase(TK)-Promotors enthalten. Dem Promotor ist eine synthetische Oligodesoxynukleotidsequenz vorgeschaltet, die ein auf cAMP ansprechendes Promotorelement ("cAMP responsive element" CRE) enthält. Anhand der CAT-Aktivität konnte gezeigt werden, daß agonistisch wirksame Verbindungen für den β-adrenergischen Rezeptor, von dem bekannt ist, daß er Adenylatzyklase aktiviert, eine dosisabhängige Steigerung der CAT-Aktivität bewirkten. Nach Co-Transfektion mit den Dopaminrezeptoren, die Adenylatzyklase inhibieren, sank diese Aktivität wieder. Dies zeigte, daß die durch cAMP induzierte Expression des Reportergens dosisabhängig positiv und negativ moduliert werden kann.

Dieser Assay ist auf die Messung der Expression von Genen beschränkt, die durch die cAMP-Konzentration reguliert wird; er erlaubt nicht die Messung der IP3/DAG-regulierten Genexpression. Auch Wechselwirkungen zwischen Adenylatzyklase- und Phospholipase-Signalübertragungsweg infolge von Crosstalk können mit diesem Assay nicht bzw. nur partiell erfaßt werden.

Der vorliegenden Erfindung lag die Aufgabe zugrunde, ein Verfahren bereitzustellen, das zum Screenen von Substanzen geeignet ist, die rezeptorabhängig einen Phospholipase-Signalübertagungsweg, insbesondere den Phospholipase C-Signalübertragungsweg modulieren. Dazu zählen Substanzen, die an die Liganden-Bindungsstelle des Rezeptors binden, allosterisch wirkende Substanzen sowie Substanzen, die in bezug auf die Liganden-Bindungsstelle nicht-kompetitiv wirken. Insbesondere sollte mit Hilfe der vorliegenden Erfindung ein Verfahren bereitgestellt werden, das automatisierbar und somit zum Screenen von Substanzen in hohen Durchsatzraten geeignet ist und das auch die Untersuchung von komplexen Substanzgemischen, wie Extrakten von Organismen, auf ihren Gehalt an pharmakologisch wirksamen Substanzen erlaubt.

Die vorliegende Erfindung betrifft somit ein Verfahren zur Bestimmung der modulierenden Wirkung einer Substanz auf einen rezeptorabhängigen Signalübertragungsweg in der menschlichen oder tierischen Zelle. Das Verfahren ist dadurch gekennzeichnet, daß man die modulierende Wirkung der Substanz auf die Aktivität einer Phospholipase oder auf einen im Signalübertragungsweg der Phospholipaseaktivierung vorgeschalteten oder nachgeschalteten Mechanismus, ausgelöst durch einen an den Signalübertagungsweg gekoppelten Rezeptor bestimmt, indem man Säugetierzellen, die
a) transformiert sind mit einer rekombinanten DNA, enthaltend ein Reportergen und eine regulatorische Sequenz, die auf die durch die Modulation der Phospholipase-Aktivität hervorgerufene Konzentrationsänderung von Inositol-1,4,5-Triphosphat (IP₃) und eines Diacylglycerins (DAG) anspricht, so daß die Expression des Reportergens durch eine Konzentrationsänderung von IP₃/DAG moduliert wird, und die weiters
b) transformiert sind mit einer rekombinanten DNA, enthaltend eine für einen Rezeptor, der mit dem Phospholipase-Effektorsystem gekoppelt ist, kodierende Sequenz derart, daß die Zellen den Rezeptor exprimieren,
   mit der zu untersuchenden Substanz inkubiert und die Konzentration des Reportergenproduktes mißt.

Die rekombinante DNA, welche auf Änderung der IP₃/DAG Konzentration - oder im Fall von Kontrollzellen, deren Einsatz im folgenden noch erläutert wird, auf Änderung der cAMP-Konzentration - anspricht, ist ebenfalls Gegenstand der vorliegenden Erfindung. Sie wird im folgenden als "Sensor-DNA" bezeichnet. Ein Reportergen ist dadurch definiert, daß sein Expressionsprodukt detektierbar und quantifizierbar durch Messung eines Signals ist, das seiner Konzentration proportional ist.

In der Sensor-DNA enthaltene regulatorischen Sequenzen, die auf die Konzentrationsänderung von IP₃/DAG ansprechen, enthalten ein oder mehrere heptamere TRE-Motive, im folgenden als "TRE-Element" bezeichnet.

In der Sensor-DNA enthaltene regulatorische Elemente, die auf die Konzentrationsänderung von cAMP ansprechen, enthalten ein oder mehrere oktamere CRE-Motive, im folgenden als "CRE-Elemente" bezeichnet.

Alternativ kann die Sensor-DNA eine Sequenz enthalten, die für ein Protein kodiert, das erst posttranskriptional direkt durch Erhöhung der Kalzium-Konzentration aktiviert wird.
Die rekombinante DNA, die eine für einen Rezeptor kodierende Sequenz enthält, wird im folgenden als "Rezeptor-DNA" bezeichnet.

Die vorliegende Erfindung betrifft in einem weiteren Aspekt Zellen, die nur mit Sensor-DNA transformiert sind. Diese Zellen werden im folgenden als "Prätestzellen" bezeichnet.

Zellen, die mit Sensor-DNA und Rezeptor-DNA transformiert sind, werden im folgenden als "Testzellen" bezeichnet; diese Zellen sind ebenfalls Gegenstand der vorliegenden Erfindung.

Wenn Substanzen im Hinblick auf ihre pharmakologische Wirkung für die Behandlung pathologischer Zustände des Menschen untersucht werden sollen, enthält die Rezeptor-DNA vorzugsweise eine für einen humanen Rezeptor kodierende Sequenz. (Das erfindungsgemäße Verfahren dient bevorzugt zum Auffinden von Substanzen, die für die Behandlung von pathologischen Zuständen des Menschen geeignet sind. Es kann jedoch auch zum Screenen von Substanzen, die für die Behandlung von Tieren zum Einsatz kommen, verwendet werden; in diesem Fall werden die entsprechenden tierischen Rezeptoren verwendet.)

Unter "Substanz" werden im Rahmen der vorliegenden Erfindung sowohl Reinsubstanzen als auch Substanzgemische verstanden.

Unter "modulierender" Wirkung wird ein agonistischer oder antagonistischer Effekt auf einen rezeptorabhängigen Signalübertragungsweg verstanden.

In einer bevorzugten Ausführungsform der vorliegenden Erfindung werden die Zellen mit einer für einen G-Protein-gekoppelten Rezeptor kodierenden DNA transformiert.

Gemäß einer bevorzugten Ausführungsform des erfindungsgemäßen Verfahrens werden zusätzlich Kontrollzellen, die nur mit rekombinanter DNA gemäß Schritt a) transformiert sind (TRE-Prätestzellen), mit der zu untersuchenden Substanz behandelt, um zu überprüfen, ob die Expression des Reportergens auf eine rezeptorabhängige Modulation zurückzuführen ist. Falls die Expression des Reportergens auf eine rezeptorunabhängige bzw. im Fall des Vorhandenseins von endogenen Rezeptoren von diesen Rezeptoren abhängige Modulation des Phospholipase-Effektorsystems zurückzuführen ist, wird diese Modulation mit den TRE-Prätestzellen erfaßt.

Um die Spezifität der zu untersuchenden Substanz auf den Phospholipase-Signalübertragungsweg zu überprüfen, werden zweckmäßig Paralleluntersuchungen mit Prätestzellen durchgeführt, deren Sensor-DNA eine regulatorische Sequenz enthält, die auf die durch die Modulation von Adenylatzyklase hervorgerufene Änderung der cAMP-Konzentration anspricht (CRE-Prätestzellen). Gegebenenfalls werden zusätzlich CRE-Testzellen als Kontrolle verwendet, die mit demselben Rezeptor transformiert sind wie die TRE-Testzellen.

CRE-Testzellen können nicht nur als Kontrollzellen, sondern auch als primäre Substratzellen in einem Screening eingesetzt werden, in dem Substanzen auf ihre rezeptorabhängig modulierende Wirkung auf den Adenylatzyklase-Signalübertragungsweg untersucht werden. In diesem Fall sind die CRE-Testzellen mit Rezeptor-DNA transformiert, die die für einen an das Adenylatzyklase-Effektorsystem gekoppelten Rezeptor kodierende Sequenz enthält. CRE-Prätestzellen werden in einem solchen Screening als Kontrollzellen eingesetzt. Gegebenenfalls dienen TRE-Prätestzellen und gegebenenfalls zusätzlich TRE-Testzellen, transformiert mit dem identischen, an das Adenylatzyklase-Effektorsystem gekoppelten Rezeptor, als weitere Kontrolle.

Zellen, die den interessierenden Rezeptor-Typ nicht endogen exprimieren, sind für die Herstellung von (Prä)Testzellen bevorzugt, weil die endogene Expression des Rezeptors selbst aufgrund der Änderung der Expression des Reportergens ein Signal gibt, das mit dem Meßergebnis interferiert. Die Verwendung von Zellen, die den Rezeptor endogen exprimieren, ist jedoch nicht ausgeschlossen, sofern gewährleistet ist, daß diese Zellen den exogenen Rezeptor stark überexprimieren, sodaß die endogene Expression im Vergleich dazu vernachlässigbar gering ist und das Meßergebnis dadurch nicht beeinträchtigt wird. Um festzustellen, ob sich eine Zelle grundsätzlich als (Prä)testzelle für das erfindungsgemäße Verfahren eignet, z.B. daß sie den speziellen Rezeptor, für den der modulierende Einfluß auf das Phospholipase-Effektorsystem durch die zu untersuchende Substanz bestimmt werden soll, nicht oder nur in geringem Ausmaß exprimiert, kann z.B. so vorgegangen werden, daß eine Säugetierzelle mit TRE-Sensor-DNA transformiert und anschließend mit einer Substanz behandelt wird, von der bekannt ist, daß sie den fraglichen Rezeptor aktiviert. Wenn die Zelle auf diese Behandlung nicht oder nur in geringem Ausmaß anspricht, kann sie für die Verwendung als Testzelle als grundsätzlich geeignet angesehen werden. (Für die Überprüfung von Zellen auf ihre Eignung als CRE-Testzellen werden die Zellen analog getestet mit dem Unterschied, daß CRE-Sensor-DNA verwendet wird.) Eine weitere Möglichkeit festzustellen, ob die Zelle den Rezeptor exprimiert, ist die direkte Messung der Expression durch molekularbiologische Methoden, z.B. durch PCR (Polymerase Chain Reaction) oder Northern Blots.

Vorzugsweise werden die Ausgangszellen für die Herstellung von (Prä)Testzellen danach ausgewählt, daß sie keine oder möglichst wenige der interessierenden Rezeptortypen endogen exprimieren, um sie mit möglichst vielen verschiedenen Rezeptortypen transformieren und damit für die Herstellung möglichst vieler verschiedener Testzellen geeignet zu machen.

Vorzugsweise werden Zellen eingesetzt, die nach Stimulierung mit Substanzen, die die IP₃/DAG-Konzentration erhöhen, starke Expression des TRE-regulierten Reportergens zeigen, bzw. Prätestzellen, die nach Stimulierung mit Substanzen, die den cAMP-Spiegel erhöhen, starke Expression des CRE-regulierten Reportergens zeigen. Um Testzellen zu erhalten, in denen die interessierenden Rezeptoren nach Einwirkung einer Droge effizient an den Phospholipase-Signalübertragungsweg koppeln, werden Säugetierzellen auf ihre Verwendbarkeit als Testzellen untersucht, indem sie zunächst mit Sensor-DNA transformiert werden (Prätestzellen), die ein oder mehrere TRE-Elemente enthalten oder die eine Sequenz enthalten, die für ein Protein kodiert, das durch Kalzium aktivieert wird. Die mit Sensor-DNA transformierte Prätestzelle wird daraufhin einerseits mit Substanzen, die eine Erhöhung der cAMP-Konzentration bewirken oder vortäuschen (z.B. mit Forskolin), andererseits mit Substanzen, die eine Erhöhung der IP₃/DAG-Konzentration bewirken oder vortäuschen (z.B. mit TPA), behandelt. Wenn die Zelle nur bei TPA-, nicht jedoch bei Forskolin-Behandlung ein Signal gibt, erfüllt sie die primäre Voraussetzung, spezifisch auf Änderungen der IP₃/DAG-Konzentration anzusprechen, d.h. es findet in dieser Zelle kein Crosstalk auf Expressionsebene statt (für CRE-Testzellen wird entsprechend verfahren; die CRE-Prätestzelle spricht auf Forskolin an). Weiters wird die Prätestzelle zweckmäßigerweise darauf untersucht, ob eine für den einzuführenden Rezeptor physiologisch "richtige" Kopplung und damit Einleitung des Signalübertragungsweges stattfindet; Voraussetzung dafür ist u.a., daß die Zelle die für den Rezeptor spezifische G-Protein-Variante (bzw. eine G-Protein-Variante, die die Rezeptor-spezifische funktionell ersetzen kann) enthält. Für eine solche Untersuchung wird z.B. die Prätestzelle mit dem fraglichen Rezeptor transformiert, mit bekannten Liganden behandelt und überprüft, ob eine Modulation der Reportergenexpression stattfindet.

Voraussetzung für die Eignung einer Zelle für ihren Einsatz im Rahmen der vorliegenden Erfindung ist weiters ihre Stabilität, und zwar sowohl im Hinblick auf ihre Verwendbarkeit als Prätestzelle (transformiert nur mit Sensor-DNA), als auch als Testzelle (transformiert mit Sensor- und Rezeptor-DNA). Um die Stabilität der Zellen (Lebensfähigkeit, stabile Integration der Fremd-DNA ins Genom) zu testen, werden über einen längeren Zeitraum unter identischen Bedingungen mit den Prätestzellen (Behandlung mit Substanzen, die die Second Messenger-Konzentration beeinflussen) und den Testzellen (Behandlung mit Rezeptor-Liganden) Versuche durchgeführt und die Reproduzierbarkeit der Meßergebnisse überprüft.

Beispiele für geeignete Zellen sind solche der Zellinien CHO ("Chinese hamster ovary"-Zellinie), COS (Affennieren-Zellinie), A549 (humane Lungenkarzinom-Zellinie) und JEG-3 (humane Choriokarzinom-Zellinie).

Die erfindungsgemäßen Prätestzellen dienen einerseits als Ausgangssubstrat für die Herstellung von Rezeptor-DNA enthaltenden Testzellen, andererseits werden sie im erfindungsgemäßen Verfahren als Kontrollzellen eingesetzt, um zu überprüfen, ob ein Signal auf eine rezeptorabhängige Modulation des Signalübertragungsweges durch die zu untersuchende Substanz zurückzuführen ist oder nicht. Erzeugt die Substanz in der Testzelle ein Signal und in der als Kontrollzelle verwendeten Prätestzelle nicht, ist die Modulation der durch das Signal erfaßten Expression des Reportergens ausschließlich rezeptorabhängig. Wenn auch die Kontrollzelle ein Signal gibt, beeinflußt die Substanz (auch) einen Prozeß im Signalübertragungsweg, der rezeptorunabhängig ist; der diesem Signal entsprechende Kontroll-Meßwert muß von dem in der Testzelle erhaltenen abgezogen werden.

Die Sensor-DNA befindet sich vorzugsweise auf einem Plasmid, das sich in einem geeigneten Wirtsorganismus, vorzugsweise E. coli, in hoher Kopienzahl vermehren läßt und nach Transfektion in Säugetierzellen und Integration ins Wirtsgenom die Expression eines Reportergens unter Kontrolle von regulatorischen Elementen ermöglicht. Es handelt sich dabei bevorzugt um einen Shuttle-Vektor, der eine Expressionskassette für das Reportergen (Sensor DNA) und einen selektionierbaren Marker für Säugetierzellen sowie mindestens einen Replikationsursprung und einen Marker für die Vermehrung und Selektion in E.coli enthält.

Zur Herstellung von permanenten Zellinien, die die Sensor-DNA stabil in ihr Genom integriert enthalten, enthält der Vektor einen dominanten Selektionsmarker. Die Verwendung eines bestimmten Selektionsmarkers ist nicht kritisch, geeignet sind z.B. das Gen für Neomycin-Phosphotransferase (neo), das eine Resistenz gegenüber dem Antibiotikum Geneticin (G-418) verleiht (Southern und Berg, 1982), das DHFR-Gen (Dihydrofolatreduktase) für DHFR-defiziente Zellen, das Gen für Xanthin-Guanin-Phosphoribosyltransferase (gpt), das Resistenz gegen Mycophenolsäure verleiht (Mulligan und Berg, 1981) oder das Hygromycin-B-Phosphotransferase-Gen (hph; Gritz und Davies, 1983). Beispiele für Promotoren, die das Selektionsmarker-Gen treiben, sind der SV40-Early-Promotor, der Cytomegalovirus-Promotor (CMV-Promotor), der Promotor des Thymidin-Kinase-Gens des Herpes simplex-Virus (TK-Promotor), der Rous Sarcoma Virus (RSV) long terminal repeat (LTR). Die Plasmide werden vorzugsweise derart konstruiert, daß einzelne wichtige Elemente, wie das Reportergen, der Promotor für das Reportergen, die regulatorischen Sequenzen für den Selektionsmarker, einfach ausgetauscht oder verändert werden können, um etwaigen geänderten Anforderungen, die sich aus dem speziellen Anwendungsfall ergeben, z.B. aufgrund der Verwendung einer anderen Zellinie, entsprechen zu können. Derartige Maßnahmen bestehen z.B. darin, vor den/die Promotor(en) oder vor das Reportergen Multiklonierstellen einzubauen, um die Klonierung von regulatorischen, den Promotor modulierenden Sequenzen bzw. von verschiedenen Reportergenen zu ermöglichen.

Bei der Auswahl eines geeigneten Reportergens wurde davon ausgegangen, einen, vorzugsweise nicht-radioaktiven, automatisierbaren Assay mit hoher Empfindlichkeit bereitzustellen.

Im Rahmen der vorliegenden Erfindung können grundsätzlich sämtliche Reportergene eingesetzt werden, die diese Voraussetzungen erfüllen:

Alkalische Phosphatase kann bei Verwendung eines chemilumineszierenden Substrats mit hoher Empfindlichkeit gemessen werden, weist allerdings den Nachteil auf, daß viele Säugetierzellen dieses Enzym relativ stark exprimieren. Es kommt daher als Reportergen im allgemeinen nur für solche Zellinien in Betracht, die es nicht oder nur schwach exprimieren.

Die Expressionsprodukte des β-Galaktosidase- und des β-Glucuronidase-Gens können das entsprechende Methylumbeliferyl-Galaktosid bzw. -Glucuronid unter Bildung fluoreszierender Gruppen spalten. Diese Enzymreaktionen werden mit Hilfe etablierter Fluoreszenz-Assays verfolgt (Wieland et al., 1985; Kricka, 1988).

Die Expression von Chloramphenicol-Actetyltransferase (CAT) ist zwar relativ empfindlich nachweisbar, der Assay weist jedoch u.a. den Nachteil auf, daß er radioaktiv und schwer automatisierbar ist (Hartmann, 1991).

Bevorzugt wird im Rahmen der vorliegenden Erfindung als Reportergen das für Photinus pyralis-Luciferase kodierende Gen (De Wet et al., 1987) verwendet. Dieses Enzym weist die Vorteile auf, daß es mit seinem Substrat Luciferin unter Zugabe von ATP Bioluminiszenz in hoher Ausbeute erzeugt, die mit Hilfe etablierter, automatisierbarer Methoden gemessen werden kann, und daß dieses Enzym von Säugetierzellen nicht endogen produziert wird. Weiters hat Luciferase eine relativ kurze *in vivo* Halbwertszeit und ist auch in hohen Konzentrationen nicht toxisch (Hartmann, 1991; Brasier et al., 1989). Die Messung der Aktivität der Firefly-Luciferase mittels Biolumineszenz stellt eine der empfindlichsten Methoden einer Enzymbestimmung dar. Daher und aufgrund des Fehlens von Luciferaseaktivität in normalen Säugetierzellen ist dieses Enzym als Reportergen in Studien zur Genregulation besonders geeignet (Subramani und DeLuca, 1987).

Ein Nachteil bei der Messung der Luciferase-Aktivität ist die geringe Stabilität des Lichtsignals unter Reaktionsbedingungen, die zur Erzielung einer maximalen Lichtausbeute optimal sind (DeLuca et al., 1979). Dies bedingt, daß die Messung der Luciferase-Aktivität am besten in Meßgeräten erfolgt, in denen die Messung des entstehenden Lichtes direkt nach Zugabe der Substratlösung mit den für die Lumineszenzreaktion erforderlichen Komponenten erfolgt. Ein weiteres Problem bei der Bestimmung der Luciferase-Aktivität in Reportergenstudien stellt die Lysierung der Zellen zur Freisetzung des Enzyms dar, wodurch ein weiterer Arbeitsschritt erforderlich wird (Brasier et al., 1989).

In dem erfindungsgemäßen Verfahren wird bevorzugt ein Reagens verwendet, das eine direkte Messung der in Zellkulturen exprimierten Luciferase-Aktivität in einem einzigen Schritt ermöglicht. Dieses Reagens lysiert einerseits mittels eines Detergens die Zellen und enthält andererseits die für die Luciferasereaktion notwendigen Substrate. Durch dieses Reagens wird durch dessen Gehalt an ausgewählten Substanzen ein besonders konstantes Lichtsignal erhalten, wodurch die Messung der Luciferaseaktivität in einem Zeitraum zwischen 2 und 20 Minuten nach Zugabe des Reagens ermöglicht wird. Weiters ist dieses Reagens im fertigen Zustand mindestens eine Woche stabil. Das Reagens besteht aus einem Grundpuffer, der geeignete Puffersubstanzen wie z.B.: Tricin, HEPES, Glycylglycin, Phosphat, Tris, bevorzugt Tricin (Leach und Webster, 1986) enthält. Der pH dieses Puffers liegt im Bereich zwischen 6 und 9, bevorzugt zwischen 7 und 8. Weiters wird dem Puffer ein Magnesiumsalz, bevorzugt Magnesiumsulfat-Heptahydrat (MgS04.7H20) in einer Konzentration zwischen 10 und
0.1 g/l, bevorzugt 4 g/l zugesetzt. Die erforderlichen Substrate für die Luciferasereaktion sind bevorzugt in folgenden Konzentrationen enthalten:
Adenosintriphosphat (ATP) von 0.05 bis 5 g/l, bevorzugt 0.7 g/l; Luciferin von 0.001 bis 0.1 g/l, bevorzugt 0.015 g/l. Der Puffer kann zusätzlich einen Komplexbildner wie Ethylendinitrilotetraessigsäure Dinatriumsalz (EDTA) in einer Menge von ca. 0.2 g/l enthalten.

Der bevorzugte Grundpuffer (plus Substrate für die Luciferase-Reaktion) setzt sich zusammen aus 25 mmol/l Tricin, 0.5 mmol/l EDTA, 16.3 mmol/l MgSO₄.7H₂O, 1.2 mmol/l ATP und 0.05 mmol/l Luciferin Na-Salz. Zur Stabilisierung der Luciferase wird ein mildes organisches Reduktionsmittel wie Dithiothreitol (DTT), β-Mercaptoethanol (BME), allein oder in Mischung mit anderen Reduktionsmitteln verwendet. Ein solches Reduktionsmittel verhindert die Oxidation von im Enzym vorhandenen SH-Gruppen und eine damit verbundene Desaktivierung der Luciferase während der LumineszenzReaktion. DTT wird in einer Konzentration zwischen 0.1 und 50 g/l, vorzugsweise von 1 g/l verwendet. BME wird in einer Konzentration zwischen 0.1 und 50 ml/l, vorzugsweise 4 ml/l zugesetzt.

Zur Stabilisierung und Verstärkung der Lumineszenz wird gegebenenfalls zusätzlich Natriumtripolyphosphat (NaTPP) verwendet, welches in einer Konzentration zwischen 0.005 und 5 g/l, vorzugsweise 0.2 g/l zugesetzt wird. Statt Natriumtripolyphosphat kann auch Natriumpyrophosphat verwendet werden.

Zur Lysierung der Zellen wird ein geeignetes Detergens wie z.B. Triton X-100, Tween 20 oder 80, NP 40, Brij oder ähnliches verwendet. Triton X-100 wird in einer Konzentration zwischen 0.01 und 5 %, vorzugsweise 0.1 % verwendet.

Alternativ kann als Reportergen das für das Enzym Apoaequorin der Qualle Aequoria victoria kodierende Gen (Tanahashi et al., 1990) verwendet werden. Dieses Enzym weist die Vorteile auf, daß es mit seinem Co-Faktor Coelenterazin nach Bindung von Kalziumionen Bioluminiszenz in hoher Ausbeute erzeugt, die mit Hilfe etablierter, automatisierbarer Methoden gemessen werden kann. Ein weiterer Vorteil ist, daß dieses Enzym von Säugetierzellen nicht endogen exprimiert wird.

Bei der Konstruktion der Sensor-DNA wird das Reportergen unter die Kontrolle von konstitutiven, vorzugsweise schwachen, Promotor-Elementen gestellt, die durch ein oder mehrere vorgeschaltete TRE- bzw. CRE-Regulationselemente modulierbar sind. Die am besten geeignete Sensor-DNA-Konstruktion wird ermittelt, indem die Zelle transient mit verschiedenen Sensor-DNA-Plasmid-Konstrukten transformiert wird, wobei einerseits hinsichtlich des Reportergens, andererseits hinsichtlich der Kontrollsequenzen variiert werden kann, und die Messung des Reportergen-Produktes auf seine Empfindlichkeit untersucht wird. Dem Fachmann sind die für die Expression in bestimmten Säugetierzellen geeigneten Kontrollsequenzen bekannt; die Auswahl kann zunächst aufgrund der einschlägigen Literatur getroffen werden (z.B. Landschulz et al., 1988, Turner und Tjian, 1989), eine Einengung bzw. Optimierung kann mit den oben angeführten, einfach durchzuführenden transienten Transfektionsexperimenten durchgeführt werden. Beispiele für geeignete Promotoren sind der β-Globin-Promotor und der TK-Promotor. Gegebenenfalls werden bekannte natürliche oder synthetische Promotoren modifiziert, z.B. durch Verkürzung auf die für die Promotorfunktion erforderliche Minimalsequenz. Gegebenenfalls wird die Regulationssequenz eines durch cAMP bzw. IP₃/DAG induzierbaren Gens verwendet, die Promotor und regulatorisches Element enthält (Montminy et. al., 1990, Deutsch et al. 1988), z.B. die 5'-regulatorische Sequenz des ICAM-1-Gens. Im Falle der Verwendung des Apoaequoringens als Reportergen wird das Gen zweckmäßig unter die Kontrolle eines starken konstitutiven Promotors gestellt.

Die Auswahl der in der Sensor DNA enthaltenen regulatorischen Sequenz (CRE- bzw. TRE-Element einschließlich seiner flankierenden Sequenzen) erfolgt im allgemeinen empirisch, wobei von literaturbekannten Elementen (s. z.B. Montminy et al., 1990, Deutsch et al., 1988) ausgegangen wird, die in Vorversuchen auf ihre Eignung im Hinblick auf eine empfindlich nachweisbare Induzierbarkeit des Reportergens in einem gegebenen Zellsystem untersucht werden. Beispiele für geeignete regulatorische Elemente, einschließlich deren flankierende Sequenzen, sind die Sequenzen von Somatostatin, "vasoactive intestinal peptide", Cytomegalovirus Enhancer, Rinder Leukämievirus-Long Terminal Repeat (BLV LTR) (CRE-Elemente) bzw. ICAM-1, Kollagenase, Parathyroidhormon (TRE-Elemente). Falls die in den natürlichen Sequenzen enthaltenen TRE-bzw. CRE-Motive keine perfekte Konsensussequenz aufweisen, können sie, und gegebenenfalls auch die benachbarten Sequenzen, durch Austausch eines oder mehrerer Nukleotide verändert werden.

Die regulatorischen Elemente (TRE- bzw. CRE-Elemente) und die sie flankierenden Sequenzen können synthetisch hergestellt werden oder natürlichen Ursprungs sein. Wenn eine natürliche Sequenz verwendet werden soll, von der bekannt ist, daß sie in Abhängigkeit vom Zelltyp auf cAMP und/oder IP₃/DAG anspricht (Karin; 1989), wird sie in der Prätestzelle darauf untersucht, auf welchen Second Messenger sie anspricht, indem die Zellen z.B. mit TPA und Forskolin behandelt werden.

Gegebenenfalls enthält die Sensor-DNA ein oder mehrere CRE-Elemente neben einem oder mehreren TRE-Elementen. Mit einer solchen Sensor-DNA werden sowohl die Aktivierung des einen oder des anderen Signalübertragungsweges einzeln oder auch die parallele Aktivierung beider Signalübertragungswege erfaßt.

Um den modulierenden Effekt von IP₃/DAG und/oder cAMP auf die Reportergenexpression zu verstärken, wird gegebenenfalls ein Konstrukt verwendet, das mehrere regulatorische CRE- und/oder TRE-Sequenzen in Tandem enthält. Vorzugsweise enthält die regulatorische Sequenz drei bis zwölf TRE- und/oder CRE-Elemente. Bei der Anordnung der Einzelelemente des Konstrukts wird der Abstand der TRE- und/oder CRE-Elemente zueinander so gewählt, daß die Bindung des Transkriptionsfaktors an die CRE- bzw. TRE-Elemente gewährleistet ist. Der optimale Abstand der regulatorischen TRE- bzw. CRE-Elemente zueinander, bei dessen Bestimmung auch Überlegungen bezüglich der sterischen Anordnung zum Tragen kommen, wird in Vorversuchen empirisch ermittelt, gegebenenfalls auch der Abstand zu anderen regulatorischen-DNA-Elementen, die einen Einfluß auf die Transkription haben, z.B. von der TATA-Box. Die TRE- bzw. CRE-Elemente und/oder die flankierenden Sequenzen können identisch oder, zumindest teilweise, verschieden sein, letztere Ausführungsform wird für die Tandem-Konstruktion bevorzugt.

Als das CRE- bzw. TRE-Element flankierende Sequenzen, von denen festgestellt wurde, daß sie ebenfalls Einfluß auf die Regulationseigenschaften der CRE- bzw. TRE-Elemente haben, werden bevorzugt, insbesondere in deren unmittelbarer Umgebung, die das spezielle regulatorische Element natürlicherweise umgebenden Sequenzen verwendet (Montminy et al., 1990, Deutsch et al., 1988). Die Sequenz bzw. deren Anordnung wird empirisch ermittelt.

Gegebenenfalls befinden sich die Elemente der Sensor-DNA und das zur Selektion benutzte Markergen auf zwei getrennten Plasmiden, wovon eines das Reportergenkonstrukt (einschließlich der Expressionskontrollsequenz, die die regulatorische Sequenz enthält) und eines das Selektionsmarkergenkonstrukt enthält. (Beispiele für geeignete Selektionsmarkergenkonstrukte sind die Plasmide pRSVneo, pSV2neo, pRSVgpt, pSV2gpt, deren Aufbau einschlägigen Handbüchern, z.B. "Cloning Vectors", entnommen werden kann.) Im Fall der Verwendung von getrennten Plasmiden werden die Zellen mit den beiden Plasmiden co-transfiziert und auf den Marker selektioniert. Das Vorhandensein des Selektionsmarkers läßt den Schluß zu, daß die Zelle auch das Reportergenkonstrukt enthält, weil bekannt ist, daß Co-Transformation zweier Gene, die sich auf physikalisch nicht miteinander verbundenen DNA-Segmenten befinden, häufig zur Expression beider co-transformierter Gene führt (Winnacker, 1985).

Im Hinblick auf die im Testverfahren einzusetzende Meßanordnung ist es zweckmäßig, das Verhältnis zwischen maximaler Änderung und Normalwert des Meßsignals zu optimieren, vorzugsweise durch Veränderung der Konstruktion der Sensor-DNA, z.B. durch strukturelle Veränderung der Promotoranordnung. Das Background-Signal ist bevorzugt niedrig genug, um eine Induktion der Reportergenexpression mit hoher Empfindlichkeit zu erfassen, andererseits aber hoch genug, um die Nachweisgrenze im Hinblick auf die Negativkontrolle bestimmen zu können.

Für das Rezeptor-DNA-Konstrukt gelten grundsätzlich dieselben Überlegungen wie für das Sensor-DNA-Konstrukt mit dem Unterschied, daß die Rezeptor-Sequenz bevorzugt unter die Kontrolle eines starken, konstitutiven Promotors gestellt wird. Gegebenenfalls befinden sich auch im Falle der Rezeptor-DNA die für den Rezeptor kodierende Sequenz und der dominante Selektionsmarker auf zwei getrennten Plasmiden, mit denen die Zellen co-transformiert werden.

Die Transfektion der Zellen mit Sensor- bzw. Rezeptor-DNA wird mit üblichen Transfektionsmethoden vorgenommen (vgl. z.B. Potter et al. 1984; Felgner et al., 1987), bevorzugt werden die Elektroporations-, die Calciumphosphat-Präzipitations- oder die Lipofektionsmethode eingesetzt.

Im allgemeinen werden die Zellen zuerst mit Sensor-DNA transformiert, um Prätestzellen zu erhalten, im Anschluß daran wird die Prätestzelle mit Rezeptor-DNA transformiert, um die Testzelle herzustellen.

Die Konstruktion der Sensor-DNA wird bevorzugt im Hinblick auf maximale Induzierbarkeit durch ein einziges spezifisches Effektorsystem der Prätest- bzw. der Testzelle optimiert, wobei minimale Induzierbarkeit durch andere Effektorsysteme angestrebt wird. Die TRE-Testzelle spricht somit vorzugsweise spezifisch auf Substanzen an, die den Phospholipase C-Signalübertragungsweg rezeptorabhängig modulieren. (Um eine rezeptorunabhängige Beeinflussung durch die zu untersuchende Substanz auszuschließen, wird, wie bereits angeführt, die entsprechende TRE-Prätestzelle parallel mit der Substanz behandelt. Um eine Aussage über die Spezifität der Substanz auf das Phospholipase C-Effektorsystem zu erlauben, wird gegebenenfalls als Negativ-Kontrolle eine Messung durchgeführt, bei der die Prätestzelle mit CRE-Sensor-DNA und die davon abgeleitete Testzelle zusätzlich mit dem zu untersuchenden identischen Rezeptor transformiert ist.)

Zur Kontrolle der Spezifität der Beeinflussung des Phospholipase C-Signalübertragungsweges bezüglich des untersuchten Rezeptors (bzw. Rezeptor-Subtyps) durch die aufgefundene Substanz werden zweckmäßigerweise für weitere Kontrollversuche TRE-Prätestzellen mit verschiedenen anderen Rezeptoren transformiert und mit der Substanz behandelt. Wenn eine Substanz nur einen Rezeptor spezifisch beeinflussen darf, was im Hinblick auf die Spezifität einer Droge im allgemeinen der Fall ist, darf die Substanz nur diesen einen Rezeptor modulieren.

Als Rezeptoren für die Transfektion von TRE-Prätestzellen, um TRE-Testzellen zu erhalten, sind sämtliche Rezeptoren geeignet, die mit dem Phospholipase C-Signalübertragungsweg koppeln können. Dazu zählen: α-Adrenoceptor(Adrenalin)-Rezeptoren vom α₁-Typ, Angiotensin II-Rezeptoren, Atrionatriuretischer Peptid-Rezeptor, Bombesin-Rezeptoren , Bradykinin-Rezeptoren, Cholecystokinin- und Gastrin-Rezeptoren, Endothelin-Rezeptoren, metabotrophische excitatorische Aminosäure-Rezeptoren, Histamin-Rezeptor (H₁), Serotonin-Rezeptoren (5-HT_{1C}, 5-HT₂), Leukotrien-Rezeptoren (LTB₄, LTD₄), muskarinische Acetylcholin-Rezeptoren (M₁, M₃, M₅), Neuropeptid Y-Rezeptoren (auch PYY, NPP), Neurotensin-Rezeptor, PAF (Platelet Activating Factor)-Rezeptor, Prostanoid-Rezeptoren (EP₁₋₃, FP, TP), P₂-Purinoceptor (P₂gamma), Neurokinin-Rezeptoren (NK_{1,2,3}), Vasopressin- und Oxytocin-Rezeptoren (V_{1A}, V_{1B}, OT), Thrombin-Rezeptor, etc. Manche dieser Rezeptoren können auch an andere Effektoren, wie Adenylatzyklase, koppeln. Die angeführten und weitere geeignete Rezeptoren sowie das Effektorsystem, an das die Rezeptoren gekoppelt sind, sind der Fachliteratur entnehmbar; eine Übersicht findet sich im TiPS Receptor Nomenclature Supplement, 1991.

Beispiele für nicht-G-Protein-gekoppelte Rezeptoren, die Phospholipase-C aktivieren und die daher im Rahmen der vorliegenden Erfindung zur Transfektion der Substratzellen verwendet werden können, sind Mitglieder der Familien der FGF-Rezeptoren, Insulin-Rezeptoren, PDGF-Rezeptoren, EGF-Rezeptoren, etc. (Ullrich und Schlessinger, 1990).

Rezeptoren, die an das Adenylatzyklase-Effektorsystem koppeln können und die zur Transfektion von CRE-Prätestzellen verwendet werden können, um CRE-Testzellen zu erhalten, sind ebenfalls dem TiPS Receptor Nomenclature Supplement, 1991, entnehmbar.

Daß von G-Proteinen auch andere Signalübertragungswege als der Phospholipase-Weg aktiviert werden, wurde auch von Spiegel, 1992, beschrieben. Ferner ist bekannt, daß das G-Protein G₀, das Phospholipasen aktiviert (Sternweis und Smrcka, 1992) unter anderem auch als Inhibitor der Adenylatzyklase wirkt (Hepler und Gilman, 1992).

Beispielhaft seien die Rezeptoren für Adenosin (A1, A2), für Adrenalin (β- und α₂-Typ), für Dopamin (D1, D₂₁ (= D_{2A}), D₂ₛ (= D_{2B}), D₅) für Histidin (H₂-Typ), für Serotonin (5-HT_{1A}- und 5-HT_{1D}-Typ, 5-HT₄), für Acetylcholin (M₂- bzw. M₄-Typ) und für Enkephaline genannt.

(Sofern ein Rezeptor eingesetzt werden soll, der noch nicht kloniert wurde bzw. von dem die cDNA nicht in entsprechenden Vektoren zur Verfügung steht, kann die Rezeptor-DNA, z.B. durch Screenen von cDNA- oder genomischen Banken, erhalten und kloniert werden.)

Für den Fall, daß ein Rezeptor negativ an Adenylatzyklase gekoppelt ist (z.B. Acetylcholinrezeptoren vom M₂- bzw. M₄-Typ, Neuropeptid Y-Rezeptor), d.h. eine Aktivierung des Rezeptors eine Senkung der cAMP-Konzentration bewirkt, wird die Senkung der cAMP-Konzentration zweckmäßigerweise wie folgt gemessen: die Zellen werden mit der zu untersuchenden Substanz behandelt; falls es sich um eine agonistisch wirkende Substanz handelt, wird der Rezeptor aktiviert, was eine Senkung des cAMP-Spiegels zur Folge hat. Gleichzeitig bzw. gegebenenfalls anschließend daran wird die Zelle mit einer Substanz behandelt, von der bekannt ist, daß sie die cAMP-Konzentration erhöht. Es kann auch umgekehrt zuerst die cAMP-Konzentration erhöht und dann mit den interessierenden Substanzen inkubiert werden. (Die Erhöhung des cAMP-Spiegels kann direkt erfolgen, z.B. mit Forskolin, oder indirekt, indem die Zellen mit einer Substanz behandelt werden, die agonistische Wirkung auf einen Rezeptor hat, der mit Adenylatzyklase positiv gekoppelt ist. Bei diesem Rezeptor handelt es sich entweder um einen endogenen Rezeptor oder um einen Rezeptor, mit dem die Zelle co-transformiert wurde.)

Als Kontrolle wird in einem parallelen Testansatz, bei identischen Inkubationsbedingungen, nur die Erhöhung der cAMP-Konzentration bestimmt. Der Unterschied in den Signalwerten entspricht der Senkung der cAMP-Konzentration, die auf die Wirkung der Substanz zurückzuführen ist; sie ist ein Maß für die rezeptorabhängige Senkung der Adenylatzyklaseaktivität. Die indirekte Messung der Rezeptoraktivierung bei negativer Adenylatzyklase-Kopplung ist erforderlich, wenn die natürlicherweise in der Zelle vorhandene cAMP-Konzentration sehr niedrig und daher eine Senkung dieser Konzentration meßtechnisch nicht erfaßbar ist.

Rezeptoren, durch deren Aktivierung neben dem Phospholipase C-Signalübertragungsweg, u.a. aufgrund von Crosstalk, das Adenylatzyklase-Effektorsystem moduliert wird, können im Rahmen der vorliegenden Erfindung ebenfalls verwendet werden. Wenn die Sensor-DNA nur auf die Konzentrationsänderung von IP₃/DAG anspricht (TRE-Sensor-DNA), wird ein Signal nur dann erzeugt, wenn ein Transkriptionsfaktor aktiviert wird, der an das TRE-Element bindet. Es spielt dabei keine Rolle, wenn parallel auch das Adenylatzyklase-Effektorsystem aktiviert wird und das Signal oder ein Anteil davon aufgrund eines Crosstalks erzeugt worden ist.

Nach Transformation der Zellen mit Rezeptor-DNA werden die positiven Klone, z.B. mit Hilfe von Bindungsassays, in denen bekannte radioaktiv markierte Agonisten und Antagonisten eingesetzt werden, auf die Expression des Rezeptors untersucht.

Mit Hilfe von Scatchard-Blots (Human Pharmacology, 1991) kann die Rezeptorzahl in Molekülen pro Zelle bestimmt werden.

Bevorzugt wird aus den stabilen, Rezeptor-DNA enthaltenden Transformanden ein Klon mit einer Rezeptorzahl gewählt, die der physiologischen Rezeptor-Konzentration möglichst entspricht. (Ist die Rezeptorzahl zu hoch, kann der Fall eintreten, daß unvollständige und möglicherweise unspezifische Kopplung stattfindet oder daß zusätzlich zur spezifischen Kopplung eine unspezifische Kopplung stattfindet, wodurch gegebenenfalls andere Effektorsysteme mitaktiviert werden. Ist die Rezeptorzahl zu niedrig, ist das Signal gegebenenfalls zu niedrig, um durch die Messung erfaßt zu werden.)

Ein Rezeptor bzw. Rezeptorsubtyp kann in zwei verschiedene Prätestzellen transfektiert werden, wobei eine davon auf die IP3/DAG-Konzentration, die andere auf die cAMP-Konzentration anspricht. (Die parallel verwendete Zelle wird mit der für das andere Effektorsystem, z.B. das Adenylatzyklase-Effektorsystem, spezifischen Sensor-DNA daraufhin untersucht, ob sie spezifisch über den cAMP-Signalübertragungsweg aktiviert wird, indem sie getrennt mit Substanzen behandelt wird, die die cAMP-bzw. die IP3/DAG Konzentration erhöhen oder eine solche Konzentrationserhöhung vortäuschen, z.B mit Forskolin und TPA. Für diese Vorversuche können die Zellen entweder nur mit Sensor-DNA transformiert sein, wobei eine stabile Transformation nicht erforderlich ist, oder auch mit der Rezeptor-DNA co-transformiert sein; in letzerem Fall dürfen sich im Medium keine Substanzen befinden, die den Rezeptor aktivieren. Nachdem festgestellt wurde, daß die Zellen nur auf Forskolin ansprechen, wird der mit TRE/Rezeptor-transformierten Zellen vorgenommene Assay unter denselben Bedingungen mit CRE-Testzellen wiederholt.) Durch Vergleich der Daten, die mit einer bestimmten Substanz und einem bestimmten Rezeptor(subtyp) in den spezifischen TRE- und in den spezifischen CRE-Zellen sowie in einer Testzelle, die auf IP3/DAG und auf cAMP anspricht, erhalten wurden, kann festgestellt werden, in welchem Ausmaß das Signal auf Crosstalk und in welchem Ausmaß es auf Beeinflussung von nur einem der beiden Effektorsysteme zurückzuführen ist.

Bei den mit Hilfe des erfindungsgemäßen Verfahrens auf ihre potentielle pharmakologische Wirkung zu untersuchenden Substanzen handelt es sich um natürliche oder synthetische Substanzen, wobei Reinsubstanzen oder Substanzgemische (z.B. Pflanzenextrakte, Fermentationsbrühen, etc.) eingesetzt werden können. Bei den Reinsubstanzen sind insbesondere niedermolekulare synthetische organische Verbindungen von Interesse. Zweckmäßig werden die Substanzen in seriellen Verdünnungen auf die Zellen aufgebracht, um einen möglichst großen Konzentrationsbereich zu erfassen. Die Inkubationszeit wird empirisch bestimmt, indem z.B. die gegebenen Testzellen mit bekannten Rezeptor-Agonisten behandelt werden und der Zeitpunkt bestimmt wird, ab dem die Induktion der Reportergenexpression reproduzierbar gemessen werden kann. Die Inkubationszeit wird im allgemeinen auf diesen Zeitpunkt abgestellt, sie beträgt im allgemeinen mindestens 1 h. Die absolute Zahl der Testzellen ist nicht kritisch. Die Zellzahl richtet sich vor allem nach der Nachweisgrenze aes Meßsignals und nach dem Wachstumsstadium, in dem sich die Zellen befinden, die Untergrenze wird außerdem durch die technisch bedingte Fähigkeit definiert, die Zellen gleichmäßig auf die Testeinheiten zu verteilen. Die Zellzahl beträgt im Fall der Verwendung von Microtiterplatten mit 96 Vertiefungen z.B. ca. 10.000 bis ca. 200.000 Zellen pro Testeinheit, kann jedoch auch bei entsprechender Empfindlichkeit des Meßsignals und exakter Verteilbarkeit der Zellen geringer sein. Das Wachstumsstadium, in dem die Zellen eingesetzt werden, hängt von den zelltypspezifischen Eigenschaften der Ausgangszelle ab, weiters wird es vor allem durch den jeweiligen Rezeptor bestimmt (bei verschiedenen Rezeptoren kann dasselbe Effektorsystem je nach Wachstumsstadium unterschiedlich bzw. unterschiedlich stark aktiviert werden); Wachstumsstadium und Zellzahl werden somit ebenfalls in Vorversuchen empirisch ermittelt, indem die Kinetik der Reportergenexpression in Prätest- und Testzellen verschiedenen Wachstumsstadiums bestimmt wird.

Im Rahmen der vorliegenden Erfindung konnte gezeigt werden, daß verschiedene mit TRE-Sensor-DNA transformierte Zellen (TRE-Prätestzellen) auf den Zusatz von Substanzen, von denen bekannt ist, daß sie DAG vortäuschen (womit dieser Zusatz für die Zelle gleichbedeutend ist einer durch Phospholipase C-Aktivierung hervorgerufenen Erhöhung des DAG-Spiegels) ansprechen, wodurch die Expression des Reportergens induziert wird, während sie auf cAMP-erhöhende Substanzen nicht ansprechen. (Die Sensor-DNA enthielt als regulatorisches Element die 1.3 kb 5'-flankierende Region des humanen ICAM-1-Gens (Interzelluläres Adhäsionsmolekül 1), die ein TRE-Element enthält, oder mehrere in Tandem angeordnete TRE-Elemente des ICAM-1-Gens). Wenn die TRE-Prätestzellen mit einem Rezeptor transformiert wurden, der spezifisch an den Phospholipase C-Signalübertragungsweg gekoppelt ist (es wurden der humane 5-HT₂-Rezeptor, der Neurokinin2-Rezeptor und der humane M₃-Rezeptor verwendet), wurde nach Behandlung mit Rezeptor-Agonisten die Expression des Luciferasegens gemessen; die durch die Agonisten vermittelte Induktion wurde durch Zugabe von Antagonisten wieder aufgehoben. Andererseits konnte dieser Effekt nicht beobachtet werden, wenn die Phospholipase C-gekoppelten Rezeptoren in Prätestzellen transformiert wurden, die auf cAMP ansprechen.

Im Rahmen der vorliegenden Erfindung konnte weiters gezeigt werden, daß in mit CRE-Sensor-DNA transformierten CHO-Zellen (CRE-Prätestzellen) die Expression des CRE-regulierten Reportergens nur durch Substanzen erhöht wird, die die Konzentration von cAMP erhöhen. Die Behandlung der CRE-Prätestzellen mit Substanzen, die eine Erhöhung der IP₃/DAG-Konzentration bewirken oder vortäuschen, ergab keine Induktion der Luciferase-Expression, ebensowenig die Behandlung mit Substanzen, die Agonisten für Dopamin- und muskarinische Acetylcholinrezeptoren sind. Letzteres Ergebnis zeigte, daß die Zellen diese Rezeptoren nicht endogen exprimieren. Wenn die CRE-Prätestzellen mit einem positiv an das Adenylatzyklase-Effektorsystem gekoppelten Rezeptor (es wurde der Dopamin-D₁-Rezeptor verwendet) transfektiert wurden, konnte die LuciferaseExpression mit Agonisten für den D₁-Rezeptor induziert und diese Induktion durch Antagonisten wieder aufgehoben werden.

Mit Hilfe der vorliegenden Erfindung wird ein empfindliches und vielseitiges funktionelles Verfahren zum Auffinden von Substanzen bereitgestellt, die rezeptorabhängig einen Signalübertragunsweg in der Zelle spezifisch beeinflussen. Die mit Hilfe des erfindungsgemäßen Verfahrens ermittelten Substanzen dienen als Leitsubstanzen für die Entwicklung von Medikamenten für die Behandlung von Erkrankungen, die mit einer Fehlfunktion eines Signalübertragungsweges assoziiert sind, und können in weiterer Folge, z.B. in einem sekundären Screening mit Primärzellen und erst im Anschluß daran im Tierversuch, näher auf ihre pharmakologischen Eigenschaften untersucht werden. Die Anzahl der benötigten Tiere wird somit durch den Einsatz des erfindungsgemäßen Verfahrens beträchtlich verringert.

Das erfindungsgemäße Verfahren bietet außerdem den Vorteil, daß es automatisierbar ist, indem die Beschickung der Zellkulturgefäße, z.B. Mikrotiterplatten mit 96 Vertiefungen, die Beschickung mit den Testsubstanzlösungen, die Inkubations- und Waschschritte sowie die Messung, z.B. im Fall von Luciferase als Reportergenprodukt mit einem Luminometer, mit Robotern durchgeführt werden. Das erfindungsgemäße Verfahren eignet sich somit für Screening-Programme mit hoher Durchsatz-Kapazität, wobei beispielsweise ca. 2000 Substanzen bzw. Substanzgemische pro Woche getestet werden können.

Mit Hilfe des erfindungsgemäßen Verfahrens ist es möglich, allosterisch wirkende Substanzen und bezüglich der Ligandenbindungsstelle nicht-kompetitiv wirkende Substanzen erfassen zu könnnen.

Ein Vorteil dieses Systems besteht weiters darin, daß für den Fall, daß für einen bestimmten mehrstufigen rezeptorabhängigen intrazellulären Signalübertragungsweg mehrere Möglichkeiten für das Eingreifen einer Substanz bestehen, die Aussichten steigen, die günstigsten Parameter für die Modulation eines bestimmten Signalübertragungsweges ermitteln zu können. Die Vielseitigkeit des Systems bezüglich der großen Zahl einsetzbarer Rezeptoren und Rezeptor-Subtypen ermöglicht seinen Einsatz zum Auffinden von pharmakologisch wirksamen Substanzen für unterschiedliche Indikationsgebiete. Das erfindungsgemäße System ermöglicht es außerdem, bei gezielter Auswahl bestimmter Rezeptoren und Rezeptor-Subtypen mit hoher Spezifität zwischen Schlüssel-Mechanismen in verschiedenen Zellsystemen, z.B. dem Zentralnervensystem und dem peripheren System zu unterscheiden.

Mit Hilfe des erfindungsgemäßen Verfahrens ist es weiters möglich, Rezeptoren zu klonieren, die pharmakologisch oder biochemisch charakterisiert und für die Liganden bekannt sind. Dabei wird von cDNA- oder genomischen Banken ausgegangen, von denen Pools in die entsprechende Prätestzellinie transformiert werden. Die Expression des Rezeptors wird durch eine Expression des Reportergens angezeigt, nachdem der Rezeptor durch Bindung eines Liganden aktiviert wurde.

### Figurenübersicht:

- Fig. 1:: Konstruktion des Plasmids pADneo
- Fig. 2:: BglII - BamHI-Fragment nach PCR für die Konstruktion von Plasmid pADneoTK
- Fig. 3:: Konstuktion des Plasmids pADneoTK
- Fig. 4A:: Plasmid pADneoTKluci
- Fig. 4B:: Plasmid pADneoBGluci
- Fig. 5:: Konstruktion des Plasmids pRc/RSVΔNaeI
- Fig. 6:: Konstruktion des Plasmids pRc/RSVneo
- Fig. 7:: Konstruktion des Plasmids pADneo2BGluci
- Fig. 8:: Oligonukleotidsequenzen, enthaltend drei CRE-Elemente
- Fig. 9:: Konstruktion des Plasmids pADneo2-6C-BGL, enthaltend sechs CRE-Elemente
- Fig. 10:: Plasmid pHEBo
- Fig. 11:: Plasmid p290
- Fig. 12:: Plasmid pAHygCMV1
- Fig. 13:: Plasmid pAHygCMV2
- Fig. 14:: Plasmid pBHluc1.3
- Fig. 15:: Plasmid pADneo(1.3ICAM)luci, enthaltend die 5'-regulatorische Sequenz des ICAM-1-Gens
- Fig. 16:: Plasmid pADneo(3TRE)BGluci, enthaltend drei TRE-Elemente des ICAM-1-Gens
- Fig. 17:: Plasmid pAD-CMV2-5HT₂, enthaltend die für den 5HT₂-Rezeptor kodierende Sequenz
- Fig. 18:: Plasmid pAD-CMV2:D1, enthaltend die für den Dopamin-D1-Rezeptor kodierende Sequenz
- Fig. 19A:: Plasmid pAHyg-NK2, enthaltend die für den NK2-Rezeptor kodierende Sequenz
- Fig. 19B:: Plasmid pAHyg-5HT2, enthaltend die für den 5HT2-Rezeptor kodierende Sequenz
- Fig. 20:: Induktion von TRE-Sensor-DNA (pBHluc1.3) durch TPA in den Zellinien A549, HeLa und COS-7
- Fig. 21A:: Induktion von TRE-Sensor-DNA (pBHluc1.3) durch TPA, nicht aber durch Forskolin, in HeLa-Zellen
- Fig. 21B:: Induktion von TRE-Sensor-DNA (pBHluc1.3) durch TPA, nicht aber durch Forskolin, in COS 7-Zellen
- Fig. 22:: Induktion von TRE-Sensor-DNA (pADneo(3TRE)BGluci) durch TPA, nicht jedoch durch Forskolin in den Zellinien A549, HeLa und COS 7
- Fig. 23A:: Induktion von TRE-Sensor-DNA, in der drei bzw. sechs TRE-Elemente in unterschiedlichem Abstand zueinander enthalten sind, durch TPA in COS 7-Zellen
- Fig. 23B:: Induktion von TRE-Sensor-DNA, in der drei bzw. sechs TRE-Elemente in unterschiedlichem Abstand zueinander enthalten sind, durch TPA in A549-Zellen
- Fig. 24:: Induktion von TRE-Sensor-DNA (pADneo(3TRE)BGluci) durch Bindung einer agonistisch wirkenden Substanz an den muskarinischen M3-Rezeptor in COS 7-Zellen
- Fig. 25:: A) Induktion von TRE-Sensor-DNA (pBHluc1.3) durch Bindung einer agonistisch wirkenden Substanz an den 5HT₂-Rezeptor in COS 7-Zellen, transformiert mit 5HT₂-Rezeptor-DNA
B) keine Induktion von CRE-Sensor-DNA (pADneo2-C6-BGL) durch eine für den 5HT₂-Rezeptor agonistisch wirkende Substanz in COS 7-Zellen, transformiert mit 5HT₂-Rezeptor-DNA
- Fig. 26:: A) Induktion von TRE-Sensor-DNA (pBHluc1.3) durch TPA, nicht jedoch durch eine für den Dopamin-D1-Rezeptor agonistisch wirkende Substanz in COS 7-Zellen, transformiert mit Dopamin-D1-Rezeptor-DNA
B) Induktion von CRE-Sensor-DNA (pADneo2-C6-BGL) durch Forskolin und durch Bindung einer agonistisch wirkenden Substanz an den Dopamin-D1-Rezeptor in COS 7-Zellen, transformiert mit Dopamin-D1-Rezeptor-DNA
- Fig. 27:: Induktion der Luciferaseexpression in der TRE-Prä-Testzellinie A20 und in der den humanen Neurokinin2-Rezeptor exprimierenden TRE-Testzellinie A20/NK2-122
- Fig. 28:: Kinetik der Luciferaseinduktion mittels eines NK2-spezifischen Agonisten
- Fig. 29A:: Dosis-Wirkungskurven der Luciferaseaktivität in Abhängigkeit von der Aktivierung des humanen Neurokinin2-Rezeptors durch Neurokinine
- Fig. 29B:: Dosis-Wirkungskurven der Luciferaseaktivität in Abhängigkeit von der Aktivierung des humanen Neurokinin2-Rezeptors durch Agonisten für einen Neurokinin-Rezeptor
- Fig. 30:: Inhibierung der agonistischen Wirkung von Neurokinin A durch NK2-spezifische Antagonisten
- Fig. 31:: Kinetik der durch Serotonin induzierten Luciferaseexpression in einer den 5HT2-Rezeptor exprimierenden Testzellinie
- Fig. 32A:: Dosis-Wirkungskurven der Luciferaseaktivität in Abhängigkeit von der Aktivierung des humanen 5HT2-Rezeptors durch Agonisten
- Fig. 32B:: Dosis-Wirkungskurven der Luciferaseaktivität in Abhängigkeit von der Aktivierung des humanen 5HT2-Rezeptors durch Antagonisten
- Fig. 33:: Behandlung von CRE-Prätestzellen (CHO-Zellen, stabil transformiert mit pADneo2-C6-BGL) mit verschiedenen Substanzen, die die cAMP- oder die IP₃/DAG-Konzentration ändern oder eine Konzentrationsänderung vortäuschen, oder mit rezeptorspezifischen Agonisten
- Fig. 34:: Dosis-Wirkungskurve der transkriptionellen Aktivierung des Luciferasegens in CRE-Prätestzellen durch Forskolin
- Fig. 35A:: Kinetik der Luciferase-Induktion in CHO-Zellen in Abhängigkeit der Forskolindosis
- Fig. 35B:: Kinetik der Luciferase-Induktion in CHO-Zellen in Abhängigkeit der Forskolindosis (ED₅₀-Bestimmung)
- Fig. 36:: Behandlung von CHO-Prätest- und Testzellen, stabil transformiert mit CRE-Sensor-DNA und mit Dopamin-D1-Rezeptor-DNA, mit verschiedenen Substanzen
- Fig. 37A, 37B, 38A und 38B:: Dosis-Wirkungskurven der Luciferaseaktivität in Abhängigkeit von der Aktivierung des Dopamin-D1-Rezeptors
- Fig. 39A, 39B:: Dosis-Wirkungskurven der Luciferaseaktivität in Abhängigkeit von der Aktivierung des Dopamin-D5-Rezeptors
- Fig. 40:: Optimierung eines Reagens zur Messung der Luciferase-Aktivität

Die Erfindung wird anhand der folgenden Beispiele näher erläutert:

Die Wirkung der in den Beispielen verwendeten Substanzen ist in folgender Tabelle angegeben:

| | |
|---|---|
| A23187 | Ca²⁺-Ionophor |
| Apomorphin | Agonist für den Dopamin-Rezeptor |
| Atropin | Antagonist für muskarinische Rezeptoren (unspezifisch) |
| Bromocryptin | Agonist für den Dopamin-Rezeptor |
| Carbachol | Agonist für muskarinische Rezeptoren |
| Clozapin | Antagonist für den Dopamin-Rezeptor (D4-spezifisch) |
| dibutyryl-cAMP (dbcAMP) | membranpermeables cAMP-Derivat |
| Dopamin | Agonist für den Dopamin-Rezeptor |
| Flupenthixol | Antagonist für den Dopamin-Rezeptor (D1- spezifisch) |
| Forskolin | Stimulator der Adenylat-Zyklase (Erhöhung von cAMP) |
| Haloperidol | Antagonist für den Dopamin-Rezeptor (D2-spezifisch) |
| IBMX | Phosphodiesterase Inhibitor (Akkumulation von cAMP) |
| Ketanserin | Antagonist für den Serotonin-Rezeptor (5-HT₂-spezifisch) |
| PMA (TPA) | Protein Kinase C Aktivator, täuscht DAG vor |
| SCH23390 | Antagonist für den Dopamin-Rezeptor (D1-spezifisch) |
| Serotonin (5-HT) | Agonist für Serotonin-Rezeptoren |
| Spiroperidol (=Spiperon) | Antagonist (5-HT₂ > 5-HT_{1A}-Rezeptor > Dopamin-Rezeptor) |
| SKF-38393 | Antagonist für den Dopamin-Rezeptor (D1-spezifisch) |

### Beispiel 1

### Herstellung von Grundvektoren für die Expression von Reportergenen in Säugetierzellen

### a) Konstruktion von Plasmid pADneo

Aus Teilen der Plasmide pBluescript SK+ (Short et al., 1988; Stratagene, La Jolla, CA) und pRc/CMV (Invitrogen, San Diego, CA; Katalog Nr. V750-20) wurde ein Plasmid hergestellt, das Replikationsursprung (ori) und Selektionsmarker für Ampicillinresistenz (Amp, β-Lactamase) in E.coli enthält. Die intergenische Region von M13 ermöglicht die Herstellung einzelsträngiger Plasmid-DNA nach Superinfektion der transformierten Bakterien mit einem Helferphagen (z.B. R408 oder M13K07; Stratagene) zur erleichterten Sequenzierung und Mutagenese der Plasmid-DNA. Weiters sind das Neomycin-Phosphotransferasegen (neo) unter der transkriptionellen Kontrolle des SV40 early Promotors (SV40) und das SV40 Polyadenylierungssignals (SV40 poly(A)) enthalten.

Das Plasmid pBluescript SK+ wurde mit HindIII linearisiert und 100 ng DNA in einem 100 µl PCR (Saiki et al., 1988) -Ansatz eingesetzt (Reaktionsmedium: 50 mM KCl, 10 mM Tris-Cl pH 8.3, 1.5 mM MgCl₂, 0.01 % (w/v) Gelatine, je 0.2 mM der vier Desoxynukleosidtriphosphate (dATP, dGTP, dCTP, dTTP); 2.5 Einheiten *Taq* Polymerase pro 100 µl). Als Primer wurden je 50 pmol der synthetischen Oligonukleotide EBI-1730 (SEQ ID NO:3) (5'-GGAATTCGCGCCCTGTAGCGGCG-3') und EBI-2134 (SEQ ID NO:4) (5'-CACTGAACTCGAGCAGCTGCGTTGCTGGCGTTTTTCC-3') eingesetzt. Nach 5 min Denaturieren bei 94°C erfolgte die PCR über 10 Zyklen (Zyklusbedingungen: 40 sec bei 94°C, 45 sec bei 55°C, 5 min bei 72°C, Perkin Elmer Cetus Thermal Cycler). Die Oligonukleotide flankieren die intergenische Region von M13 bzw. den Replikationsursprung (ori) mit dem dazwischenliegenden Gen für die β-Lactamase. Gleichzeitig wird am Ende des ori eine XhoI- und am anderen Ende eine EcoRI-Schnittstelle erzeugt (in der Oligonukleotidsequenz unterstrichen). Das Reaktionsgemisch wurde durch Extraktion mit Phenol-Chloroform von Protein befreit und die DNA mit Äthanol präzipitiert. Die erhaltene DNA wurde mit XhoI und EcoRI geschnitten und nach Elektrophorese in einem Agarosegel ein Fragment mit 2.2 kb isoliert.

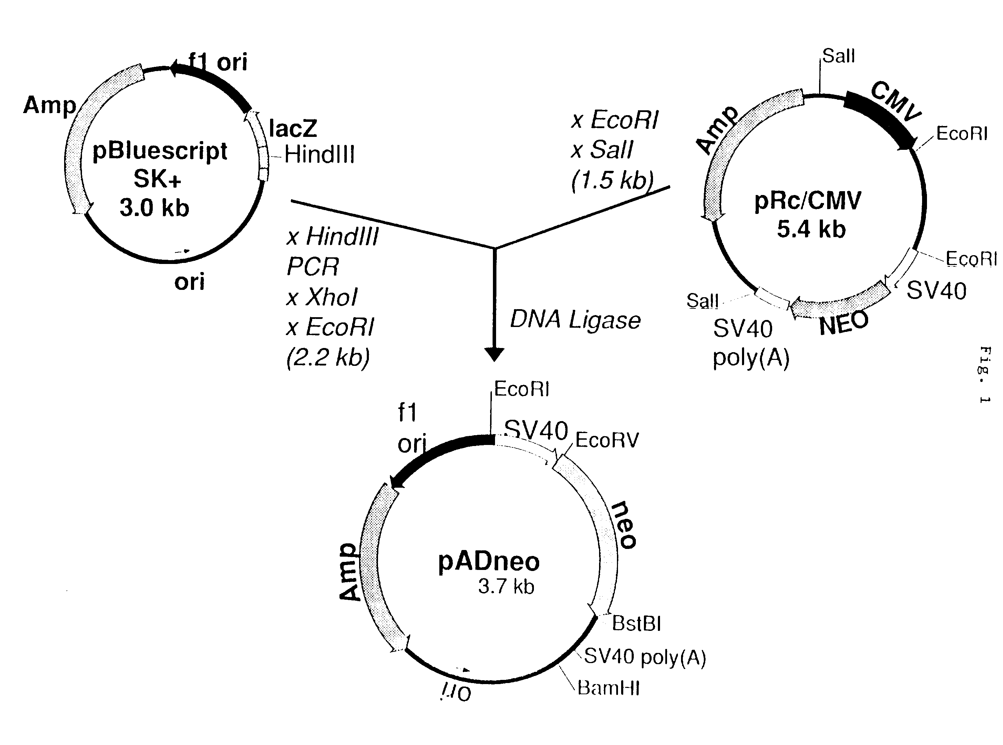

Das Plasmid pRc/CMV wurde mit EcoRI und SalI doppelt geschnitten, in einem Agarosegel elekrophoretisch aufgetrennt und ein 1.5 kb Fragment isoliert, das den SV40-Promotor, das *neo*-Gen und das SV40 poly(A)-Signal enthält. 100 ng der 2.2 kb Vektor-DNA wurden mit der zwei- bis dreifachen Menge an 1.5 kb Insert-DNA über Nacht bei 14°C mit T4 DNA-Ligase inkubiert, anschließend wurden für die Aufnahme von DNA kompetent gemachte (Chung und Miller, 1988) E.coli JM101-Zellen transformiert und auf Ampicillinresistenz selektionert. Von den entstandenen Kolonien wurde die Plasmid-DNA präpariert und durch Schneiden mit verschiedenen Restriktionsenzymen charakterisiert. Ein Plasmid der gewünschten Struktur wurde pADneo benannt (Fig.1).

### b) Konstruktion von Plasmid pADneoTK

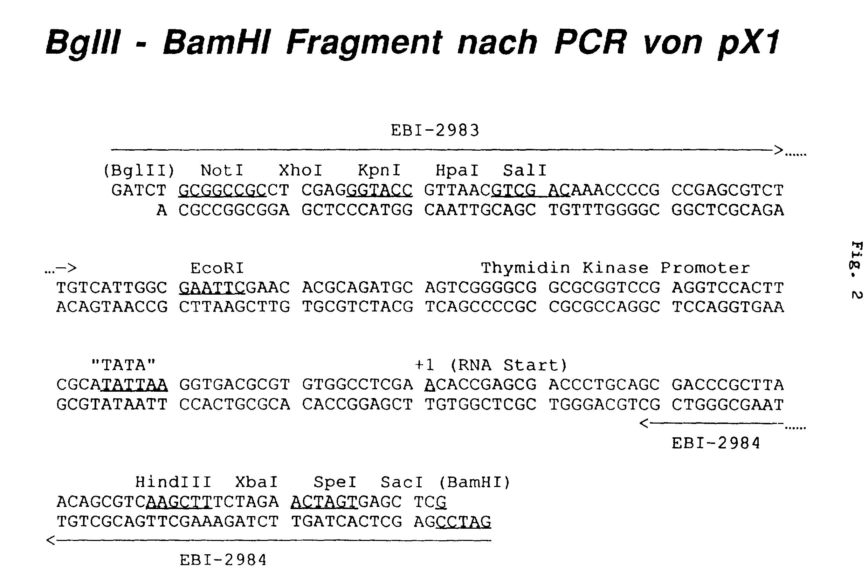

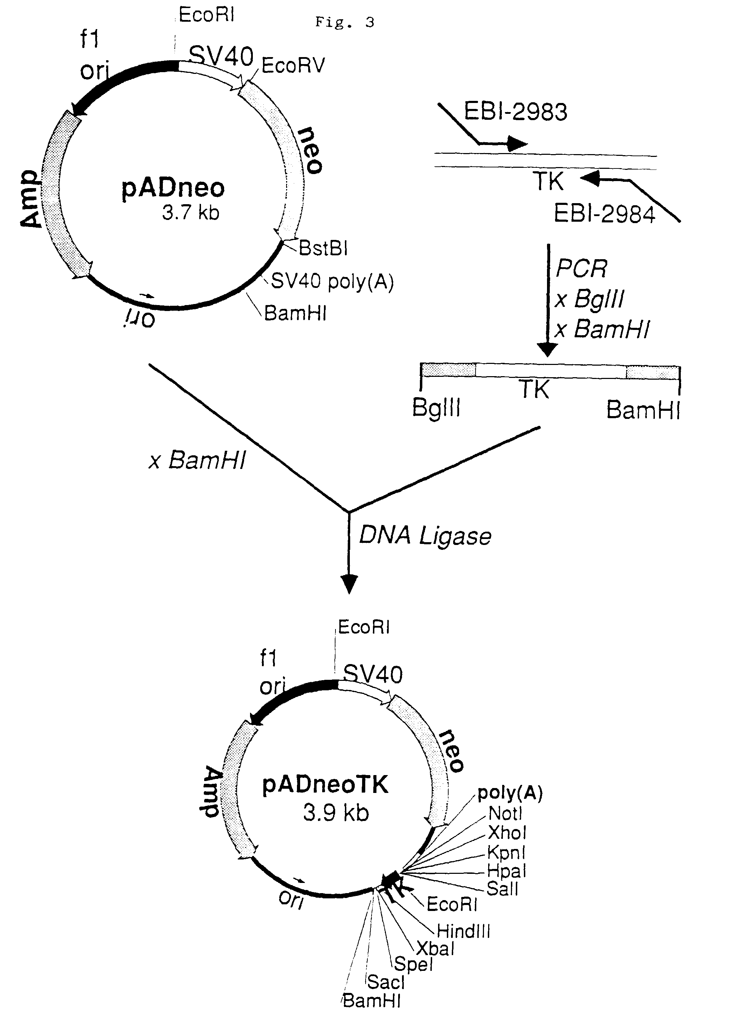

In das Plasmid pADneo wurde die Promotorregion des Thymidinkinase (TK)-Gens des Herpes Simplex Virus Typ I (HSV-I), flankiert von zwei Polyklonierstellen, eingefügt. Dieses DNA Fragment wurde durch PCR hergestellt. Als Vorlage für den TK-Promotor wurde das Plasmid pX1 (Wagner et al., 1981) verwendet, die Polyklonierstellen wurden durch Verlängerungen der Amplifikationsprimer am 5'-Ende, die nicht mehr komplementär zur Vorlage sind, erzeugt. 100 ng Plasmid pX1 wurden mit je 50 pmol der Oligonukleotide EBI-2983 (SEQ ID NO:5) (5'-GACTTCAGATCTGCGGCCGCCTCGAGGGTACCGTTAACGTCGACAAACCCCGCCCAGCGTCTTG-3') und EBI-2984 (SEQ ID NO:6) (5'-GACTTCGGATCCGAGCTCACTAGTTCTAGAAAGCTTGACGCTGTTAAGCGGGTCGC-3') 20 PCR Zyklen (Zyklusbedingungen: 40 sec 94°C, 45 sec 55°C, 1 min 72°C) unterworfen. Nach Entfernung der *Taq* Polymerase durch Phenol/Chloroform-Extraktion und Äthanolfällung der DNA wurden die Enden mit BamHI und BglII nachgeschnitten (unterstrichene Sequenz) und das 0.2 kb Fragment nach Elektrophorese aus einem Agarosegel isoliert (Fig.2). Anschließend wurde diese DNA mit BamHI-linearisiertem Plasmid pADneo ligiert und E.coli JM101 transformiert. Ein erhaltenes Plasmid, das den TK-Promotor in der gleichen Orientierung wie das neo-Gen enthielt, wurde pADneoTK benannt (Fig.3). Es enthält 5' vom TK-Promotor singuläre Schnittstellen für NotI, XhoI, KpnI, HpaI und SalI zur Klonierung von regulatorischen, den Promotor modulierenden Sequenzen, sowie 3' vom TK-Promotor singuläre Schnittstellen für HindIII, XbaI, SpeI, SacI und BamHI zur Insertion eines Reportergens.

### c) Konstruktion von Plasmid pADneoTKluci

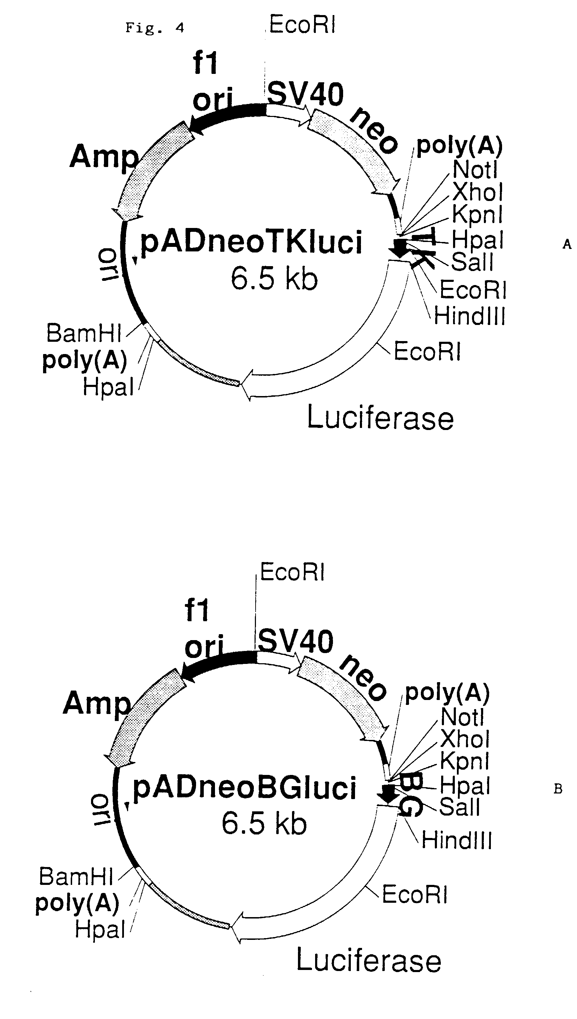

Das Gen für die *Photinus pyralis* Luciferase mit der SV40 poly(A) Region wurde aus einem Derivat von Plasmid pSV232AL-AΔ5' (De Wet et al., 1987), pBHluc (Voraberger et al., 1991) als 2.5 kb HindIII-BamHI-Fragment isoliert. pADneoTK wurde mit HindIII und BamHI doppelt geschnitten und mit dem 2.5 kb HindIII-BamHI Fragment aus dem Plasmid pBHluc ligiert. Ein nach Transformation von E.coli JM101 erhaltenes Plasmid der gewünschten Struktur wurde pADneoTKluci benannt (Fig.4A). Dieses Plasmid erlaubt die Expression von Luciferase unter der Kontrolle des TK-Promotors in Säugetierzellen.

### d) Konstruktion von Plasmid pADneoBGluci

Für die Optimierung der Induktionsfähigkeit der Reportergens wurde der TK-Promotor durch eine minimale Promotorsequenz des Kaninchen β-Globin-Gens ersetzt. Plasmid pADneoTKluci wurde mit SalI und HindIII doppelt geschnitten und der Vektoranteil aus einem Agarosegel isoliert. Der β-Globin Promotor mit flankierenden SalI-und HindIII-kompatiblen Enden wurde durch die synthetischen Oligonukleotide EBI-3182 (SEQ ID NO:7) (5'-GACTTCGGATCCGAGCTCACTAGTTCTAGAAAGCTTGACGCTGTTAAGCGGGTCGC-3') und EBI-3184 (SEQ ID NO:8) (5'-AGCTTGTAAGCAGCAGCTGCAGTGCTCTGCCTTTTATGCCCAAGG-3') hergestellt. Die beiden Oligonukleotide wurden am 5'-Ende durch Inkubation mit T4-Polynukleotidkinase und ATP phosphoryliert und anschließend mit dem oben beschriebenen Vektor ligiert. Ein nach Transformation von E.coli JM101 erhaltenes Plasmid, das die korrekte Sequenz enthielt, wurde pADneoBGluci benannt (Fig.4B).

### e) Konstruktion von Plasmiden pRc/RSVΔNaeI und pRc/RSVneo

Für die Verwendung von Sensor-DNA in Zellinen, welche die Replikation von Plasmiden mit SV40 Replikationsursprung erlauben (z.B. Cos-7 (ATCC CRL1651) und 293 (ATCC CRL1573)), war es für vergleichende Studien mit anderen Zellinien wünschenswert, den SV40 Promotor, unter dessen Kontrolle das *neo*-Gen in den zuvor beschriebenen Plasmiden steht, gegen einen anderen Promotor (z.B. Rous Sarcoma Virus (RSV) long terminal repeat (LTR)) zu ersetzen.

Um eine neue Expressionskassette für das *neo*-Gen herzustellen, wurden die nachfolgend beschriebenen Plasmide pRc/RSVΔNaeI und pRc/RSVneo hergestellt.

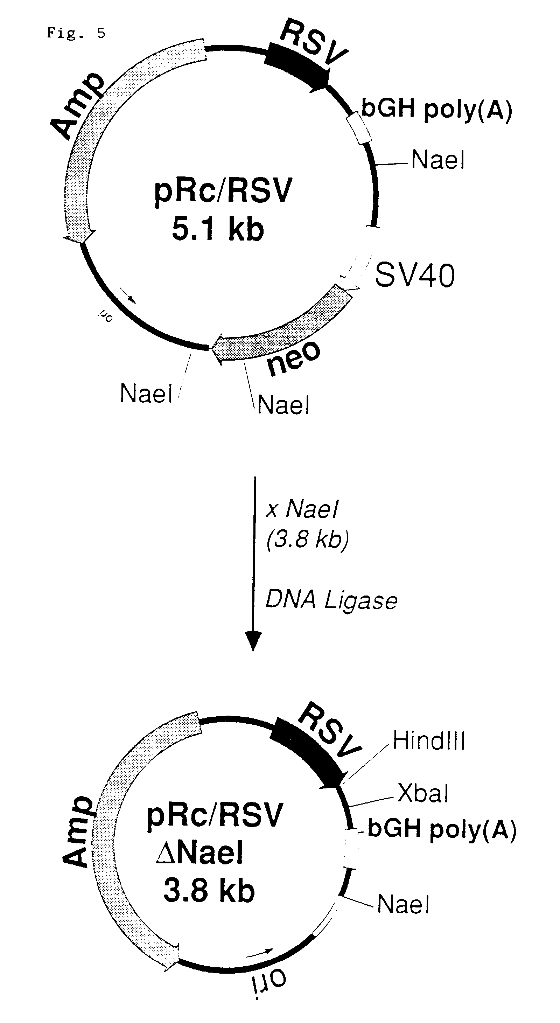

Plasmid pRc/RSV (Invitrogen, San Diego, CA; Katalog Nr. V780-20) wurde mit NaeI geschnitten, der 3.8 kb Vektoranteil aus einem Agarosegel isoliert und religiert. Dadurch wurde das 1.6 kb Fragment mit dem *neo*-Gen deletiert. Das erhaltene Plasmid wurde pRc/RSVΔNaeI benannt (Fig.5).

pRc/RSVΔNaeI wurde mit HindIII und XbaI doppelt geschnitten und die DNA Enden durch anschließende Behandlung mit dem Klenow Fragment der E.coli DNA Polymerase (Klenow-Enzym) in Gegenwart aller vier Desoxynukleotide stumpf gemacht.

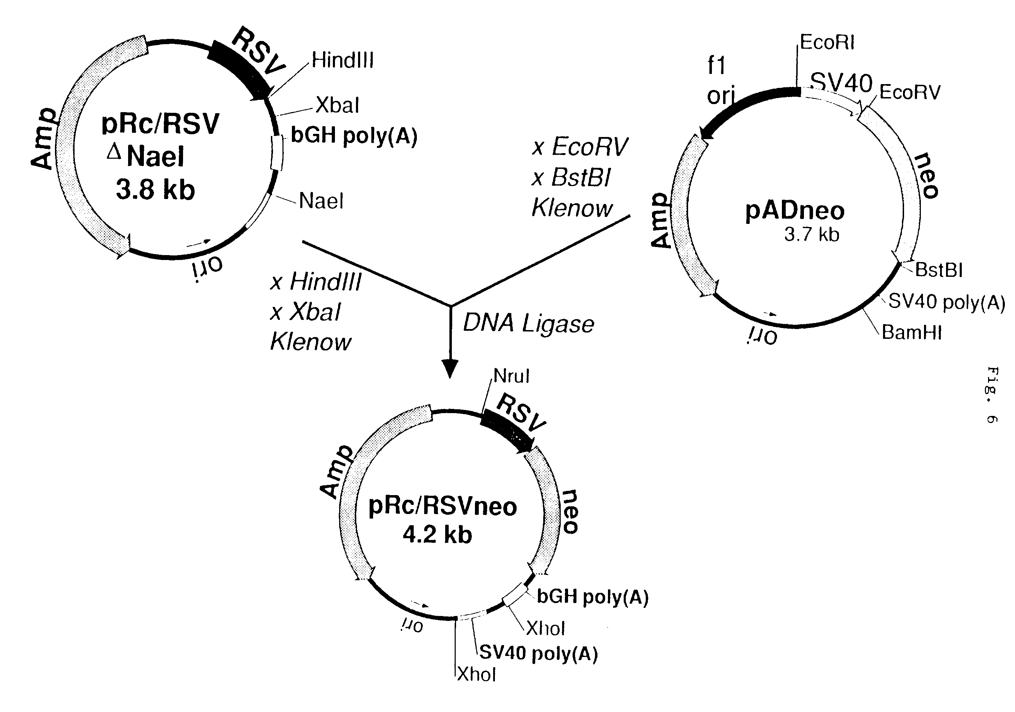

Plasmid pADneo wurde mit EcoRV und BstBI doppelt geschnitten, die DNA Enden ebenso durch anschließende Behandlung mit Klenow-Enzym stumpf gemacht und ein 0.86 kb DNA Fragment, welches das *neo*-Gen enthält, isoliert. Nach Ligation mit dem oben beschriebenen Vektor und Transformation wurde ein erhaltenes Plasmid der gewünschten Struktur pRc/RSVneo benannt (Fig.6). Dieses Plasmid enthält das *neo*-Gen unter der transkriptionellen Kontrolle des RSV Promotors und die Polyadenylierungssignale des Rinderwachstumshormons (bGH) und von SV40.

### f) Konstruktion von Plasmid pADneo2BGluci

Die Expressionskassette SV40 Promotor - *neo*-Gen - SV40 poly(A) Signal von pADneoBGluci wurde gegen die Expressionskassette RSV Promotor - *neo*-Gen - bGH poly(A) aus Plasmid pRc/RSVneo ausgetauscht.

Das Plasmid pRc/RSVneo wurde mit XhoI geschnitten und die DNA Enden anschließend mit Klenow-Enzym stumpf gemacht. Ein XhoI-NotI-Adapter, hergestellt aus den Oligonukleotiden EBI-3285 (SEQ ID NO:9) (5'-TCGATGCGGCCGCGACTTCAG-3') und EBI-3286 (SEQ ID NO:10) (5'-CTGAAGTCGGCCGCA-3'), wurde mit der geschnittenen pRc/RSVneo DNA ligiert. Dadurch wird die XhoI-Schnittstelle zerstört und eine NotI Stelle (unterstrichen) eingeführt. Nach Hitzeinaktivierung der DNA-Ligase wurde die DNA mit NruI und NotI doppelt geschnitten und ein 1.54 kb Fragment aus einem Agarosegel isoliert. Dieses DNA Fragment enthält die RSV-*neo*-bGH-poly(A)-Kassette.

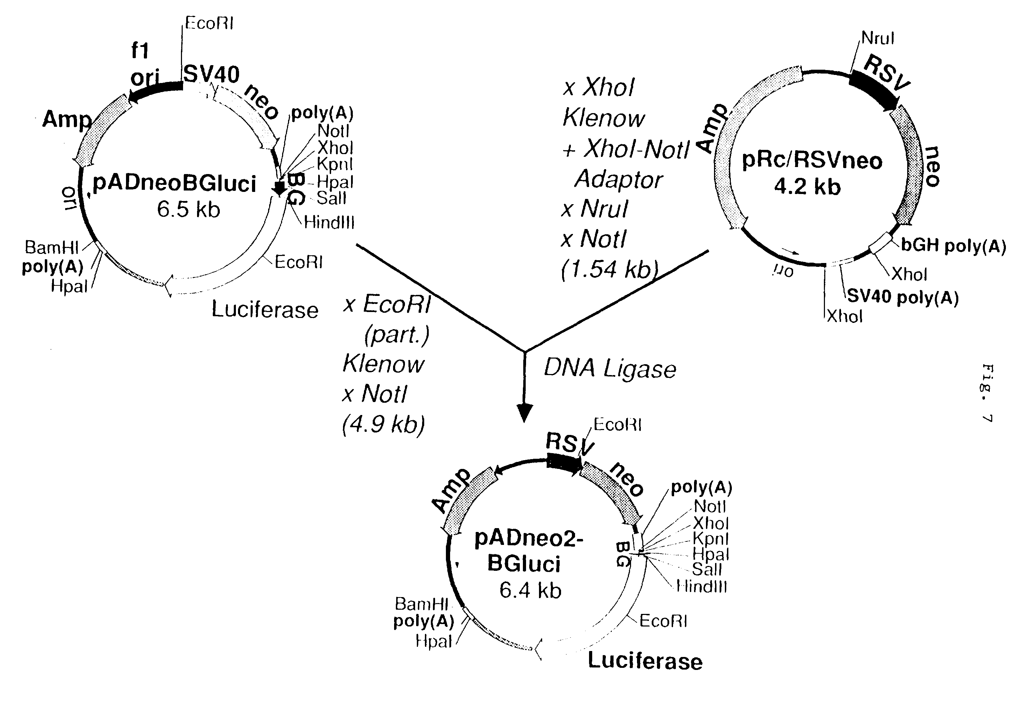

Plasmid pADneoBGluci wurde mit EcoRI partiell geschnitten, die Enden mit Klenow-Enzym begradigt und anschließend mit NotI nachgeschnitten. Ein 4.9 kb langes DNA Fragment wurde aus einem Agarosegel isoliert und mit dem zuvor beschriebenen 1.54 kb NruI - NotI - Fragment ligiert. Ein nach Transformation von E.coli erhaltenes Plasmid der gewünschten Struktur wurde pADneo2BGluci benannt (Fig.7).

### g) Konstruktion von Reporterplasmiden mit durch cAMP regulierbaren Elementen (CRE-Sensor-DNA)

Zur Kontrolle einer eventuellen Querempfindlichkeit der Sensor-DNA für IP₃/DAG (enthaltend TRE-Elemente) wurde die Expressionskassette für das Luciferase-Reportergen unter die Kontrolle von verschiedenen CRE-Elementen gestellt. Die Auswahl der CRE-Sequenzen erfolgte nach der Zusammenstellung von charakterisierten CRE-Elementen (Montminy et al., 1990). Es wurden CRE-Sequenzen gewählt, welche einerseits die perfekte 8 Basen lange, palindromische Konsensussequenz TGACGTCA und andererseits möglichst keine größeren Gruppierungen von GC-Paaren-aufwiesen, die Ähnlichkeit mit den Erkennungssequenzen für Spl-Transkriptionsfaktoren (CCGCCC oder GGGCGG) in den umgebenden Sequenzen enthalten. Es wurden mehrere CRE-Sequenzen in Tandem verwendet, um den modulierenden Effekt von cAMP zu verstärken. Eine Kombination unterschiedlicher CRE-Sequenzen wurde verwendet, um zu vermeiden, daß Klonierung und Stabilität in E.coli ungünstig beeinflußt werden, wenn vollkommen identische Sequenzen in mehreren Wiederholungen auftreten.

Durch Einsetzen synthetischer Oligonukleotide in das Plasmid pADneo2BGluci 5' zum β-Globin-Promotor wurden zwei Plasmide mit je 3 hintereinanderliegenden CRE-Sequenzen (pADneo2-C3BVC-BGL und pADneo2-C3SVC-BGL), und durch Kombination dieser Plasmide das Plasmid pADneo2-C6-BGL mit 6 CRE-Sequenzen hergestellt.

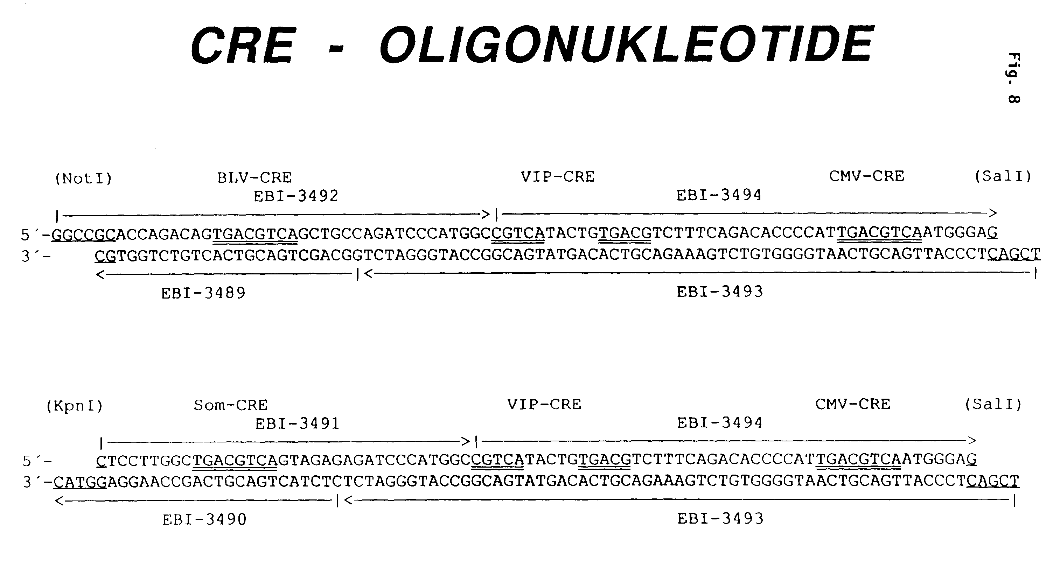

Die dreifachen CRE-Sequenzen wurden durch Ligation von zwei Oligonukleotidpaaren mit zueinander komplementären DNA Enden hergestellt (Fig.8). Je 20 pmol Oligonukleotid EBI-3489 (SEQ ID NO:11) (5'-GGCAGCTGACGTCACTGTCTGGTGC-3') und EBI-3491 (SEQ ID NO:12) (5'-CTCCTTGGCTGACGTCAGTAGAGAGATCCCATGGC-3') wurden in 15 µl Kinasepuffer (70 mM Tris-HCl pH 7.6; 10 mM MgCl₂, 5 mM Dithiotreitol, 2 mM ATP) mit 15 Einheiten Polynukleotid-Kinase 1 h bei 37°C inkubiert und das Enzym durch Hitze (5 min bei 95°C) inaktiviert. In gleicher Weise wurde das 5'-Ende der Oligonukleotide EBI-3490 (SEQ ID NO:13) (5'-CTCTACTGACGTCAGC-CAAGGAGGTAC-3') und EBI-3494 (SEQ ID NO:14) (5'-CGTCATACTGTGACGTCTTTCAGACACCCCATTGACGTCAATGGGAG-3') phosphoryliert.

Äquimolare Mengen der jeweils komplementären Oligonukleotide ohne 5'-Phosphatgruppe wurden mit den phosphorylierten Oligonukleotiden gemischt: EBI-3492 (SEQ ID NO:15) (5'-GGCCGCACCAGACAGTGACGTCAGCTGCCAGATCCCATGGC-3') mit EBI-3489; EBI-3491 (SEQ ID NO:16) (5'-CTCCTTGGCTGACGTCAGTAGAGAGATCCCATGGC-3') mit EBI-3490; EBI-3493 (SEQ ID NO:17) (5'-TCGACTCCCATTGACGTCAATGGGGTGTCTGAAAGACGTCACAGTATGACGGCCATGGGATCT-3') mit EBI-3494 und durch 5 min Inkubation bei 56°C angelagert. Jeweils 10 pmol der nun doppelsträngigen Oligonukleotidpaare EBI-3489 / EBI-3492 und EBI-3493 / EBI-3494 wurden in 30 µl Ligationspuffer (70 mM Tris-HCl pH 7.6; 10 mM MgCl₂, 5 mM Dithiotreitol, 1 mM ATP) mit 1 Einheit T4 DNA-Ligase über Nacht bei 14°C inkubiert und das Enzym anschließend 10 min bei 70°C inaktiviert. Die ligierten Oligonukleotidpaare wurden am 5'-Ende in 50 µl Kinasepuffer mit 15 Einheiten Polynukleotid-Kinase 1 h bei 37°C phosphoryliert.

In gleicher Weise wurden die Oligonukleotidpaare EBI-3490 / EBI-3491 und EBI-3493 / EBI-3494 miteinander verbunden und anschließend phosphoryliert.

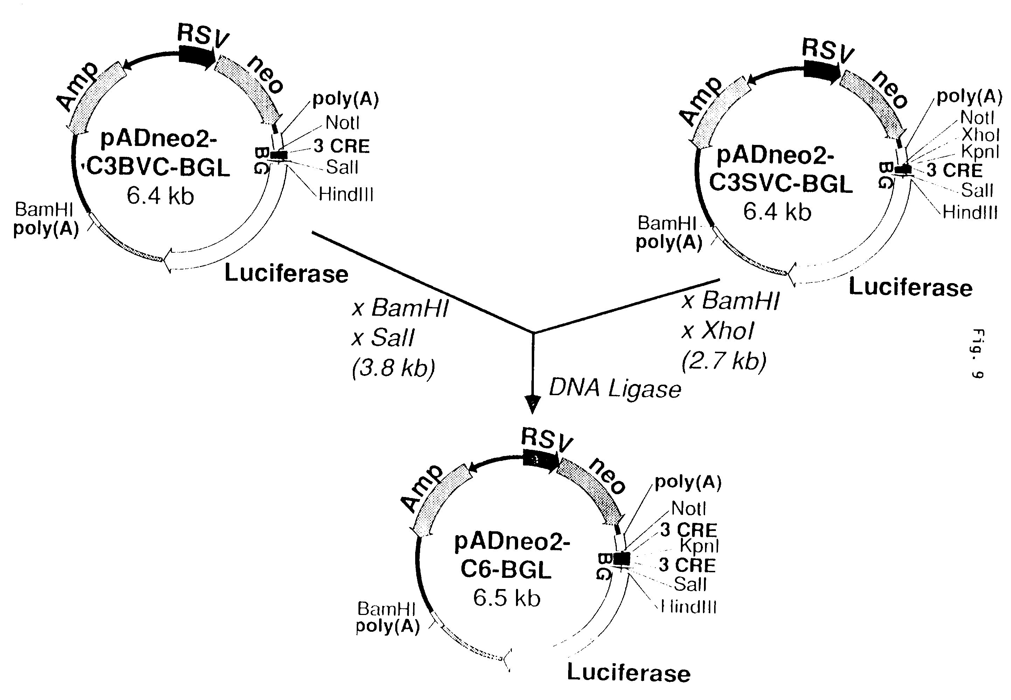

100 ng mit NotI und SalI doppelt geschnittenes Plasmid pADneo2BGluci wurden mit 0.2 pmol ligiertem Oligonukleotidkomplex aus EBI-3489/3492/3493/3494 in 20 µl Ligationspuffer mit 1 Einheit T4 DNA-Ligase 4 h bei 22°C inkubiert und anschließend E.coli JM101 transformiert. Daraus erhaltene Plasmide wurden der Sequenzanalyse unterworfen und ein Plasmid der gewünschten Struktur wurde pADneo2-C3BVC-BGL benannt (Fig.9). Dieses Plasmid enthält 3 CRE-Sequenzen, abgeleitet von Bovine Leukemia Virus LTR (BLV), Vasoactive Intestinal Peptide (VIP) und Cytomegalovirus Promotor (CMV). (Die Plasmidbezeichnung nimmt auf diese Sequenzen durch die Abkürzung "BVC" Bezug.)

In analoger Weise wurde der Oligonukleotidkomplex EBI-3490/3491/3493/3494 in mit KpnI und SalI doppelt geschnittenen Plasmidvektor pADneo2BGluci geklont und Plasmid pADneo2-C3SVC-BGL erhalten (Fig.9). Dieses Plasmid enthält 3 CRE-Sequenzen, abgeleitet von Somatostatin (Som), VIP und CMV. (Die Plasmidbezeichnung nimmt auf diese Sequenzen durch die Abkürzung "SVC" Bezug.)

Zur Herstellung eines Plasmids mit 6 CRE-Sequenzen wurde das Plasmid pADneo2-C3BVC-BGL mit BamHI und SalI doppelt geschnitten und der 3.8 kb Vektoranteil aus einem Agarosegel isoliert. Plasmid pADneo2-C3SVC-BGL wurde mit BamHI und XhoI doppelt geschnitten und das 2.7 kb Insert isoliert. Diese beiden DNA Fragmente wurden ligiert, E.coli transformiert. Ein erhaltenes Plasmid der gewünschten Struktur wurde pADneo2-C6-BGL benannt (Fig.9).

### h) Herstellung von Grundvektoren für die Expression von Genen in Säugetierzellen mit Hygromycin B Resistenzmarker

Ausgehend von den Expressionsplasmiden pAD-CMV1 und pAD-CMV2 (EP-A 393 438) und pHEBo (Sugden et al. 1985) wurden Plasmide für die Expression von Genen oder cDNAs unter der transkriptionellen Kontrolle des CMV Promoter/Enhancers und die Selektion für Hygromycin B Resistenz hergestellt.

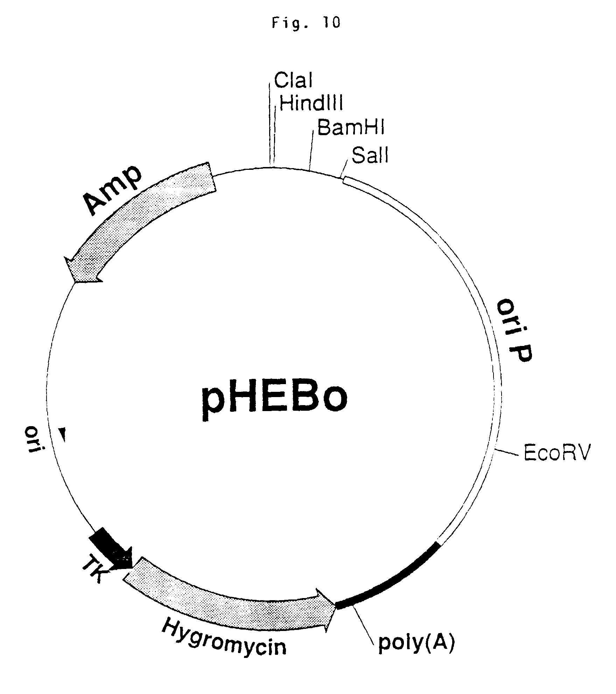

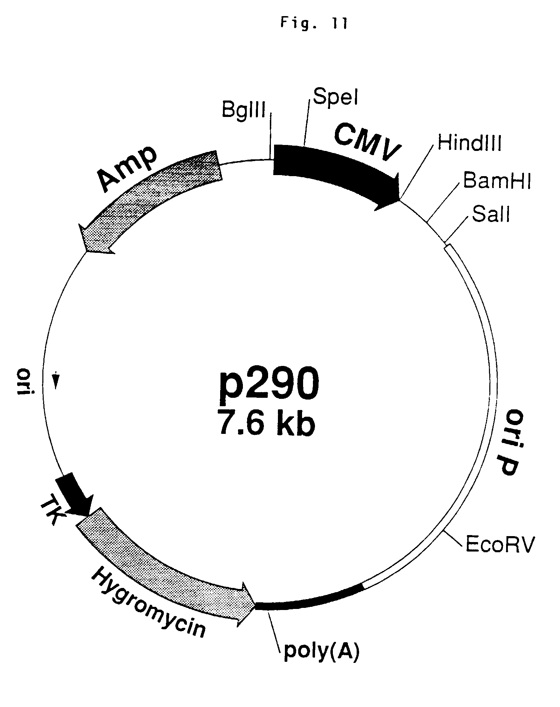

Plasmid pHEBo (Sugden et al. 1985; Fig. 10), welches das bakterielle Hygromycin-B-Phosphotransferase Gen (Gritz und Davies 1983) unter der transkriptionellen Kontrolle des HSV Thymidin Kinase Promoters (McKnight 1980) enthält, wurde mit ClaI geschnitten, die DNA Enden mit Klenow-Enzym aufgefüllt und anschließend mit BamHI geschnitten. In diesen Vektor wurde ein 0,76 kb BamHI-HindIII Fragment (BamHI Ende mit Klenow Enzym aufgefüllt) mit der CMV Promoter/Enhancer Sequenz (Stinski und Roehr 1985) kloniert und Plasmid p290 (Fig. 11) erhalten.

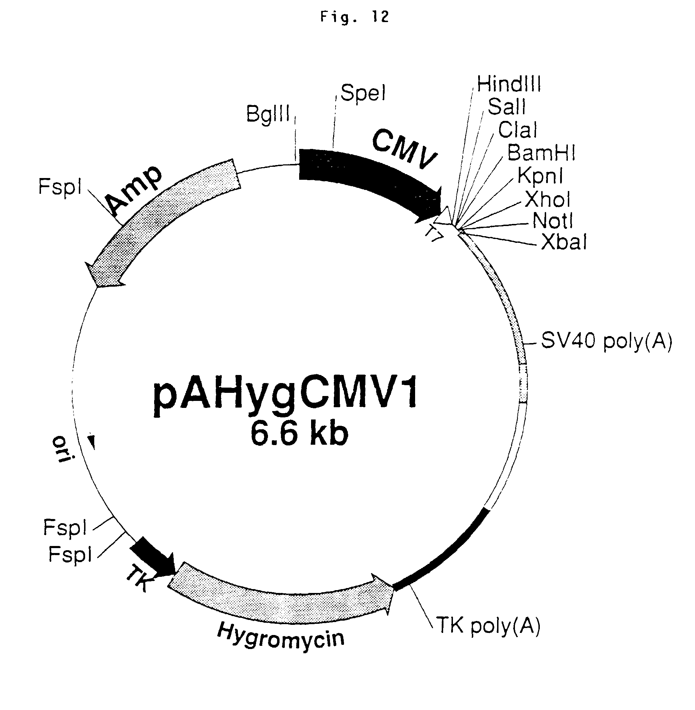

Das SpeI-EcoRV Fragment von Plasmid p290, das CMV Promoter und EBV ori P enthält, wurde herausgeschnitten. Plasmid pAD-CMV1 wurde mit BglII geschnitten, die DNA Enden mit Klenow Enzym stumpf gemacht und anschließend mit SpeI geschnitten. Das Gel-gereinigte 1,6 kb DNA Fragment, das CMV Promoter, Polyklonierstelle, SV40 Splice und poly(A) Signale enthält, wurde mit dem zuvor beschriebenen p290 Vektorteil ligiert. Das erhaltene Plasmid wurde pAHygCMV1 benannt (Fig. 12).

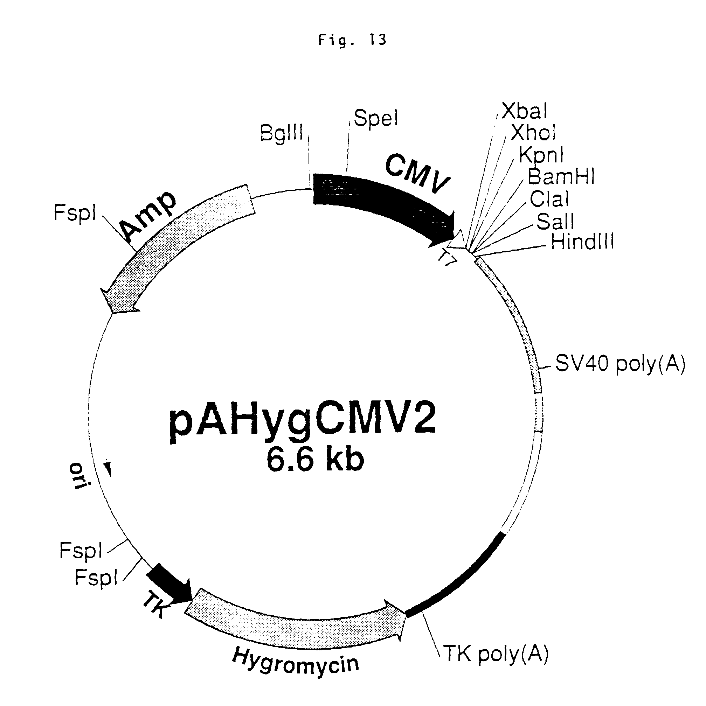

Auf gleiche Weise wurde aus Plasmid pAD-CMV2 das SpeI-BglII Fragment isoliert und mit dem p290 SpeI-EcoRV Vektorteil ligiert. Das erhaltene Plasmid, welches die Polyklonierstelle in umgekehrter Orientierung als in pAHygCMV1 enthält, wurde pAHygCMV2 benannt (Fig. 13).

Die vollständige Nukleotidsequenz des Plasmids pAHygCMV1 ist in SEQ ID NO:36 wiedergegeben.

Die Abschnitte auf dem Plasmid pAHygCMVl (angegeben in der Numerierung der Basen) entsprechen folgenden

| Sequenzen: | |
|---|---|
| 1 - 767 | CMV Promoter |
| 768 - 785 | T7 Promoter |
| 794 - 854 | Polyklonierstelle |
| 854 - 1552 | SV40 t Intron und poly-Adenylierungssignale |
| 1553 - 1736 | 5' nicht kodierende Region des Hamster DHFR Gens |
| 1737 - 2261 | EBV ori P Teilsequenz |
| 2262 - 2856 | HSV Thymidin Kinase3' nicht kodierende Region mit poly-Adenylierungssignal |
| 2857 - 3912 | Hygromycin B Phosphotransferase Gen |
| 3913 - 4161 | HSV Thymidin Kinase Promoter |
| 4162 - 6531 | pBR322 Vektor Teil |
| 6532 - 6623 | Linkersequenzen entstanden aus diversen Kloniervorgängen, teilweise von plink322 (Maniatis et al. 1982) |

Die Nukleotidsequenz des Plasmids pAHygCMV2 ist in SEQ ID NO:37 wiedergegeben. Sie unterscheidet sich von pAHygCMV1 nur im Bereich der Polyklonierstelle; das Cytosin an Position 856 in pAHygCMV2 entspricht Cytosin an Position 849 in pAHygCMVl.

### Beispiel 2

### Konstruktion von Reporterplasmiden mit durch Phorbolester (TPA) induzierbaren Elementen (TRE-Sensor-DNA)

Klonierung und Deletionsanalyse von 1.3 kb der 5'-flankierenden Region des humanen Gens für das Interzelluläre Adhesionsmolekül ICAM-1 haben ergeben, daß dieses Fragment i) in der Lungenadenokarzinom-Zellinie A549 (ATCC CCL 185) durch TPA induziert, jedoch nicht durch Forskolin aktiviert werden kann, und ii) ein TPA Response Element (TRE) mit der DNA Sequenz TGATTCA enthält (Voraberger et al., 1991). Das 1.3 kb lange ICAM-1 Fragment, bzw. Oligonukleotide, die drei TRE-Elemente in Tandem-Orientierung enthalten, wurde daher zur Konstruktion von Vektoren verwendet, in denen das Luciferase-Gen durch TPA induziert werden kann.

### a) Herstellung des Plasmids pBHluc1.3

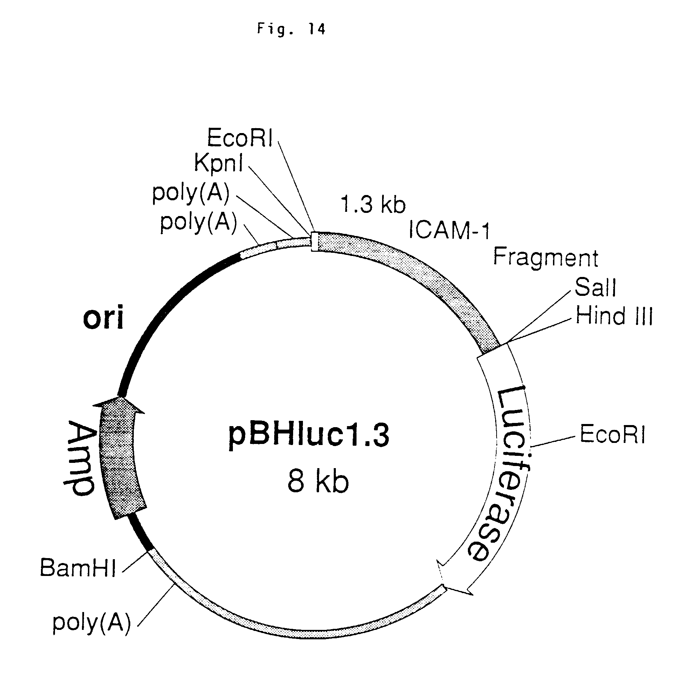

Das Plasmid pBHlucl.3 (Fig. 14) enthält die 1.3 kb lange Regulationsregion des ICAM-1 Gens, die dem Luciferase-Gen vorgeschaltet ist. Seine Herstellung wurde von Voraberger et al., 1991, beschrieben.

### b) Herstellung des Plasmids pADneo(1.3ICAM)luci (TRE-Sensor-DNA)

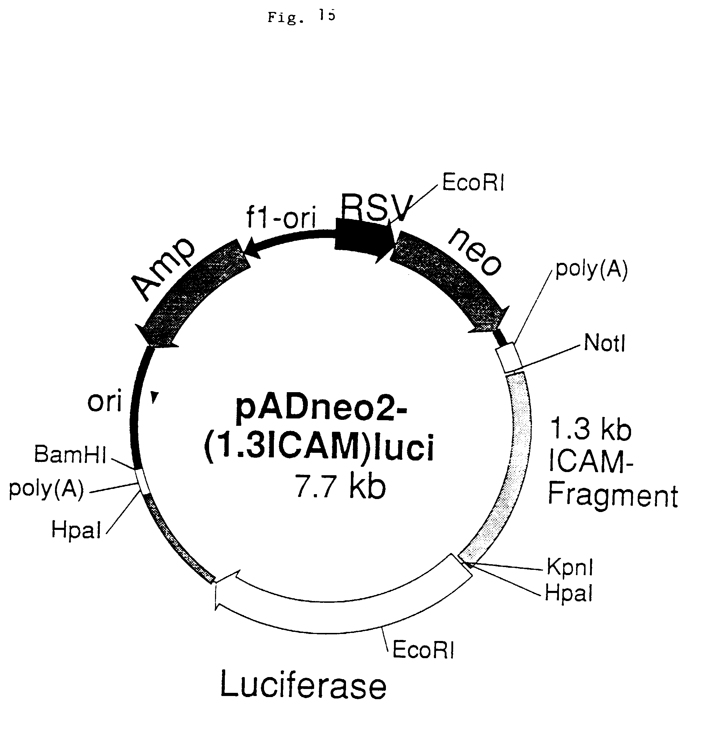

Aus dem Plasmid pADneo2BGluci (siehe Beispiel 1) wurde zuerst der β-Globin-Promotor herausgeschnitten, indem das Plasmid mit den Restriktionsenzymen SalI und HindIII geschnitten, die DNA-Enden des Plasmids durch Zugabe aller 4 dNTPs und von Klenow-Enzym "blunt end" gemacht, und schließlich das Plasmid durch Zugabe von T4-DNA-Ligase religiert wurde. Dieses Plasmid ohne β-Globin-Promotor, genannt pADneo2luci, wurde anschließend mit NotI und KpnI geschnitten, das 1.3 kb lange ICAM-1 Fragment wurde aus dem Plasmid Bluescript KS (siehe Voraberger et al., 1991) ebenfalls mit NotI und KpnI herausgeschnitten und in pADneo2luci hineinligiert. Dieses Plasmid, das pADneo(1.3ICAM)luci genannt wurde (Fig. 15), enthält die Regulations- und Promotorregion von ICAM-1, vorgeschaltet dem Luciferase-Gen.

### c) Herstellung des Plasmids pADneo(3TRE)BGluci (TRE-Sensor-DNA)

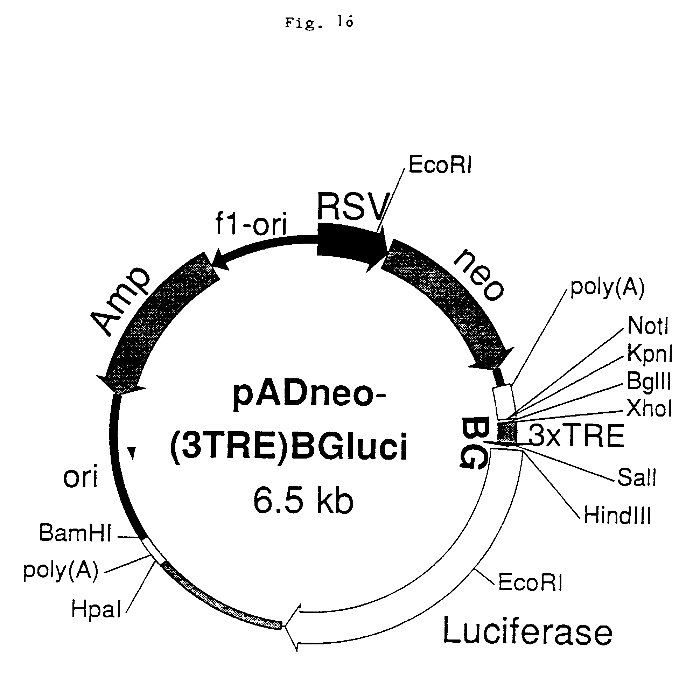

Dieses Plasmid wurde durch Einsetzen von synthetischen Oligonukleotiden, kodierend für die Restriktionsstellen KpnI, BglII und XhoI, gefolgt von 3 hintereinanderliegenden TRE-Sequenzen, 5' zum β-Globin Promotor von Plasmid pADneo2BGluci hergestellt. Hierzu wurden die Oligonukleotide EBI-3677 (SEQ ID NO:18) (5'-GGCCGCAGGTACCAGATCTACTCGAGTGTAGACCGTGATTCAAGCTTAGCTGTAGAC-3'), EBI-3671 (SEQ ID NO:19) (5'-GCTTGAATCACGGTCTACACTCGAGTAGATCTGGTACCTGC-3'), EBI-3678 (SEQ ID NO:20) (5'-TCGACTAAGCTTGAATCACGGTCTACAGCTAAGCTTGAATCACGGTCTACAGCTAA-3') und EBI-3672 (SEQ ID NO:21) (5'-CGTGATTCAAGCTTAGCTGTAGACCGTGATTCAAGCTTAG-3') phosphoryliert und äquimolare Mengen der komplementären Oligonukleotide EBI-3677 und EBI-3671, und EBI-3678 und EBI-3672 aneinander angelagert, wie in Beispiel 1 beschrieben. Der Vektor pADneo2BGluci wurde mit NotI und SalI geschnitten, und äquimolare Mengen an geschnittenem Plasmid pADneo2BGluci, des Oligonukleotidpaares EBI-3677/3671 und des Oligonukleotidpaares EBI-3672/3678 wurden zusammengemischt und durch Zugabe von T4-DNA-Ligase miteinander ligiert. Daraus erhaltene Plasmide wurden der Sequenzanalyse unterworfen und ein Plasmid, das die gewünschten drei TRE-Sequenzen enthält, wurde pADneo(3TRE)BGluci genannt (Fig. 16).

### d) Herstellung der Plasmide pADneo(nTREdx)BGluci

Diese Plasmide enthalten eine Anzahl von n TRE-Elementen, deren Abstand untereinander x Basen beträgt. Die Herstellung der Plasmide pADneo(3xTREd16)BGluci, pADneo(3xTREd21)BGluci, pADneo(3xTREd24)BGluci und pADneo(3xTREd34)BGluci unter Verwendung der folgenden Oligonukleotide erfolgte wie unter c) beschrieben:

EBI-3775 (SEQ ID NO:22) (5'-GGCCGCAGGTACCAGATCTACTCGAGTGTAGACCGTGATTCAAGCTTAGTGTAGAC-3') und Komplement-Oligonukleotid EBI-3671 (siehe oben), EBI-3776 (SEQ ID NO:23) (5'-TCGACCTTGAATCACGGTCTACACTAAGCTTGAATCACGGTCTACACTAA-3') und Komplement-Oligonukleotid EBI-3777 (SEQ ID NO:24) (5'-CGTGATTCAAGCTTAGTGTAGACCGTGATTCAAGG-3') für pADneo(3xTREd16)BGluci;

EBI-3771 (SEQU ID NO:25) (5'-GGCCGCAGGTACCAGATCTACTCGAGTGTAGACCGTGATTCAAGCTTAGCCTG-3') urd Komplement-Oligonukleotid EBI-3671 (siehe oben), EBI-3772 (SEQ OD NO:26) (5'-TCGACTAAGCTTGAATCACGGTCTACACCAGGCTAAGCTTGAATCACGGTCTACACCAGGCTAA-3') und Komplement-Oligonukleotid EBI-3774 (SEQ ID NO:27) (5'-GTGTAGACCGTGATTCAAGCTTAGCCTGGTGTAGACCGTGATTCAAGCTTAG-3') für pADneo(3xTREd21)BGluci;

EBI-3780 (SEQ ID NO:28) (5'-GGCCGCAGGTACCAGATCTACTCGAGTGTAGACCGTGATTCAAGCTTAGCCTGGCGGTGTAGAC-3') und Komplement-Oligonukleotid EBI-3778 (SEQ ID NO:29) (5'-CCAGGCTAAGCTTGAATCACGGTCTACACTCGAGTAGATCTGGTACCTGC-3'), EBI-3779 (SEQ ID NO:30) (5'-CGTGATTCAAGCTTAGCCTGGCGGTGTAGACCGTGATTCAAGCTTAGCCTG-3') und Komplement-Oligonukleotid EBI-3781 (SEQ ID NO:31) (5'-TCGACAGGCTAAGCTTGAATCACGGTCTACACCGCCAGGCTAAGCTTGAATCACGGTCTACACCG-3') für pADneo(3xTREd24)BGluci;

EBI-3786 (SEQ ID NO:32) (5'-GGCCGCAGGTACCAGATCTACTCGAGTGTAGACCGTGATTCAAGCTTAGCCTGGCCGGTTAGCGCGGTGTAGAC-3') und Komplement-Oligonukleotid EBI-3782 (SEQ ID NO:33) (5'-CGCGCTAACCGGCCAGGCTAAGCTTGAATCACGGTCTACACTCGAGTAGATCTGGTACCTGC-3'), EBI-3790 (SEQ ID NO:34) (5'-TCGACAGGCTAAGCTTGAATCACGGTCTACACCGCGCTAACCGGCCAGGCTAAGCTTGAATCACGGTCTACAC-3') und Komplement-Oligonukleotid EBI-3791 (SEQ ID NO:35) (5'-CGTGATTCAAGCTTAGCCTGGCCGGTTAGCGCGGTGTAGACCGTGATTCAAGCTTAGCCTG-3') für pADneo(3xTREd34)BGluci.

Um die entsprechenden Plasmide mit 6 TRE-Elementen zu erhalten, wurden die jeweiligen Plasmide mit 3 TRE-Elementen mit NotI und XhoI geschnitten und die entsprechenden Oligonukleotide nochmals hineinligiert. Bedingt durch die Konstruktion der Oligonukleotide ergibt sich dadurch eine Verdopplung der TRE-Elemente mit den entsprechenden Abständen. Die erhaltenen Plasmide wurden pADneo(6xTREd16)BGluci, pADneo(6xTREd21)BGluci, pADneo(6xTREd24)BGluci und pADneo(6xTREd34)BGluci genannt.

### Beispiel 3:

### Klonierung von G-Protein gekoppelten Rezeptoren und Herstellung von Expressionsplasmiden (Rezeptor-DNA)

Die cDNA des interessierenden humanen 5-HT₂-Rezeptors wurde durch Screenen einer cDNA-Bank erhalten und in die Expressionsvektoren pAD-CMV1 bzw. pAD-CMV2 (EP-A 393 438) hineinkloniert.

### a) Isolierung eines Klons, enthaltend die für den humanen 5-HT₂-Rezeptor kodierende Sequenz

Durch Homologiescreenen einer humanen Hippocampus cDNA Bank im Lambda ZAP Vektor (Stratagene 936205) mit einem Klon, enthaltend die Ratten-5-HT₂-Rezeptorsequenz (Julius et al., 1990, Pritchett et al., 1988), wurde ein Klon isoliert und das Insert, enthalten im Plasmid pBluescript SK, sequenziert. Die erhaltene DNA- und die abgeleitete Aminosäuresequenz sind in SEQ ID NO:1 und SEQ ID NO:2 angegeben. Der Vergleich der Aminosäuresequenz des humanen 5-HT₂-Rezeptors mit der publizierten Aminosäuresequenz des Ratten 5-HT₂-Rezeptors (Julius et al., 1990) ergab eine Übereinstimmung der beiden Aminosäuresequenzen von 90 %.

### b) Subklonierung der 5-HT₂-Rezeptorsequenz in den Expressionsvektor pAD-CMV2

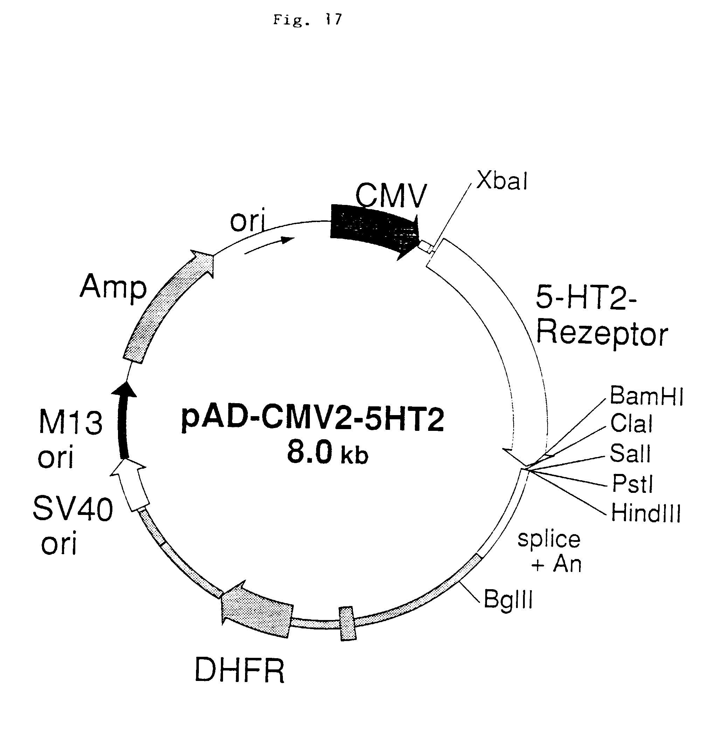

Der Expressionsvektor pAD-CMV2 wurde mit EcoRI geschnitten, die DNA-Enden durch Zugabe aller 4 dNTPs und von Klenow-Enzym "blunt end" gemacht, und der Vektor danach mit BamHI geschnitten. In diesen Vektor wurde die mit SmaI und BamHI aus dem Plasmid pBluescript herausgeschnittene 5-HT₂-Rezeptorsequenz hineinligiert, und ein so erhaltener Klon wurde pAD-CMV2-5HT₂ genannt (Fig. 17).

### c) Klonierung der Dopamin-D1-Rezeptor-Sequenz in den Expressionsvektor pAD-CMV2

Plasmid pHD₁-Gem, das ein 3 kb EcoRI-SacI Fragment der humanen Dopamin-D1-Rezeptor-Sequenz in pGEM Blue Plasmid Vektor (Promega) enthält (Zhou et al., 1990), wurde mit EcoRI und BamHI doppelt geschnitten und das 3 kb DNA-Insert isoliert. Expressionsplasmid pAD-CMV2 (EP-A 393 438) wurde mit EcoRI und BamHI doppelt geschnitten und das 3 kb D1-Rezeptor-Fragment gerichtet kloniert, sodaß die Transkription des Dopamin-D1-Rezeptors unter der Kontrolle des Cytomegalovirus (CMV) Promotor/Enhancer Elements steht. Das erhaltene Plasmid wurde pAD-CMV2:D1 benannt (Fig. 18).

### d) Subklonierung der NK2- bzw. der 5HT2-Rezeptorsequenz in den Expressionsvektor pAHygCMVl

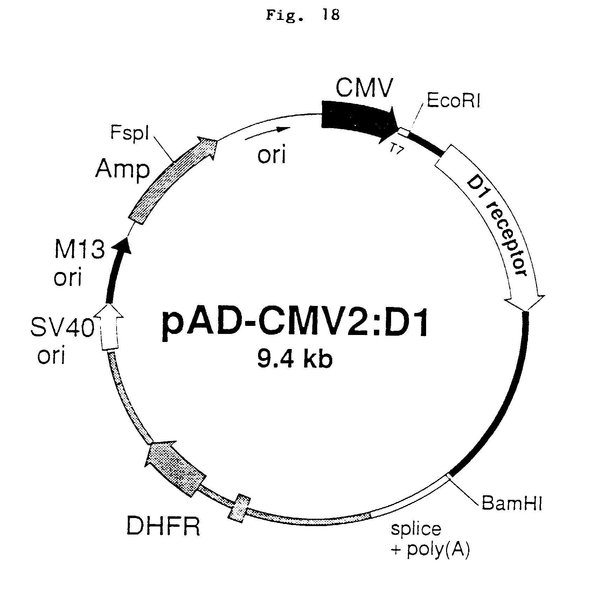

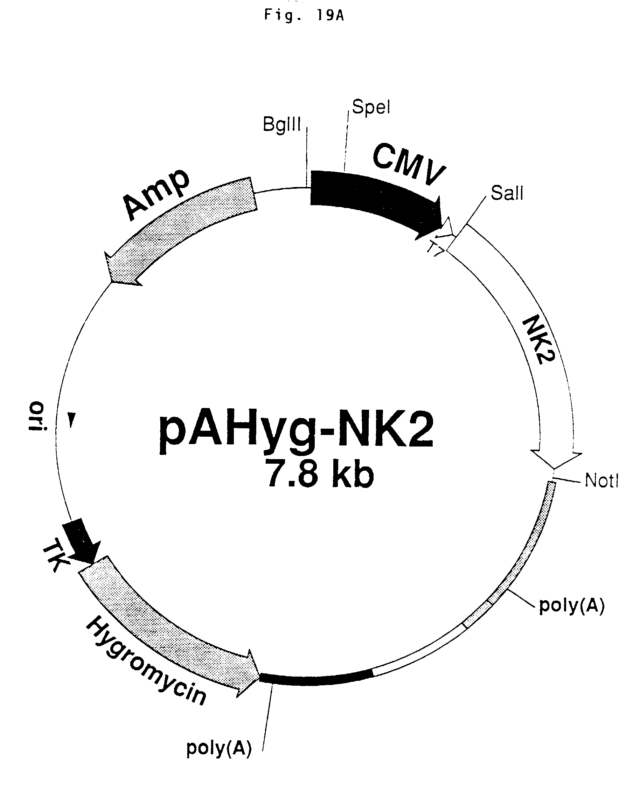

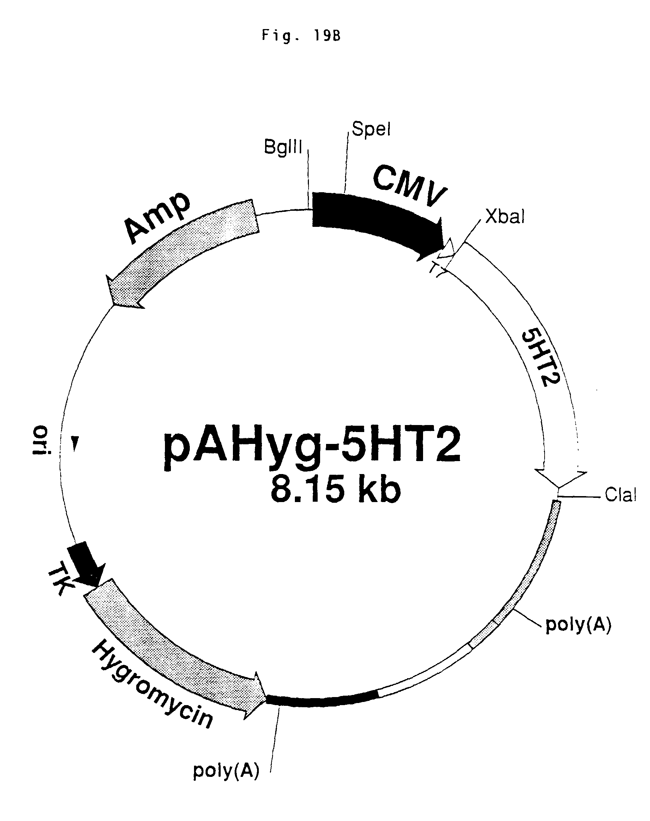

Die cDNA des humanen NK2-Rezeptors wurde mit Hilfe der Restriktionsenzyme SalI und NotI aus dem Plasmid pBluescript-NK2, das die NK2-Rezeptor cDNA im Plasmid Vektor pBluescript SK+ (Stratagene) enthält (Gerard et al., 1990), herausgeschnitten und in den Expressionsvektor pAHygCMVl (s. Beispiel 1 h)) kloniert und das dadurch entstandene Plasmid pAHyg-NK2 genannt (Fig. 19A).
Die 5HT2-Rezeptorsequenz wurde als XbaI-ClaI Fragment aus dem oben beschriebenen Vektor pAD-CMV2-5HT2 herausgeschnitten und in den Vektor pAHygCMV2 (s. Beispiel 1h) kloniert. Das so erhaltene Konstrukt wurde pAHyg-5HT2 genannt (Fig.19B).

### Beispiel 4:

### Induktion von TRE-Sensor-DNA durch TPA in drei verschiedenen Prätestzellinien

Es wurden folgende Zellinien mit TRE-Sensor-DNA (pBHluc1.3) transient transfiziert: die humane Lungenkarzinom-Zellinie A549 (ATCC CCL 185), die humane Cervixkarzinom-Zellinie HeLa (ATCC CCL 2) und die Affennieren-Zellinie COS-7 (ATCC CRL 1651), wobei A549- und COS-7 Zellen in RPMI-1640 Medium (Gibco) und HeLa Zellen in MEM Medium mit Earle's BSS (Gibco) jeweils mit 10 % hitzeinaktiviertem fötalem Kälberserum (FCS) angesetzt und bei 37°C in 5 % CO₂ inkubiert wurden. Etwa 1x10⁷ Zellen pro Transfektion wurden mit Trypsin von der Oberfläche der Kulturschale gelöst und 5 min bei 1200 UpM bei Raumtemperatur abzentrifugiert (Heraeus Minifuge), einmal mit 5 ml serumfreiem Medium gewaschen, 5 min bei 1200 UpM zentrifugiert und in 1 ml serumfreiem Medium suspendiert. Anschließend wurden die Zellen mit 250 µg/ml DEAE-Dextran, 5 µg Plasmid DNA und 50 µg/ml Chloroquin versetzt, 30 min bei 37°C inkubiert, einmal mit Medium ohne FCS gewaschen und mit 10 ml frischem serumhaltigen Medium bei 37°C über Nacht inkubiert. Danach wurde das Medium gewechselt, und nach 4 h wurden die Zellen induziert, und zwar entweder mit 10 ng TPA/ml Medium oder mit 20 µM Forskolin. Nach weiteren 18 h wurden die Zellen mit PBS gewaschen, mit einem Gummischaber von der Petrischale gelöst und 5 min bei 1200 UpM bei Raumtemperatur abzentrifugiert (Heraeus Minifuge). Die Zellen wurden durch Zugabe von 100 µl Lysierpuffer (1 % Triton X-100, 25 mM Glycylglycin pH 7.8, 15 mM MgSO₄, 4 mM EDTA und 1 mM DDT) lysiert, das Lysat 5 min zentrifugiert und der Überstand in ein neues Röhrchen überführt. Der Luciferase-Assay (De Wet et al., 1985) wurde durchgeführt, indem 30 µl vom Überstand zu 350 µl Assay-Puffer (25 mM Glycylglycin pH 7.8, 5 mM ATP, 15 mM MgSO₄) zugegeben, das Röhrchen in das Luminometer Lumat 9501 (Berthold) gestellt und die Reaktion durch Injektion von 300 µl Injektionspuffer (0.2 mM Luciferin, 20 mM Glycylglycin pH 7.8) gestartet wurde. Die Meßzeit der Lichtemission betrug 10 sec.

### a) Induktion von pBHluc1.3 durch TPA, aber nicht durch Forskolin

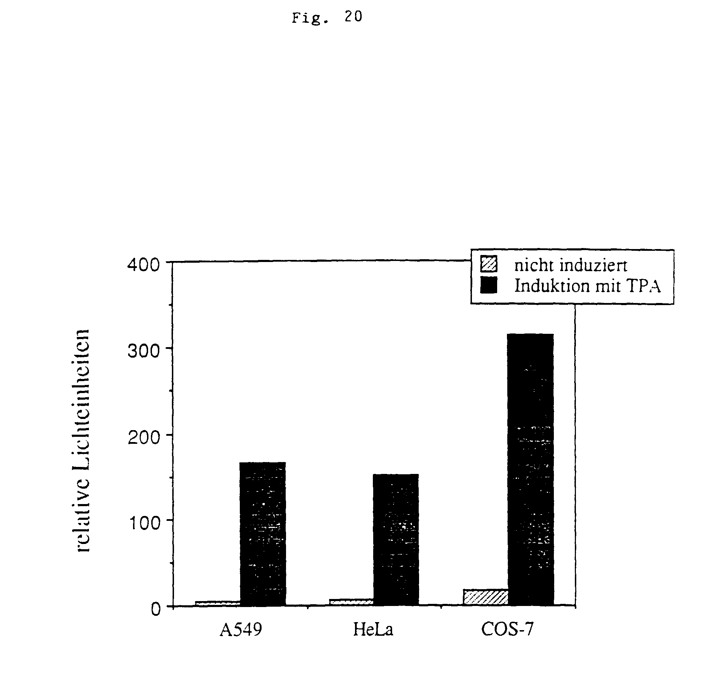

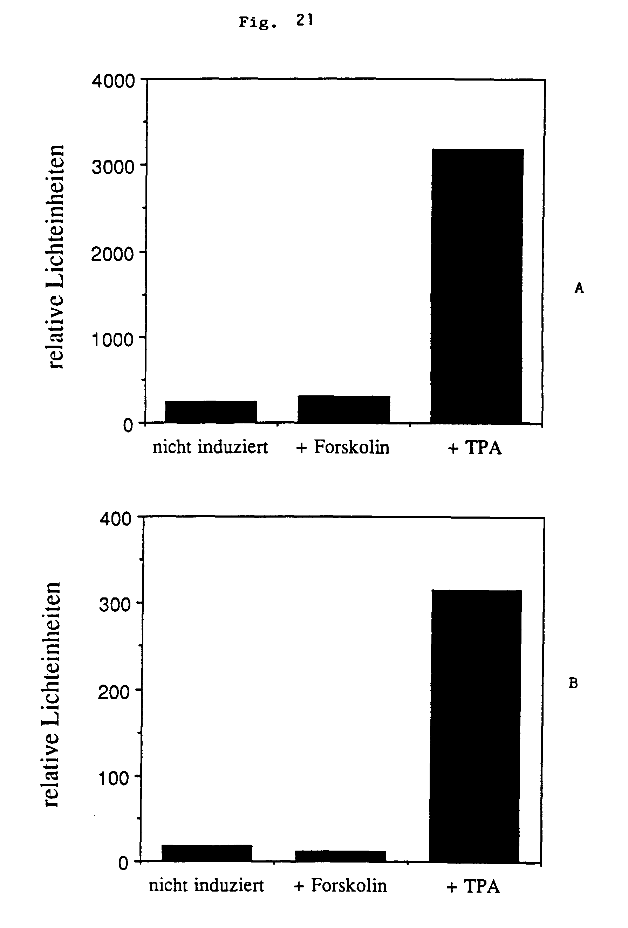

Die Zellinien A549, HeLa und COS-7 wurden durch Zugabe von Plasmid pBHluc1.3 transient transfiziert, wie oben beschrieben, und durch Zugabe von TPA induziert. Als negative Kontrolle dienten Zellen, die nur transfiziert, aber nicht induziert wurden. Nach der angegebenen Inkubationszeit wurden die Zellen lysiert und der Luciferase-Assay durchgeführt. Das Ergebnis des Experiments ist in Fig. 20 zu sehen und zeigt, daß Plasmid pBHlucl.3 in den getesteten Zellen mehr als 10-fach induzierbar ist. Voraberger et al., 1991, haben gezeigt, daß dieses Konstrukt in A549 Zellen nicht durch Forskolin induziert werden kann. Um dieses Ergebnis auch für HeLa und COS-7 Zellen zu verifizieren, wurden diese Zellen in einem weiteren Experiment wieder mit pBHluc1.3 transfiziert und entweder mit TPA oder mit Forskolin induziert, wobei wieder nicht-induzierte Zellen als Kontrolle dienten. Als Ergebnis dieser Versuche konnte auch für HeLa und COS-7 Zellen gezeigt werden, daß diese zwar durch TPA, aber nicht durch Forskolin induzierbar sind, wie in den Fig. 21A und 21B zu sehen ist.

### b) Induktion von pADneo(3TRE)BGluci durch TPA, aber nicht durch Forskolin

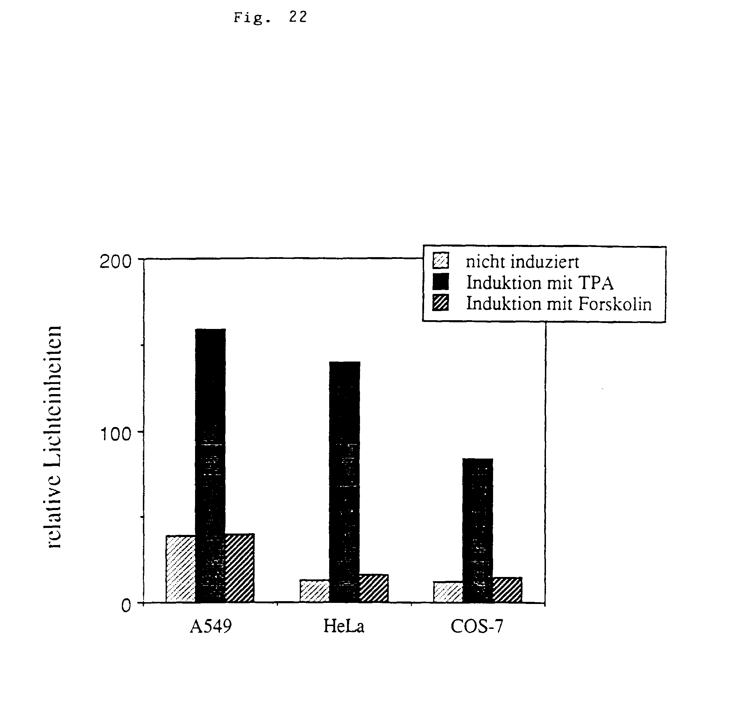

Die Zellinien A549, HeLa und COS-7 wurden durch Zugabe von Plasmid pADneo(3TRE)BGluci transient transfiziert und durch Zugabe von TPA oder Forskolin induziert. Als negative Kontrolle dienten wieder Zellen, die nur transfiziert, aber nicht induziert worden waren. Nach der angegebenen Inkubationszeit wurden die Zellen lysiert und der Luciferase-Assay durchgeführt. Das Ergebnis des Experiments ist in Fig. 22 zu sehen und zeigt, daß auch dieser Vektor, der nur die TRE-Elemente des ICAM-1 Gens enthält, in den getesteten Zellen durch TPA, nicht aber durch Forskolin induzierbar ist.

### c) Induktion der Plasmide pADneo(nTREdx)BGluci durch TPA, aber nicht durch Forskolin

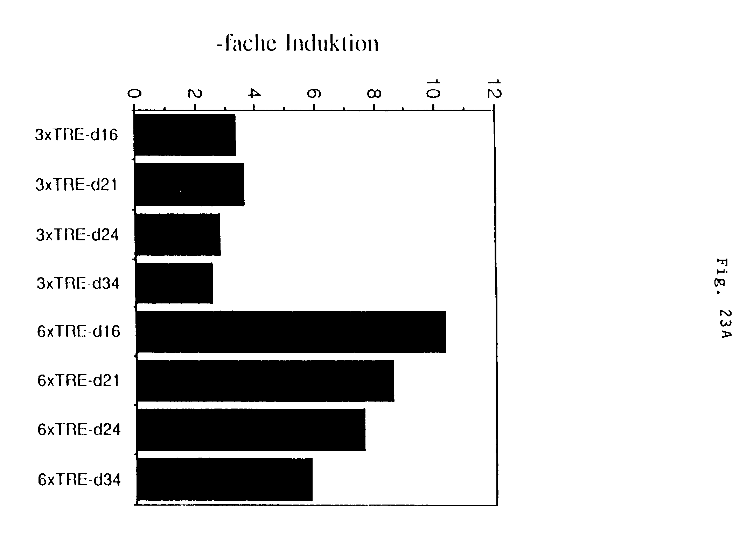

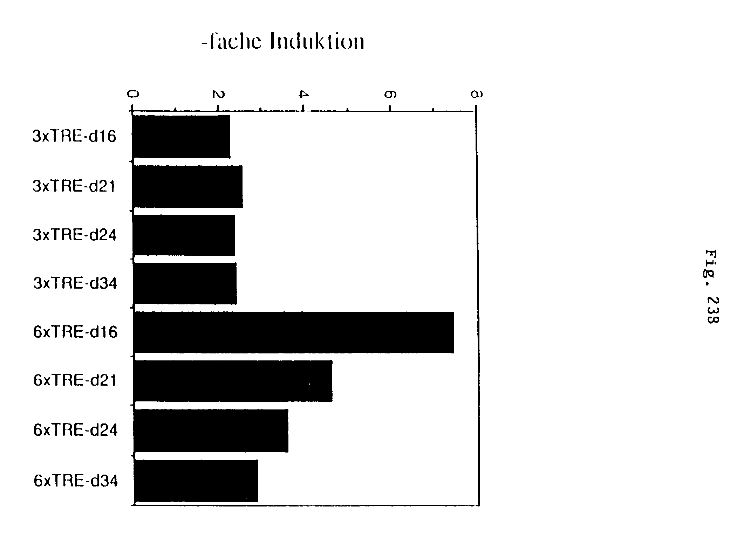

Die Zellinien COS-7 und A549 wurden durch Zugabe von Plasmid
pADneo(3xTREd16)BGluci, pADneo(3xTREd21)BGluci,
pADneo(3xTREd24)BGluci, pADneo(3xTREd34)BGluci,
pADneo(6xTREdl6)BGluci, pADneo(6xTREd21)BGluci,
pADneo(6xTREd24)BGluci oder pADneo(6xTREd34)BGluci
transient transfiziert und durch Zugabe von TPA oder Forskolin induziert. Als negative Kontrolle dienten wieder Zellen, die nur transfiziert, aber nicht induziert wurden. Nach der angegebenen Inkubationszeit wurden die Zellen lysiert und der Luciferase-Assay durchgeführt. Das Ergebnis des Experiments ist in Fig. 23A und 23B zu sehen und zeigt, daß i) die Induktion mit TPA für die Konstruktionen mit 6 TRE-Elementen höher ist als für die Konstruktionen mit 3 TRE-Elementen (keine der Konstruktionen ist mit Forskolin induzierbar), und ii) die Induktion mit TPA für die Konstruktionen mit 6 TRE-Elementen bei geringerem Abstand der TRE-Elemente höher ist als bei größerem Abstand der TRE-Elemente.

### Beispiel 5

### Rezeptor-vermittelte Induktion der TRE-Sensor-DNA

Um zeigen zu können, daß mit TPA induzierbare Sensor-DNA auch dann induziert werden kann, wenn die Zellinie einen Rezeptor an der Oberfläche exprimiert, der über G-Proteine an das Phospholipase C-Effektor-System gekoppelt ist, und ein rezeptorspezifischer Agonist zu einem geeigneten Zeitpunkt zugegeben wird, wurden COS-7 Zellen mit TRE-Sensor-DNA und Rezeptor-DNA co-transfiziert. Die Verwendung von COS-7 Zellen und einer Rezeptor-DNA, die den SV40-Replikationsursprung enthält, ermöglichte die autonome Replikation der Rezeptor-DNA zu hohen Kopienzahlen und damit hohe Raten für die transiente Expression des Rezeptors auf der Zelloberfläche. Die Co-Transfektion erfolgte nach der in Beispiel 4 beschriebenen DEAE-Dextran-Methode mit dem Unterschied, daß je 5 µg TRE-Sensor-DNA und Rezeptor-DNA transfiziert wurden. In dieser Versuchsreihe wurden, nach Inkubation über Nacht und Wechseln des Mediums, in parallelen Experimenten entweder rezeptorspezifische Agonisten, oder Agonist und kompetitiver Antagonist, oder TPA als positive Kontrolle zugegeben. Als negative Kontrolle dienten wieder nicht-induzierte Zellen. Nach 18-stündiger Inkubationszeit wurden die Zellen lysiert und der Luciferase-Assay durchgeführt, wie in Beispiel 4 beschrieben.

### a) Induktion von pADneo(3TRE)BGluci durch Bindung einer agonistisch wirkenden Substanz an den muskarinischen M3-Rezeptor

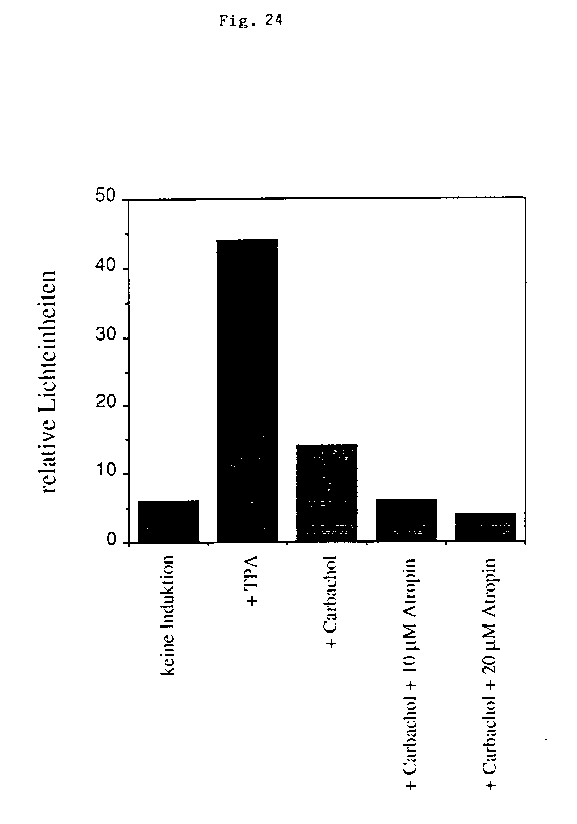

COS-7 Zellen wurden durch Zugabe von Sensor-DNA pADneo(3TRE)BGluci und von Rezeptor-DNA pCD-M3 (Buckley et al., 1989), die die Sequenz des humanen muskarinischen M3-Rezeptors im Okayama/Berg pCD Expressionsvektor (Okayama und Berg, 1983) enthält, co-transfiziert. Die Induktion erfolgte mit i) 10 ng/ml TPA (Sigma P8139), ii) 1 mM Carbachol (Sigma, C4382), iii) 1 mM Carbachol und 10 µM Atropin (Sigma A0132), und iv) 1 mM Carbachol und 20 µM Atropin. Das Ergebnis des Luciferase-Assays nach Inkubation und Lyse der Zellen ist in Fig. 24 zu sehen und zeigt, daß die Expression der Luciferase sowohl durch TPA als auch durch Carbachol, einen Agonisten für den muskarinischen Rezeptor, induziert wird. Die durch den Agonisten vermittelte Induktion wird durch gleichzeitige Zugabe des selektiven Antagonisten Atropin verhindert.

### b) Induktion von pBHluc1.3, aber nicht von pADneo2-C6-BGL durch Bindung einer agonistisch wirkenden Substanz an den Serotonin-5-HT₂-Rezeptor

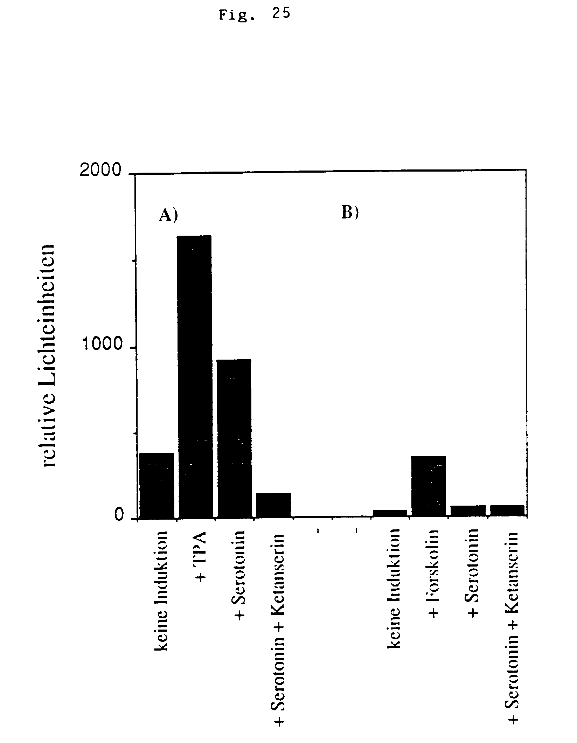

COS-7 Zellen wurden durch Zugabe von Sensor-DNA pBHluc1.3 und von Rezeptor-DNA pAD-CMV2-5HT₂, die die Sequenz des humanen 5HT₂-Rezeptors im Expressionsvektor pAD-CMV2 (siehe Beispiel 3) enthält, co-transfiziert. Die Induktion erfolgte mit i) 10 ng/ml Medium TPA (Sigma P8139), ii) 10 µM α-Methylserotonin-Maleat (RBI Research Biochemicals Incorporated M-110), und iii) 10 µM α-Methylserotonin-Maleat und 10 µM Ketanserin-Tartrat (RBI S-006). Das Ergebnis des Luciferase-Assays nach Inkubation und Lyse der Zellen ist in Fig. 25A) zu sehen und zeigt, daß die Expression der Luciferase sowohl durch TPA als auch durch α-Methylserotonin-Maleat, einen Agonisten für den 5-HT₂-Rezeptor, induziert wird. Die durch den Agonisten vermittelte Induktion wird durch gleichzeitige Zugabe des selektiven Antagonisten Ketanserin-Tartrat verhindert. In einem parallelen Experiment wurden COS-7 Zellen durch Zugabe von Sensor-DNA pADneo2-C6-BGL, die 6 CRE-Elemente enthält, und von Rezeptor-DNA pAD-CMV2-5HT₂ co-transfiziert. Die Induktion erfolgte mit i) 20 µM Forskolin (Sigma P8139), ii) 10 µM α-Methylserotonin-Maleat (RBI M-110), und iii) 10 µM α-Methylserotonin-Maleat und 10 µM Ketanserin-Tartrat (RBI S-006). Das Ergebnis des Luciferase-Assays nach Inkubation und Lyse der Zellen ist in Fig. 25B) zu sehen und zeigt, daß die Expression der Luciferase zwar durch Forskolin, aber nicht durch den 5-HT₂-Rezeptor-Agonisten α-Methylserotonin-Maleat induziert wird. Aus obigen Ergebnissen folgt, daß 5-HT₂-Rezeptor-abhängig selektiv nur Regulationselemente aktiviert werden, die auf IP₃/DAG ansprechen (TRE), aber keine Regulationselemente, die auf cAMP ansprechen (CRE).

### c) Induktion von pADneo2-C6-BGL, aber nicht von pBHluc1.3 durch Bindung einer agonistisch wirkenden Substanz an den Dopamin-D1-Rezeptor

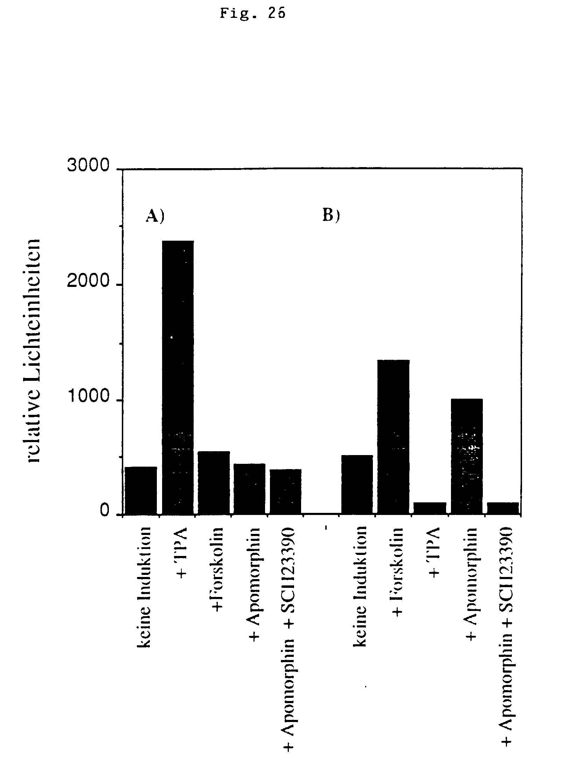

COS-7 Zellen wurden durch Zugabe von Sensor-DNA pBHluc1.3 und von Rezeptor-DNA pAD-CMV2-D1, die die Sequenz des humanen Dopamin-D1-Rezeptors im Expressionsvektor pAD-CMV2 (siehe Beispiel 3) enthält, co-transfiziert. Die Induktion erfolgte mit i) 10 ng/ml TPA (Sigma P8139), ii) 20 µM Forskolin (Sigma P8139), iii) 1 µM Apomorphin (RBI D-004), und iv) 1 µM Apomorphin und 1 uM SCH23390 (RBI D-054). Das Ergebnis des Luciferase-Assays nach Inkubation und Lyse der Zellen ist in Fig. 26A) zu sehen und zeigt, daß die Expression des Luciferase-Gens zwar durch TPA, aber nicht durch Apomorphin, einen Agonisten für den D1-Rezeptor, induziert wird. In einem parallelen Experiment wurden COS-7 Zellen durch Zugabe von Sensor-DNA pADneo2-C6-BGL, die 6 CRE-Elemente enthält, und von Rezeptor-DNA pAD-CMV2-D1 co-transfiziert und induziert. Das Ergebnis des Luciferase-Assays nach Inkubation und Lyse der Zellen ist in Fig. 26B) zu sehen und zeigt, daß die Expression der Luciferase sowohl durch Forskolin als auch durch Apomorphin induziert wird. Die durch den Agonisten vermittelte Induktion wird durch gleichzeitige Zugabe des selektiven Antagonisten SCH23390 verhindert. Aus obigen Ergebnissen folgt, daß der Dopamin-D1-Rezeptor selektiv nur Regulationselemente aktiviert, die auf das Adenylatzyklase-Signaltransduktionssystem ansprechen (CRE), aber keine Regulationselemente, die auf das Phospholipase C-Signaltransduktionssystem ansprechen (TRE).

### Beispiel 6

### a) Entwicklung von rekombinanten A549 Zellinien, die Luciferase in Abhängigkeit von der intrazellulären IP₃/DAG Konzentration exprimieren (TRE-Zellinien)

Die TRE-Sensor-DNA pBHluc1.3 hatte sich in transienten Transfektionsversuchen in der Zellinie A549 mehr als 10fach durch Zugabe von TPA induzieren lassen. (Beispiel 4a). Zur Herstellung einer stabilen (Prä-)Test-Zellinie für Substanzen, die durch direkte oder rezeptor-vermittelte Modulation des IP₃/DAG-Signaltransduktionsweges die Expression des Luciferase-Gens beeinflussen, wurden A549 Zellen gleichzeitig mit dem Plasmid pBHluc1.3 und dem Selektionsplasmid pRSVneo durch Elektroporation wie folgt transfiziert:
Die Zellen wurden nach Entfernung des Mediums mit Hilfe einer Trypsin/PBS-Lösung von der Oberfläche gelöst, in Medium suspendiert und 5 min bei 250xg sedimentiert. Die Zellen wurden in serumfreiem RPMI-1640 Medium (Gibco) gewaschen, wiederum abzentrifugiert und in einer Dichte von 1.25 x 10⁷ Zellen/ml in serumfreiem RPMI-1640 resuspendiert. 0.8 ml Zellsuspension wurde mit 20 µg pBHlucl.3 und 2 µg pRSVneo versetzt. Beide Plasmide waren zuvor mit BamHI linearisiert worden. Die Transfektion wurde mit dem PG200 Progenetor II Elektroporationsgerät (Hoefer Scientific Instr.) mit einem einzelnen Strompuls von 270 V, 1080 µF, 1000 msec durchgeführt. Anschließend wurden die Zellen in RPMI-1640 Medium, versetzt mit 10% fötalem Kälberserum, verdünnt und mit einer Dichte von 2-5 x 10⁵ Zellen pro 90 mm Kulturschale ausgesät. Vom Tag nach der Transfektion an wurden die Zellen in Selektionsmedium (RPMI-1640, angereichert mit 10% dialysiertem fötalem Kälberserum, Na-Penicillin G (100 Einheiten/ml), Streptomycin (50 Einheiten/ml) und 800 µg/ml Geneticin (G-418, Gibco-BRL)) gezüchtet.

15-20 Tage nach der Transfektion wurden einzelne Zellklone in Mikrotiterplatten mit 96 Vertiefungen überführt und weitergezüchtet. G-418 - resistente Klone wurden auf die Induzierbarkeit der Luciferaseexpression durch Zugabe von TPA getestet. Ungefähr 40000 Zellen jedes Klons wurden in 6facher Ausfertigung in 200 µl pro Vertiefung in einer lichtundurchlässigen, gewebekulturbeschichteten Mikrotiterplatte mit 96 Vertiefungen (Microlite™, Dynatech Laboratories) ausgesät und über Nacht bei 37°C inkubiert. Jeweils drei Ansätze wurden mit 10ng TPA/ml behandelt und weitere 8h inkubiert. Anschließend wurde das Medium entfernt und die Zellen zweimal mit PBS gewaschen. Die Zellen wurden in 150 µl Lyse-Puffer (25mM Tricine, 0.5 mM EDTA, 0.54 mM Natriumtripolyphosphat, 6.5 mM DTT, 16.3 mM MgSO₄.7H₂O, 0.1% Triton X-100, 1.2 mM ATP, 0.05 mM Luciferin; pH7.8) pro Ansatz aufgenommen und die Luciferaseaktivität in einem 96well Luminometer (ML-1000, Dynatech) gemessen.
Zellklon A20 wurde für weitere Experimente ausgewählt, weil er einen meßbaren Basiswert an Luciferaseaktivität sowie eine 15-20fache Induzierbarkeit durch TPA aufwies.

### b) Entwicklung von rekombinanten A549 Test-Zellinien, . die Luciferase in Abhängigkeit von der Aktivierung des humanen Neurokinin 2-Rezeptors exprimieren

Dieses Beispiel zeigt die Herstellung einer Test-Zellinie für den humanen Neurokinin 2 (NK2)-Rezeptor, der an den Phospholipase C-Signaltransduktionsweg gekoppelt ist. Diese Test-Zellinie ermöglicht über die Messung der Luciferaseaktivität die Identifizierung von Substanzen, die rezeptorabhängig die intrazelluläre IP₃/DAG Konzentrationen modulieren.

Die Prä-Test-Zellinie A20 wurde, wie unter a) beschrieben, durch Elektroporation mit dem Plasmid pAHyg-NK2, das zuvor mit BglII linearisiert worden war, transfiziert. Vom Tag nach der Transfektion an wurden die Zellen in Selektionsmedium, wie für die Zellinie A20 verwendet und zusätzlich angereichert mit 150 µg/ml Hygromycin B (Sigma), gezüchtet. Einzelklone wurden wie unter a) beschrieben auf Induzierbarkeit der Luciferaseaktivität getestet. In diesem Fall wurde jedoch Neurokinin A (1 µM, Sigma) anstelle von TPA als Induktor verwendet. Klon A20/NK2-122 zeigte in wiederholten Experimenten eine 5-7fache Induktion der Luciferaseaktivität nach Aktivierung des Neurokinin 2-Rezeptors.

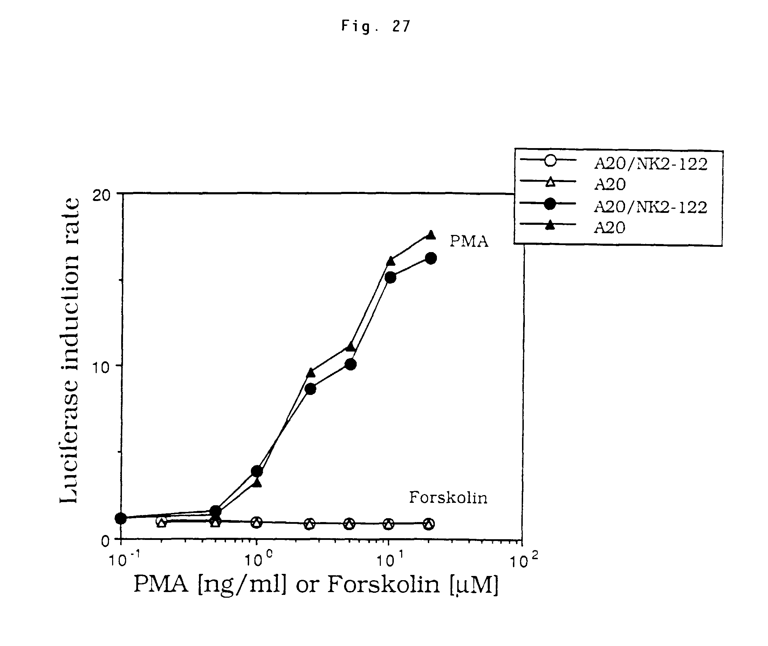

Wie in Fig. 27 gezeigt, wird in der Prä-Test-Zellinie A20 und ebenso in der NK2-Test-Zellinie A20/NK2-122 die Expression der Luciferase nur über den IP₃/DAG-Signaltransduktionsweg erhöht, nicht jedoch durch Erhöhung der intrazellulären cAMP Konzentration. Während in beiden Zellinien die Luciferaseaktivität durch TPA dosis-abhängig bis maximal etwa 18fach induzierbar war, bewirkte Forskolin (Stimulator der Adenylatzyklase) keine Induktion der Luciferaseaktivität.

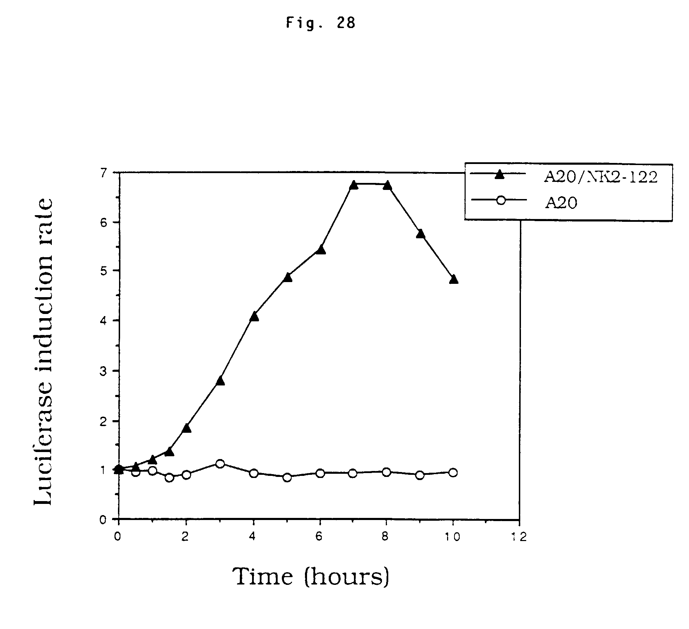

Die Kinetik der Luciferase-Induktion mittels des NK2-spezifischen Agonisten NKA GR64349 (1µM, Neosystem S.A.) in der Test-Zellinie A20/NK2-122 ist in Fig. 28 gezeigt. Die maximale Luciferaseaktivierung wurde nach einer Induktionszeit von 7-8h gemessen. In der Zellinie A20 ließ sich dagegen die Luciferaseaktivität nicht durch Zugabe des NK2-Agonisten GR64349 induzieren. Das bedeutet, daß die Prä-Test-Zellinie A20 keine endogenen NK2-Rezeptcrmoleküle enthält und somit eine geeignete Kontroll-Zellinie für die NK2-Test-Zellinie ist.

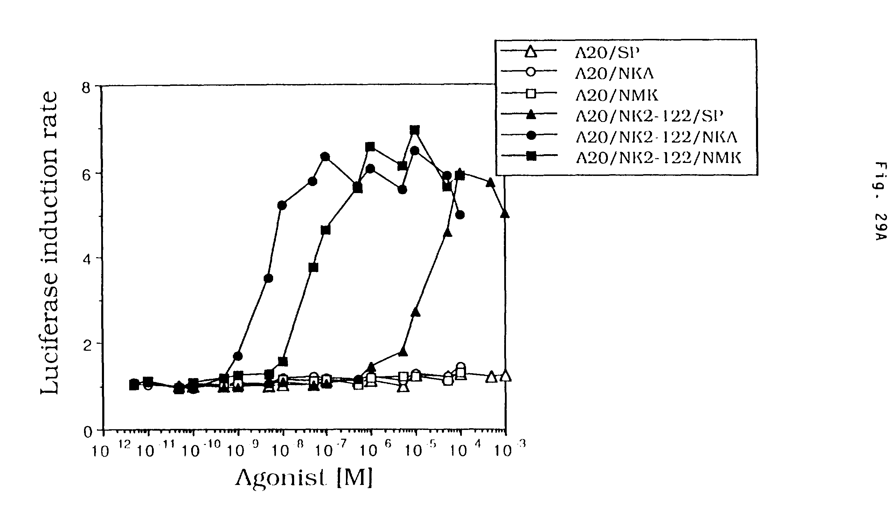

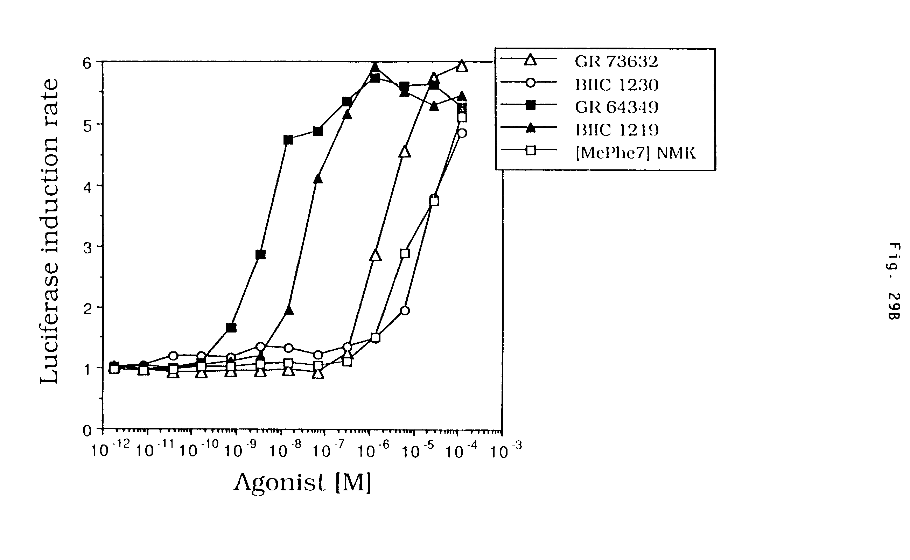

Fig. 29A zeigt die Dosis-Wirkungskurven der Luciferaseaktivität in Abhängigkeit von der Aktivierung des humanen Neurokinin 2-Rezeptors durch Neurokinine. Die relative Wirksamkeit der Neurokinine (Sigma), NKA > Neuromedin K (NMK) > Substanz P (SP), in der Zellinie A20/NK2-122 stimmt überein mit bereits in der Literatur beschriebenen Daten aus Rezeptor-Bindungsstudien. Die Zellinie A20 ließ sich durch keines der drei Neurokinine induzieren, das heißt, daß sie keinen der Neurokinin-Rezeptoren endogen enthält.

Die dosis-abhängige Wirkung einer Reihe von Agonisten, die für jeweils einen der drei Neurokinin-Rezeptoren spezifisch sind: NK1: GR73632 (Neosystem S.A.), BIIC 1230 ([β-Ala⁴,Sar⁹,Met(O₂)¹¹]SP(4-11)); NK2: GR64349 (Neosystem S.A.), BIIC 1219 ([β-Ala⁸]NKA(4-10)); NK3: [MePhe⁷]NMK (Bachem Feinchemikalien AG), ist in Fig. 29B abgebildet.

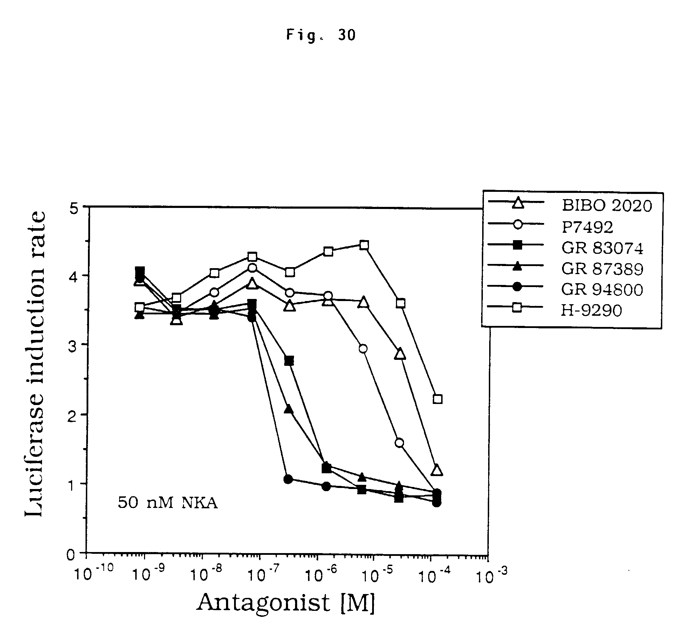

Die agonistische Wirkung von Neurokinin A in der Zellinie A20/NK2-122 konnte durch Zugabe von NK2-spezifischen Antagonisten (GR83074, GR87389, GR94800, Neosystem S.A.) inhibiert werden (Fig. 30). Dazu wurden die Zellen gleichzeitig mit einer konstanten Menge Neurokinin A (50 nM) und ansteigenden Mengen Antagonist behandelt. NK1-spezifische Antagonisten, (d.h.BIBO 2020 (±cis-3-(2-methoxybenzylamino)-2-benzhydrylquinuclidine), P7492 (Peninsula Lab.)) oder NK3-spezifische Antagonisten (H-9290, Bachem Feinchemikalien AG) waren hingegen erst bei höheren Konzentrationen wirksam (P7492 > BIBO 2020 > H-9290).

### c) Entwicklung von A549 Zellinien, die Luciferase in Abhängigkeit von der Aktivierung des humanen 5HT2-Rezeptors exprimieren

Der humane 5HT2-Rezeptor ist ebenfalls ein Beispiel eines an den Phospholipase C-Signaltransduktionsweg gekoppelten Rezeptors. Die TRE-Zellinie A20 eignet sich daher auch in diesem Fall als Ausgangszellinie zur Etablierung einer Test-Zellinie zur Auffindung von Substanzen, die die Aktivität des 5HT2-Rezeptors modulieren.

Das Plasmid pAHyg-5HT2, linearisiert mit BglII, wurde dazu wie zuvor unter a) beschrieben, durch Elektroporation in die Zellinie A20 transfiziert und in Selektionsmedium gezüchtet. Hygromycin B-resistente Zellklone wurden auf Induzierbarkeit der Luciferaseaktivität nach Zugabe des 5HT2-spezifischen Agonisten α-Methylserotonin-Maleat (10 µM, Research Biochemicals Inc.) getestet, und der Klon A20/5HT2-11, in dem eine 4-5fache Induktion gemessen wurde, für weitere Experimente ausgewählt.

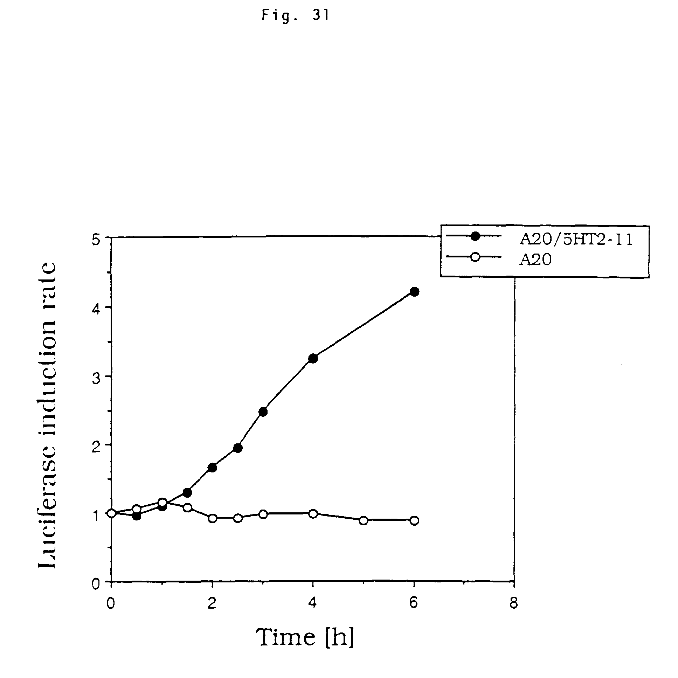

Die Kinetik der Luciferase-Induktion mittels Serotonin (1 µM) in der Test-Zellinie A20/5HT2-11 ist in Fig. 31 gezeigt. Wie in Fig. 28 steigt die Luciferaseaktivität bis zu einer Induktionszeit von 6 h kontinuierlich an. Eine Induktionszeit von 6 bis 8 h ist daher ausreichend zum Testen von agonistisch bzw. antagonistisch wirkenden Substanzen. In der Zellinie A20 ließ sich die Luciferaseaktivität nicht durch Zugabe von Serotonin induzieren. Das bedeutet, daß die Prä-Test-Zellinie A20 keine endogenen 5HT2-Rezeptormoleküle enthält und somit eine geeignete Kontroll-Zellinie für die 5HT2-Test-Zellinie ist.

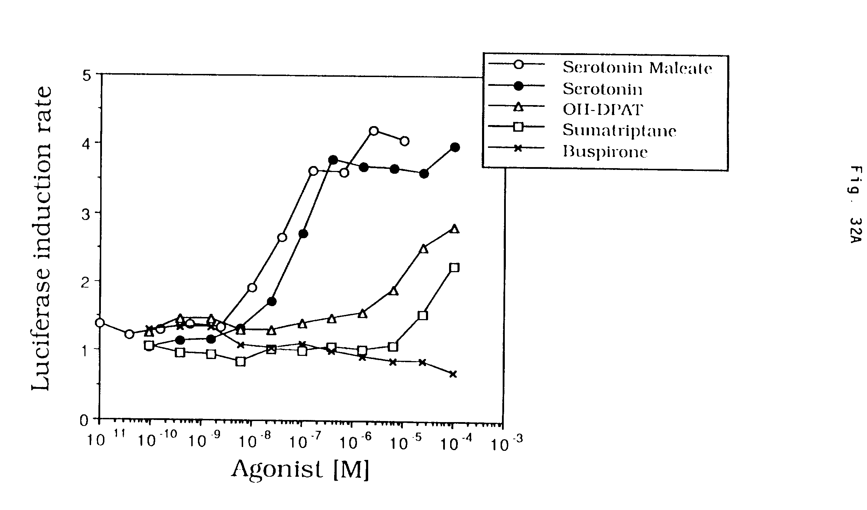

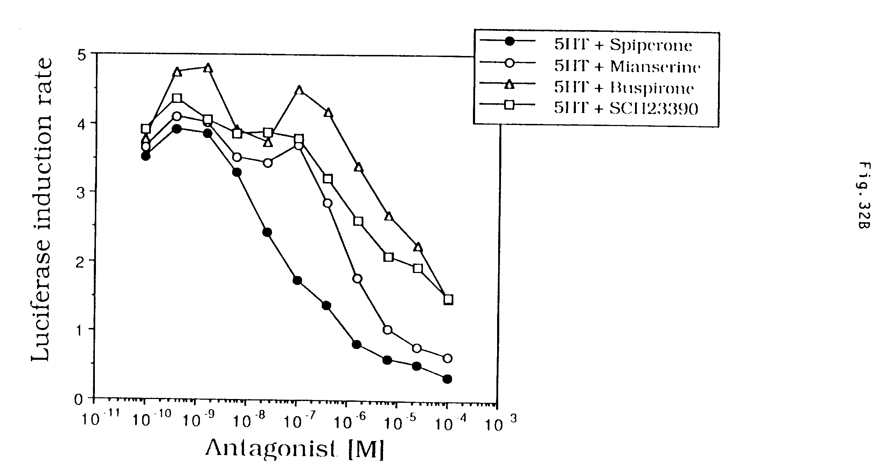

Fig. 32A zeigt die Dosis-Wirkungskurven der Luciferaseaktivität in Abhängigkeit von der Aktivierung des humanen 5HT2-Rezeptors durch Agonisten. Wie erwartet sind die Dosis-Wirkungskurven selektiv für Serotonin und den 5HT2-Rezeptor Agonisten Serotonin-Maleat im Gegensatz zu den 5HT1A-Rezeptor Agonisten 8OH-DPAT (Research Biochemicals Inc.) und Buspirone.

Die agonistische Wirkung von Serotonin in der Zellinie A20/5HT2-11 konnte durch Zugabe der 5HT2-spezifischen Antagonisten Spiperone bzw Mianserine (Research Biochemicals Inc.) inhibiert werden (Fig. 32B). Dazu wurden die Zellen gleichzeitig mit einer konstanten Menge Serotonin (1 µM) und ansteigenden Mengen Antagonist behandelt.

### Beispiel 7

### a) Entwicklung von rekombinanten "Chinese hamster ovary (CHO)"-Zellinien, die Luciferase in Abhängigkeit von der intrazellulären cAMP-Konzentration exprimieren (CRE-Zellinien)

Für die Herstellung von (Prä)-Testzellen zur Untersuchung von Substanzen, die den intrazellulären cAMP Spiegel direkt oder durch Wechselwirkung mit Rezeptormolekülen indirekt beeinflussen, wurde die "Chinese hamster ovary" Zellinie CHO-DXB11 (Urlaub und Chasin, 1980) mit der Sensor-DNA pADneo2-C6-BGL transfiziert.

Die parentale Zellinie CHO-DXB11 wurde in Roswell Park Memorial Institute (RPMI) Medium 1640 (Gibco), supplementiert mit 10 % fötalem Rinderserum (Sebak), Hypoxanthin (100 µM), Thymidin (16 µM), Natrium-Penicillin G (100 Einheiten/ml), und Streptomycin (50 Einheiten/ml), gezüchtet. Einen Tag vor der Transfektion wurden die Zellen in frischem Medium angesetzt.

Die Transfektion mittels Elektroporation wurde wie folgt durchgeführt: Die Zellen wurden nach Entfernen des Mediums mit Hilfe einer Trypsin/PBS-Lösung von der Oberfläche gelöst, in Medium suspendiert und 5 min bei 250x *g* pelletiert. Die Zellen wurden in HBS (10 mM HEPES pH 7.4, 150 mM NaCl) gewaschen und durch Zentrifugation pelletiert. Die Zellen wurden in einer Dichte von 1 x 10⁷ Zellen/ml in HBS suspendiert. 0.8 ml Zellsuspension wurde mit 20 µg DNA des mit ScaI linearisierten Plasmids pADneo2-C6-BGL versetzt und in eine Elektroporationsküvette überführt. Die Transfektion wurde mit dem PG200 Progenetor II Elektroporationsgerät (Hoefer Scientific Instruments, San Francisco, CA) mit einem einzelnen Strompuls von 320 V, 1080 µF, 1000 msec durchgeführt. Nach der Elektroporation wurden die Zellen im oben genannten Medium verdünnt, 20.000 Zellen pro 90 mm Kulturschale ausgesät und über Nacht bei 37°C inkubiert. Vom Tag nach der Transfektion an wurden die Zellen mit Selektionsmedium (RPMI 1640 Medium supplementiert mit 10 % fötalem Rinderserum, Hypoxanthin (100 µM), Thymidin (16 µM), Natrium-Penicillin G (100 Einheiten/ml), und Streptomycin (50 Einheiten/ml), 700 µg/ml Geneticin (G-418, Gibco-BRL) gezüchtet und im folgenden visuell auf Zellwachstum überprüft.

Sieben bis 10 Tage nach der Transfektion wurden einzelne entstandene Zellklone in Zellkultur-Platten mit 24 Vertiefungen überführt und nachfolgend weitergezüchtet. 25 isolierte G-418 resistente Zellklone wurden auf die Induzierbarkeit der Luciferaseexpression durch Aktivierung der Adenylatzyklase getestet.

Ungefähr 300.000 Zellen jedes Klons wurden in vierfacher Ausfertigung in je eine Vertiefung einer Zellkultur-Platte mit 6 Vertiefungen ausgesät und 24 h bei 37°C inkubiert. Jeweils zwei Ansätze wurden mit 20 µM Forskolin behandelt und weitere 5 h bei 37°C inkubiert. Anschließend wurde das Medium von allen Zellen entfernt und die Zellen mit PBS gewaschen. Die Zellen wurden mit 1 % Triton X-100 lysiert und die Luciferaseaktivität in einem Berthold Lumat LB9501 Luminometer bestimmt (Brasier et al., 1989). Zellklon C6-13 wurde für weitere Experimente ausgewählt, weil dieser den höchsten Basiswert an Luciferaseaktivität bei gleichzeitig sehr hoher Induzierbarkeit durch Forskolin aufwies.

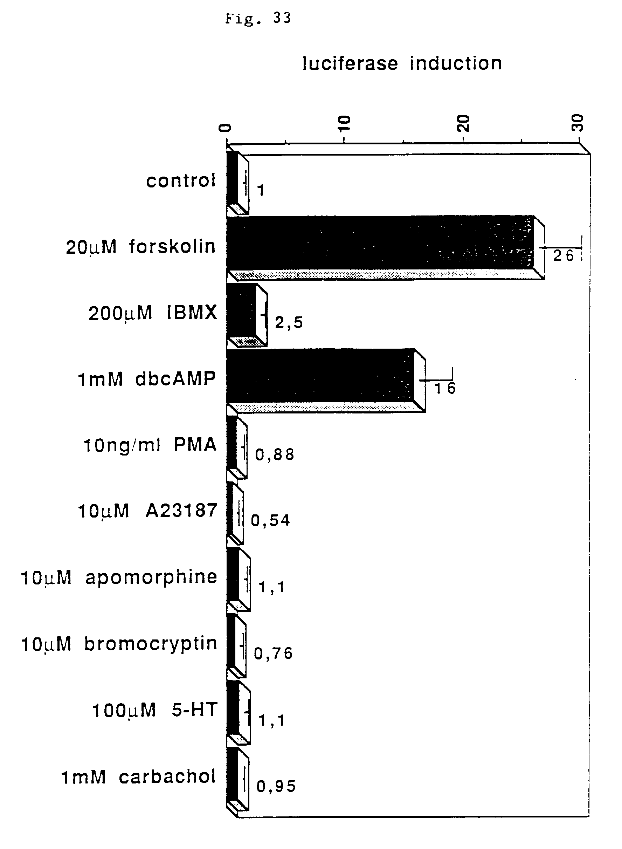

Zellen der Zellinie CHO C6-13 wurden 3 h lang mit verschiedenen Substanzen, die die intrazelluläre Konzentrationen von cAMP oder von IP₃/DAG ändern oder eine Konzentrationsänderung vortäuschen, sowie mit Rezeptor-spezifischen Agonisten behandelt, welche die genannten Signalübertragungsmechanismen beeinflussen. Wie in Fig. 33 gezeigt, wird in der Zellinie CHO C6-13 die Expression der Luciferase nur durch cAMP erhöhende Substanzen wie Forskolin (Stimulator der Adenylatzyklase), Dibutyryl-cAMP (membranpermeables cAMP-Derivat) und Isobutylmethylxanthin (IBMX, Phosphodiesterase-Inhibitor) erhöht. Phorbolester PMA, Ca²⁺-Ionophor A23187, sowie agonistische Verbindungen für Dopamin-Rezeptoren (Apomorphin, Bromocryptin), muskarinische Acetylcholin-Rezeptoren (Carbachol) und Serotonin bewirkten keine signifikante Änderung der Luciferaseaktivität. Das bedeutet, daß die Luciferaseexpression in der Zellinie CHO C6-13 nur durch die Änderung der cAMP-Konzentration moduliert wird, nicht jedoch durch die Änderung der IP₃/DAG-Konzentration, und weiters, daß die Zellen keine biologisch nachweisbaren cAMP stimulierenden Rezeptoren vom dopaminergen, muskarinischen oder Serotonin-Typ enthalten.

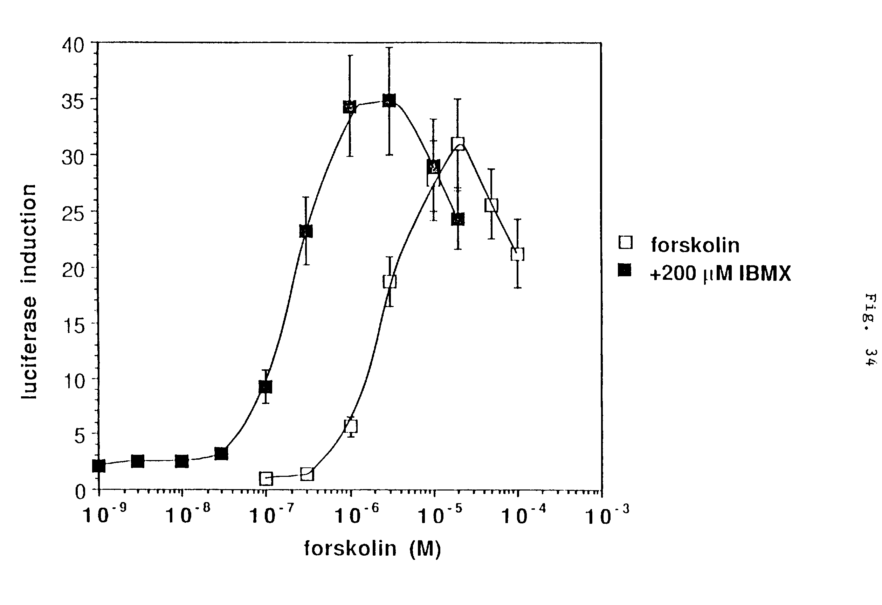

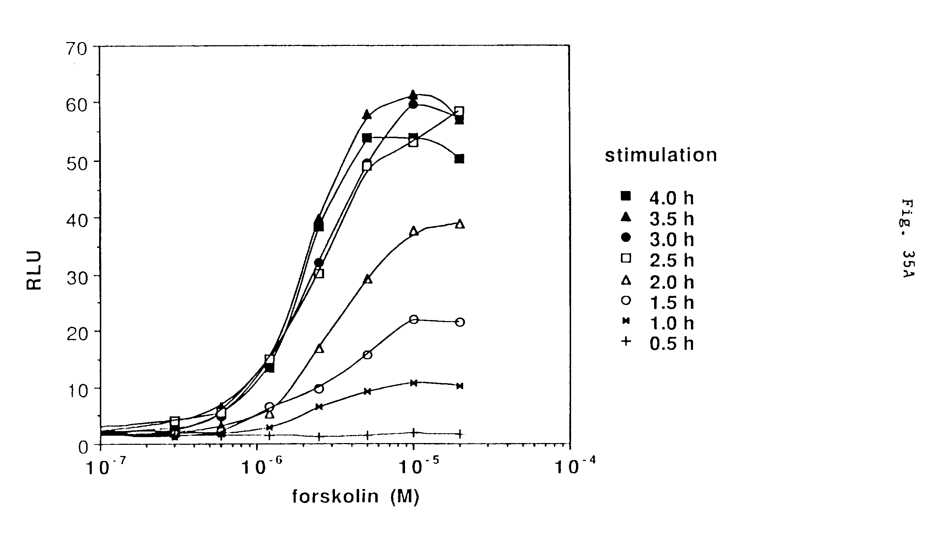

Die in Fig. 34 gezeigte Dosis-Wirkungskurve der transkriptionellen Aktivierung des Luciferase-Reportergens durch Forskolin-induzierte Erhöhung der cAMP-Konzentration ergibt maximale Induktion bei 20 µM Forskolin. Der Abfall der Luciferase-Induktion bei noch höheren Forskolinkonzentrationen dürfte auf toxische Effekte durch Forskolin selbst oder durch den überhöhten cAMP Spiegel zurückzuführen sein. Die Hemmung der Phosphodiesterase durch IBMX bewirkt einen verminderten Abbau des gebildeten cAMP und dadurch bei gleichzeitiger Aktivierung der Adenylatzyklase erhöhte cAMP Werte. Die maximale Induktion des Luciferase-Reportergens wurde durch IBMX-Behandlung nicht signifikant beeinflußt, was bedeutet, daß die maximale Aktivierung der Transkription bereits bei einer cAMP-Konzentration, bedingt durch 20 µM Forskolin, erreicht wird. Die Verschiebung der Dosis-Wirkungskurve mit IBMX um eine Größenordnung zu geringeren Forskolinkonzentrationen zeigt deutlich die Akkumulierung von cAMP durch Hemmung des für den Abbau verantwortlichen Enzyms.

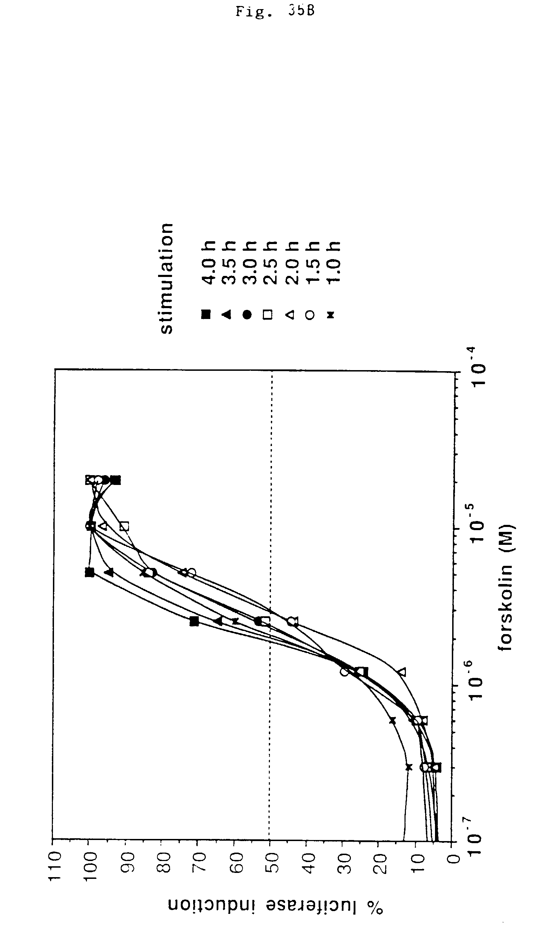

Die in Fig. 35A gezeigte Kinetik der Luciferase-Induktion in Abhängigkeit der Forskolin-Dosis ergab, daß 60 min nach Stimulation mit Fcrskolin eine erhöhte Luciferaseaktivität nachgewiesen werden kann. Volle Induktion der Luciferaseexpression wird nach 2.5 h erreicht und ändert sich nicht bis 4 h nach Forskolin-Stimulierung. Obwohl die absolute Höhe der Luciferase-Induktion bis 2.5 h zunimmt, bleiben die ED₅₀-Werte nahezu unbeeinflußt von der Dauer der Forskolin-Behandlung (Fig. 35B).

### b) Entwicklung von rekombinanten CHO-Test-Zellinien, die Luciferase in Abhängigkeit von der Aktivierung des humanen Dopamin-D1-Rezeptor exprimieren

Dieses Beispiel (wie das folgende Beispiel 8) zeigt die Herstellung von (Kontroll-)CRE-Testzellen, die auch für ein zelluläres Screeningsystem für Substanzen, die spezifische, bevorzugt humane Adenlyatzyklasegekoppelte Rezeptoren modulieren, geeignet sind. Für die Herstellung von Rezeptor-DNA wurde die humane Dopamin-D1-Rezeptor-Sequenz verwendet. Die zuvor charakterisierte Zellinie CHO C6-13 wurde, wie unter a) beschrieben, durch Elektroporation mit Plasmid pAD-CMV2:D1, das zuvor mit FspI linearisiert wurde, transfektiert. Vom Tag nach der Transfektion an wurden die Zellen in Selektionsmedium für die Selektion nach Dihydrofolatreduktase (DHFR) (nukleotidfreies Medium α-MEM (Gibco), 10 % dialysiertes fötales Rinderserum (Sebak), Natrium-Penicillin G (100 Einheiten/ml), und Streptomycin (50 Einheiten/ml), 700 µg/ml Geneticin (G-418, Gibco-BRL) gezüchtet und im folgenden visuell auf Zellwachstum überprüft. 24 einzelne isolierte Zellklone wurden, wie oben beschrieben, in Zellkulturplatten mit 6 Vertiefungen gezüchtet und 4 h nach Behandlung mit 10 µM Apomorphin (Agonist für den Dopaminrezeptor) auf die Zunahme der Luciferaseaktivität im Vergleich zu unbehandelten Zellen überprüft. Klon CHO 13D1-38 zeigte in wiederholten Experimenten die höchste Zunahme der Luciferaseaktivität nach Aktivierung des Dopamin-D1-Rezeptors.

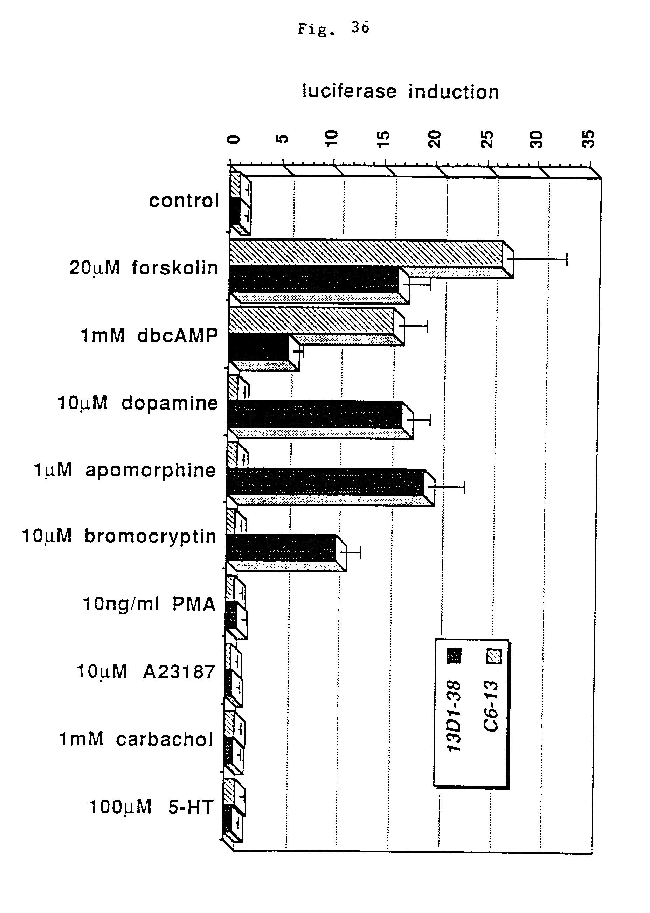

Für die Verwendung von Testzellinien in einem automatischen Screeningsystem mit hoher Durchsatzrate ist es von Vorteil, Mikrotiterplatten mit 96 Vertiefungen pro Platte zu verwenden. Dazu wurden 60.000 Zellen (CHO C6-13 oder CHO 13D1-38) in 200 µl Medium pro Vertiefung in lichtundurchlässigen, gewebekulturbeschichteten Mikrotiterplatten (Microlite™, Dynatech Laboratories) ausgesät und über Nacht bei 37°C inkubiert. Anschließend wurden den Zellen verschiedene Chemikalien zugesetzt und für eine bestimmte Zeitdauer (meist 3 h) inkubiert. Nach Entfernen des Mediums wurden die Zellen mit PBS gewaschen, in 1 % Triton X-100 lysiert und die Luciferaseaktivität in einem 96-well Luminometer (ML-1000, Dynatech) gemessen (Fig. 36).

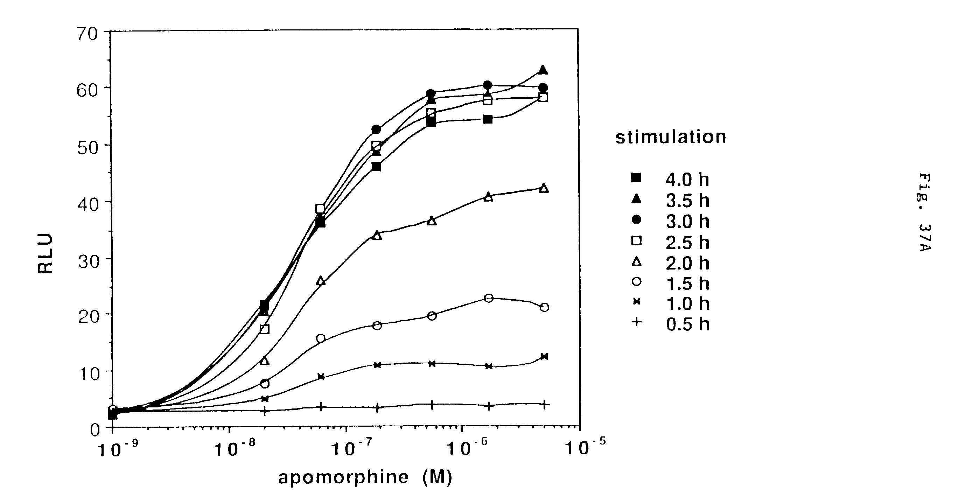

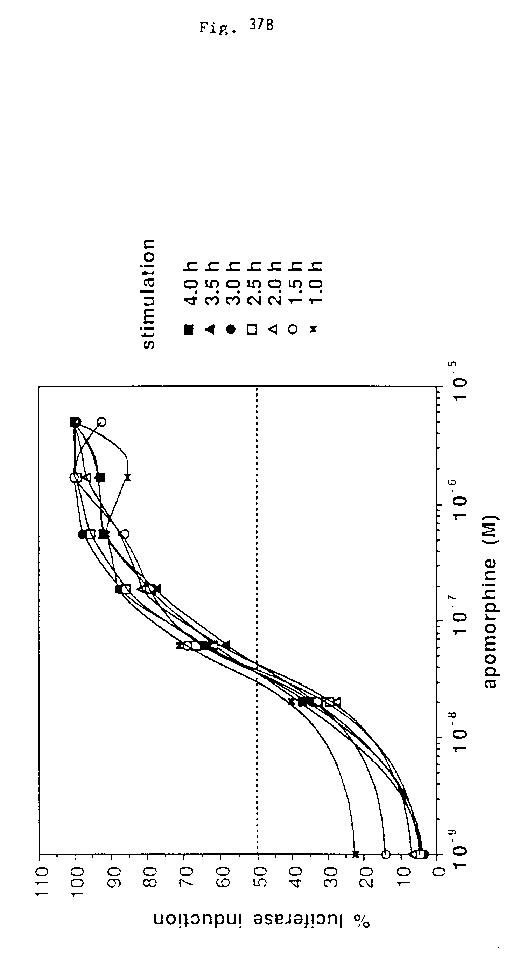

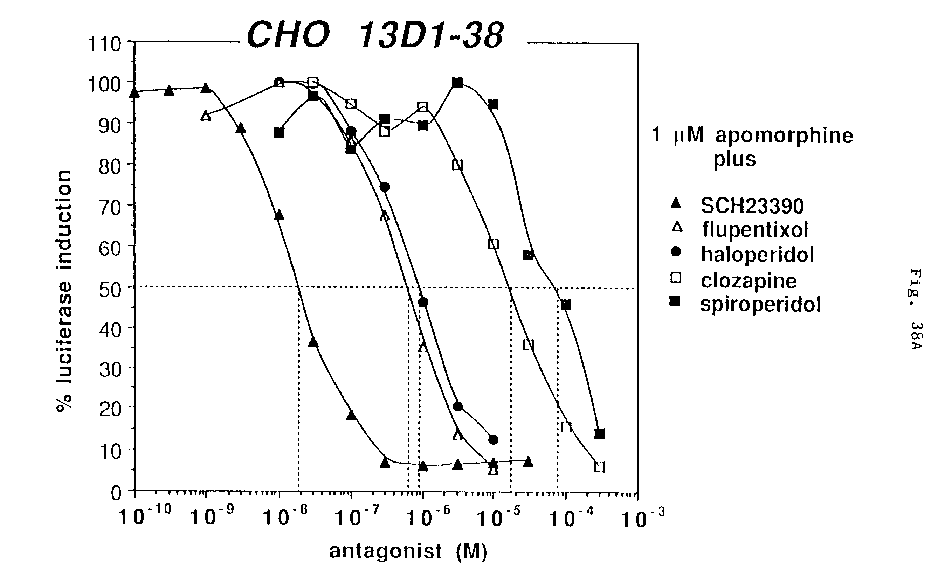

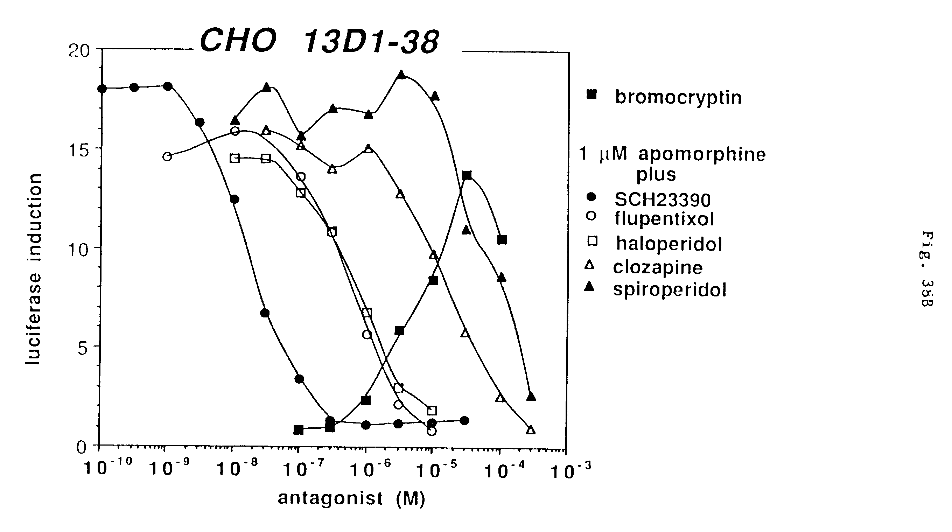

Die in den Fig. 37A und 37B und in Fig. 38A und 38B dargestellten Dosis-Wirkungskurven der Luciferaseaktivität in Abhängigkeit von der Aktivierung des humanen Dopamin-D1-Rezeptors wurden im Mikrotiterplatten-Format erstellt und repräsentieren für jeden Datenpunkt den Mittelwert aus jeweils 4 Einzelbestimmungen; die Standardabweichung betrug ca. 15 %.

In Analogie zur Kinetik der Luciferase-Induktion mittels Forskolin in der Prätestzellinie CHO C6-13 (Beispiel 7a)) wurde in der Dopamin-Dl-Rezeptor Testzellinie CHO 13D1-38 eine Stunde nach Stimulierung des D1-Rezeptors mit Apomorphin erhöhte Luciferaseaktivität gemessen (Fig. 37A). Die maximale Luciferase-Aktivierung wurde nach 2.5 h Stimulierung erreicht und blieb bis 4 h konstant. Der Vergleich mit der Kinetik nach direkter Aktivierung der Adenylatzyklase durch Forskolin (Fig. 35A) läßt den Schluß zu, daß der geschwindigkeitsbestimmende Schritt die Transkription und Translation des Luciferase-Reportergens ist, nicht jedoch die Stimulierung der Adenylatzyklase durch den aktivierten Rezeptor.

Die geänderte Auftragung der Meßwerte zur Bestimmung der ED₅₀ Werte von Apomorphin in Fig. 37B zeigte keine signifikante Abhängigkeit der ED₅₀-Werte von der Dauer der Rezeptorstimulierung mit dem Agonisten.

Fig. 38A zeigt, daß die Induktion des Reportergens durch die Aktivierung des D1-Rezeptors mit einem Agonisten (Apomorphin) dosisabhängig mit Antagonisten verhindert werden konnte. Dazu wurden die Testzellen 24 h nach dem Aussäen in die Mikrotiterplatten mit dem Antagonisten versetzt und gleich danach eine gleichbleibende Menge Apomorphin (1 µM Endkonzentration) zugesetzt, die, wie in Fig. 37A gezeigt, eine maximale Induktion des Luciferase-Reportergens bewirkt. Um einen Screeningprozeß zu simulieren, bei dem die zu untersuchenden Substanzen bevorzugt in einem einheitlichen Lösungmittel angeboten werden, wurde in diesem Versuch eine Endkonzentration von 1 % DMSO verwendet.

Die Wirksamkeit der verwendeten Substanzen in diesem Testverfahren korreliert gut mit den Daten, die in der Literatur durch Rezeptor-Bindungsstudien beschrieben wurden.

Fig. 38B zeigt dieselben Werte in anderer Auftragungsart (x-fache Induktion der Luciferase gegenüber identischen, unbehandelten Zellen als Kontrolle) und zusätzlich die dosisabhängige Wirkung des Agonisten Bromocryptin. Der Rückgang der Luciferase-Induktion bei der höchsten Bromocryptin-Konzentration (100 µM) ist auf Zelltoxizität von Bromocryptin selbst oder auf den niedrigen pH-Wert zurückzuführen, der nötig war, um Bromocyptin zu lösen.

### Beispiel 8

### a) Entwicklung von rekombinanten "Chinese hamster ovary (CHO)"-Zellinien, die Luciferase in Abhängigkeit von der intrazellulären cAMP-Konzentration exprimieren (CRE-Zellinien)

Für die Herstellung von (Prä)-Testzellen zur Untersuchung von Substanzen, die den intrazellulären cAMP Spiegel direkt oder durch Wechselwirkung mit Rezeptormolekülen indirekt beeinflussen, wurde die "Chinese hamster ovary" Zellinie CHO-DXB11 (Urlaub und Chasin, 1980) mit der Sensor-DNA pADneo2-C12-TKL transfiziert. (Dieses Plasmid unterscheidet sich von dem in Beispiel 1) beschriebenen pADneo2-C6-BGL dadurch, daß der 6CRE-Elemente enthaltende Abschnitt verdoppelt und der β-Globin-Promotor durch den TK-Promotor (s. Beispiel 1 b)) ersetzt wurden. Dazu wurde das SalI-HindIII-β-Globin-Promotor-Fragment durch ein gleichgeschnittenes Fragment, enthaltend den TK-Promotor (McKnight, 1980), ausgetauscht.)

Die parentale Zellinie CHO-DXB11 wurde in Roswell Park Memorial Institute (RPMI) Medium 1640 (Gibco), supplementiert mit 10 % fötalem Rinderserum (Sebak), Hypoxanthin (100 µM), Thymidin (16 µM), Natrium-Penicillin G (100 Einheiten/ml), und Streptomycin (50 Einheiten/ml), gezüchtet. Einen Tag vor der Transfektion wurden die Zellen in frischem Medium angesetzt.

Die Transfektion und die Austestung der Zellklone wurde wie in Beispiel 7 a) vorgenommen. Zellklon C12-32 wurde für weitere Experimente ausgewählt, weil dieser den höchsten Basiswert an Luciferaseaktivität bei gleichzeitig sehr hoher Induzierbarkeit durch Forskolin aufwies.

### b) Entwicklung von rekombinanten CHO-Test-Zellinien, die Luciferase in Abhängigkeit von der Aktivierung des humanen Dopamin-D5-Rezeptor exprimieren

Dieses Beispiel (wie das vorangegangene Beispiel 7) zeigt die Herstellung von (Kontroll-)CRE-Testzellen, die auch für ein zelluläres Screeningsystem für Substanzen, die spezifische, bevorzugt humane Adenylatzyklase-gekoppelte Rezeptoren modulieren, geeignet sind. Für die Herstellung von Rezeptor-DNA wurde die humane Dopamin-D5-Rezeptor-Sequenz verwendet.

Die zuvor charakterisierte Zellinie CHO C12-32 wurde, wie unter a) beschrieben, durch Elektroporation mit Plasmid pAD-CMV1:D5, das zuvor mit StuI linearisiert worden war, transfektiert. (Das Plasmid pAD-CMV1:D5 wurde hergestellt, indem das 1.6 kb große SalI-XbaI-Fragment von phD5-Gem, das die kodierende Region des humanen D5-Rezeptorgens enthält (Grandy et al., 1991), in den ebenso geschnittenen humanen Vektor pAD-CMV1 hineinligiert wurde.) Vom Tag nach der Transfektion an wurden die Zellen in Selektionsmedium für die Selektion nach Dihydrofolatreduktase (DHFR) (nukleotidfreies Medium α-MEM (Gibco), 10 % dialysiertes fötales Rinderserum (Sebak), Natrium-Penicillin G (100 Einheiten/ml), und Streptomycin (50 Einheiten/ml), 700 µg/ml Geneticin (G-418, Gibco-BRL) gezüchtet und im folgenden visuell auf Zellwachstum überprüft. 24 einzelne isolierte Zellklone wurden, wie oben beschrieben, in Zellkulturplatten mit 6 Vertiefungen gezüchtet und 4 h nach Behandlung mit 10 µM Apomorphin (Agonist für den Dopaminrezeptor) auf die Zunahme der Luciferaseaktivität im Vergleich zu unbehandelten Zellen überprüft. Klon CHO 32D5-39 zeigte in wiederholten Experimenten die höchste Zunahme der Luciferaseaktivität nach Aktivierung des Dopamin-D5-Rezeptors.

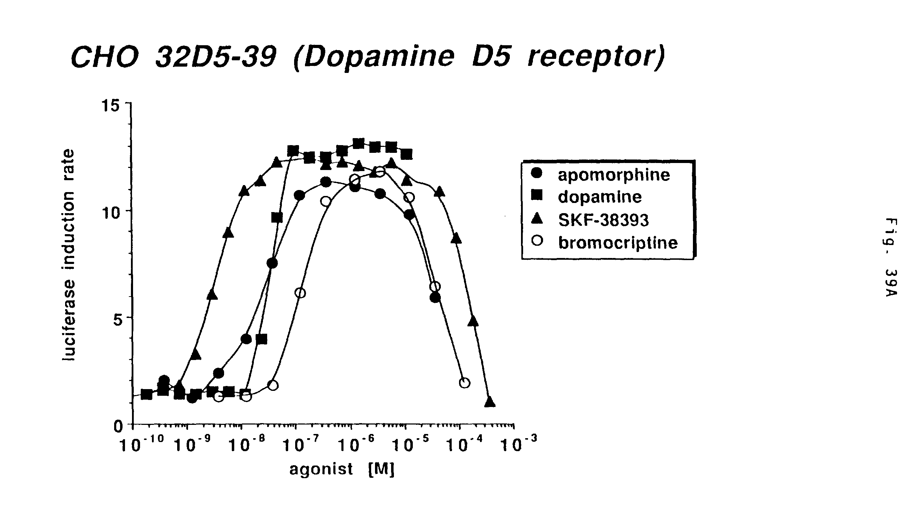

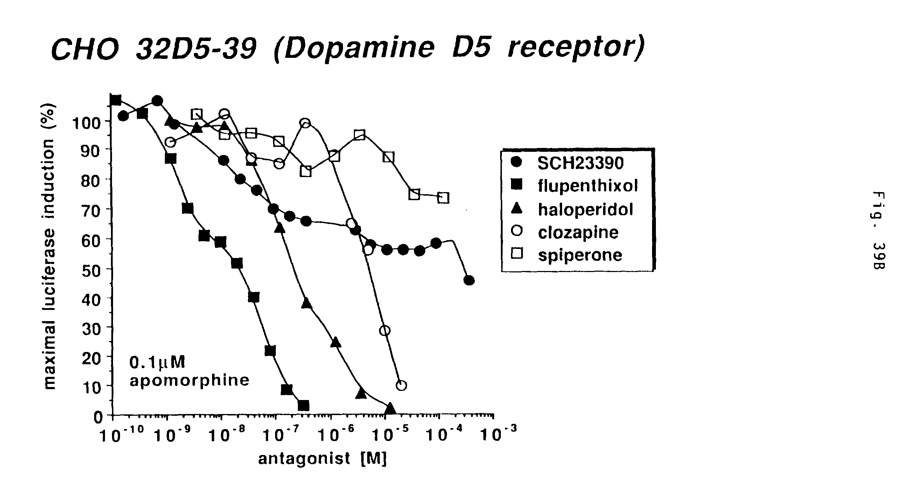

Fig. 39B zeigt, daß die Induktion des Reportergens durch die Aktivierung des D5-Rezeptors mit einem Agonisten (Apomorphin) dosisabhängig mit Antagonisten verhindert werden konnte. Dazu wurden die Testzellen 24 h nach dem Aussäen in die Mikrotiterplatten mit dem Antagonisten versetzt und gleich danach eine gleichbleibende Menge Apomorphin (0.1 µM Endkonzentration) zugesetzt, die, wie in Fig. 39A gezeigt, eine maximale Induktion des Luciferase-Reportergens bewirkt. Das Ergebnis dieser Versuche ist in Fig. 39B dargestellt.

Die Wirksamkeit der verwendeten Substanzen in diesem Testverfahren korreliert gut mit den Daten, die in der Literatur durch Rezeptor-Bindungsstudien beschrieben wurden.

### Beispiel 9

### Entwicklung eines Reagens zur Messung der Luciferaseaktivität

Es wurde der Einfluß der Variation der Konzentration von ATP, Luciferin, MgSO₄.7H₂O, Dithiothreitol (DTT), β-Mercaptoethanol (BME), Natriumtripolyphosphat (NaTPP), Triton X-100 und des pH-Werts auf das erzielte Luciferase-Meßsignal ermittelt (Tabelle 2). Die übrigen Komponenten waren jeweils in den dem bevorzugten Grundpuffer gemäß Tabelle 1 entsprechenden Konzentrationen enthalten. Es wurden die 3 Minuten nach Reagenszugabe erhaltenen Meßwerte zum Vergleich herangezogen (Angabe in Prozent vom maximal erzielten Meßsignal).

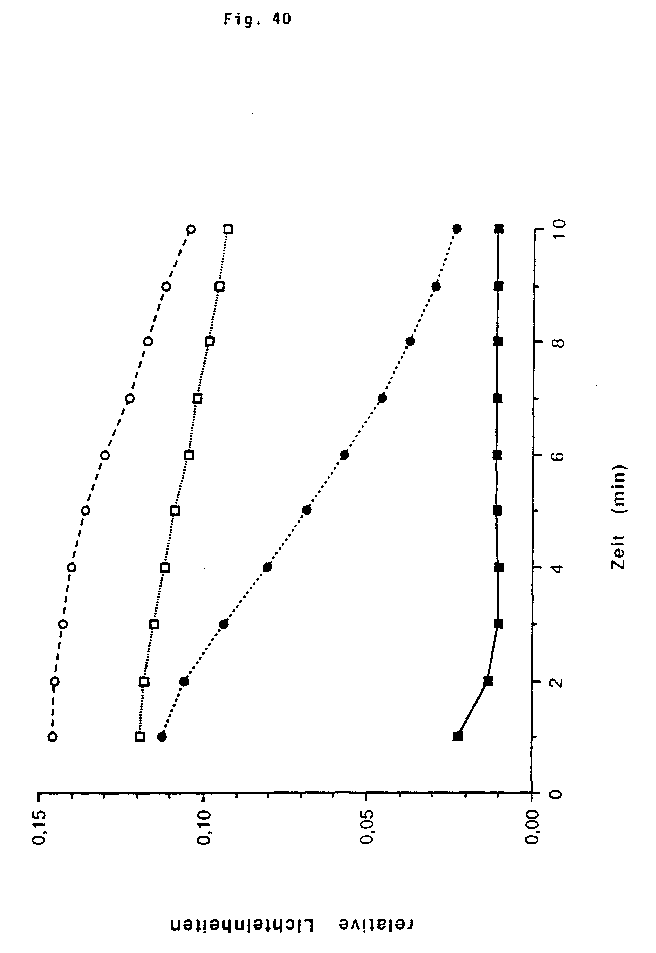

Fig. 40 zeigt den Einfluß des Zusatzes von β-Mercaptoethanol und/oder Natriumtripolyphosphat zum Grundpuffer auf das Luciferase-Meßsignal (gefüllte Quadrate: Grundpuffer; offene Quadrate: Zusatz von 4 µl/ml β-Mercaptoethanol; geschlossene Kreise: Zusatz von 0.2 mg/ml Natriumtripolyphosphat; offene Kreise: Zusatz von 4 µl/ml β-Mercaptoethanol plus 0.2 mg/ml Natriumtripolyphosphat). Für sämtliche Versuche wurde ein Luminometer der Marke Microlite ML 1000, Fa. Dynatech, verwendet.

**Tabelle 1**

| Substanz | nmol/l | MG | g/l |
|---|---|---|---|
| Tricin * | 25 | 179 | 4.48 |
| EDTA | 0.5 | 372 | 0.186 |
| MgSO₄.7H₂O | 16.3 | 246 | 4.0 |
| ATP | 1.2 | 605 | 0.726 |
| Luciferin, | 0.05 | 302 | 0.015 |
| Na-Salz | | | |
| DTT | 6.5 | 154 | 1.0 |
| NaTTP | 0.54 | 368 | 0.2 |

| | | | |
|---|---|---|---|
| * N-tris-(Hydroxymethyl)Methyl-Glycin | | | |

Triton X-100: 1 ml/l. der pH-Wert wird mit 1 N NaOH auf 7.8 eingestellt.

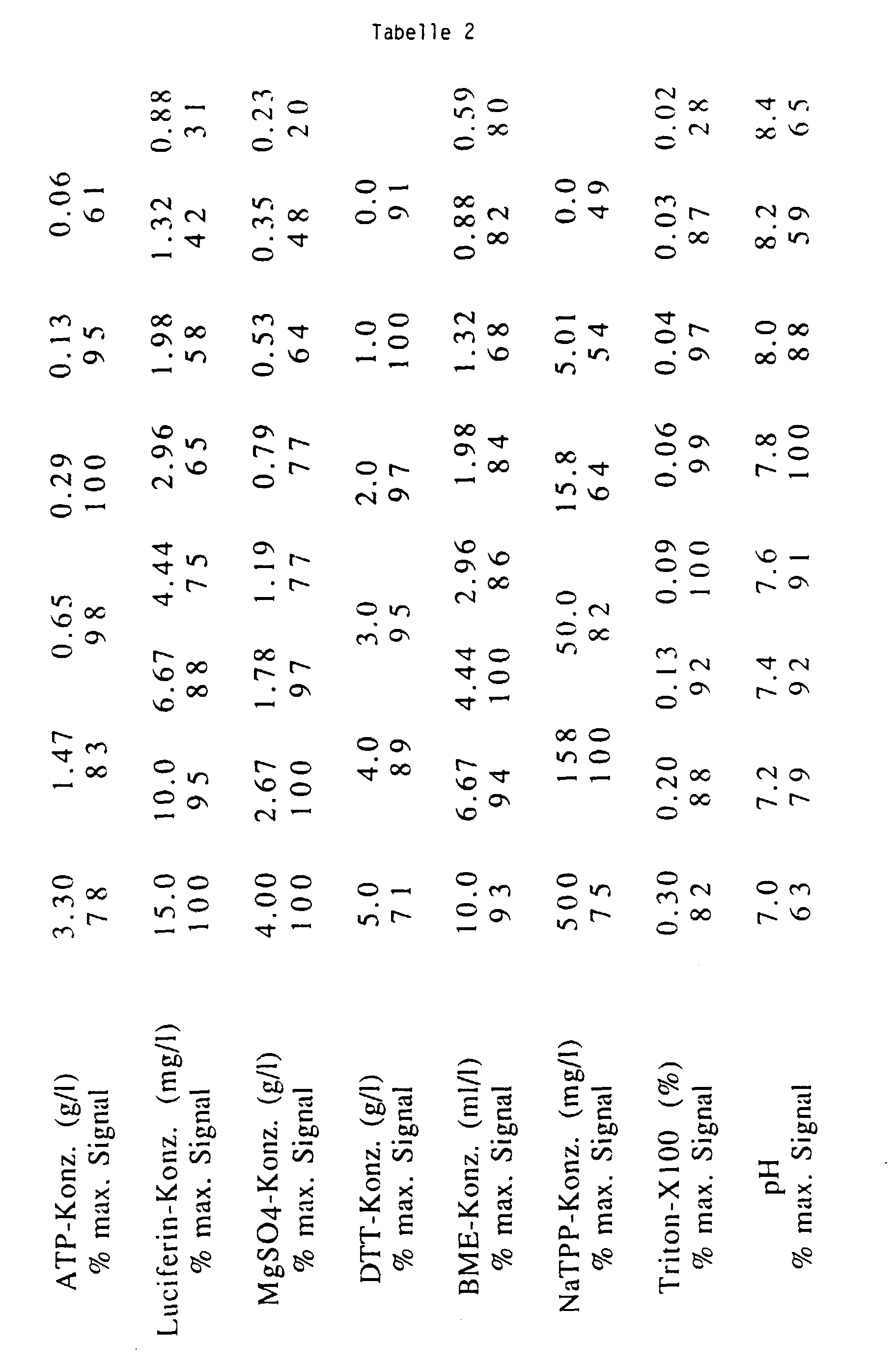

### Literatur:

Angel, P., Baumann, I., Stein, B., Delius, H., Rahmsdorf, H.J. und Herrlich, P., 1987a, Mol. Cell. Biol. 7, 2256-2266
Angel, P., Imagawa, M., Chiu, R., Stein, B., Imbra, R.J., Rahmsdorf, H.J., Jonat, L., Herrlich, P. und Karin, M., 1987b, Cell 49, 729-739
Billah, M.M., Pai, J.-K., Mullmann, T.J. Egan, R.W. und Siegel, 1989, J. Biol. Chem. 264, 9069-9076.
Brasier, A.R., Tate, J.E. und Habener, J.F. 1989, BioTechniques 7, 1116-1122
Buckley, N.J. et al., 1989, Molecular Pharmacology 35, 469-476
Chung, C.T. und Miller, R.H., 1988, Nucl. Acids Res. 16, 3580
Deutsch, P.J., Hoeffler, J.P., Jameson, J.L. und Habener, J.F., 1988, Proc.Natl.Acad.Sci. USA 85, 7922-7926
DeLuca, M., Wannlund, J. und McElroy, W.D., 1979, Anal. Biochem. 95, 194-198
De Wet, J.R., Wood, K.V., Helinski, D.R., und DeLuca, M., 1985, Proc.Natl.Acad.Sci. USA 82, 7870-7873
De Wet, J.R., Wood, K., DeLuca, M., Helinski, D. und Subramani, S., 1987, Mol. Cell. Biol. 7, 725-737
Dohlman, H.G. et al., 1991, Ann. Rev. Biochem. 60, 653-688
Doods, N.H. und von Meel, J.C.A., 1991, Receptor Data for Biological Experiments, Ellis Horwood Series in Pharmacological Sciences
Felgner, P. L., Gadek, T. R., Holm, M., Roman, R., Chan, H. N., Wenz, M., Northrop, J. P., Ringold, G. M., und Danielsen, M., 1987, Proc.Natl.Acad.Sci. USA 84, 7413-7417.
Grandy, D.K., Zhang, Y., Bouvier, C., Zhou, Q.Y. et al., 1991, Proc. Natl. Acad. Sci. USA 88, 9175-9179
Gritz, L. und Davies, J., 1983, Gene 25, 179-188
Hartmann, A., 1991, BioTec 5, 40-45
Houslay, M.D., 1991, Eur. J. Biochem. 195, 9-27
Julius, D., Huang, K.N., Livelli, T.J. Axel, R. und Jessell, T.M., 1990, Proc.Natl.Acad.Sci. USA 87, 928-932
Karin, M., 1989, TIG 5, 65-67
King, K., Dohlman, H.G., Thorner, J., Caron, M.G. und Lefkowitz, R.J., 1990, Science 250, 121-123
Kricka, L.J., 1988, Analyt. Biochem. 175, 14-21
Leach, F.R. und Webster, J.J., 1986, in: Bioluminescence and Chemiluminescence, Part B, Methods in Enzymology 133, 51-70
Lee, W., Mitchell, P. und Tjian, R., 1987, Cell 49, 741-752
Landschulz, W.H., Johnson, P.F. und McKnight, S.L., 1988, Science 240, 1759-1764
Maniatis, T., Fritsch, E.F. und Sambrook, J., 1982, Molecular Cloning: A Laboratory Manual, Cold Spring Harbor, New York, Cold Spring Harbour Laboratory
McKnight, S.L, 1980, Nucleic Acids Res. 8, 5949-5964
Montmayeur, J.-P. und Borrelli, E., 1991, Proc.Natl.Acad.Sci. USA 88, 3135-3139
Montminy, M.R., Gonzalez, G.A. und Yamamoto, K.K., 1990, Trends Neurosci. 13, 185
Mulligan, R. und Berg, P., 1981, Proc.Natl.Acad.Sci. USA 78, 2072-2076
Okayama, H. und Berg, P., 1983, Mol. Cell. Biol. 3, 280-289
Potter, H., Weir, L., und Leder, P., 1984, Proc.Natl.Acad.Sci. USA 81, 7161.
Pritchett et al., 1988, EMBO Journal 7, 4135-4140
Probst, W.C., Snyder L.A., Schuster D.I., Brosius J. und Sealfon S.C., 1992, DNA and Cell Biol.ll, 1-20
Saiki, R.K., Gelfand, D.H., Stoffel, S., Scharf, S.J., Higuchi, R., Horn, G.T., Mullis, K.B. und Erlich, H.A., 1988, Science 239, 487-491
Sassone-Corsi, P., Ransone, L.J. und Verma, I.M., 1990, Oncogene 5, 427-431
Short, et al., 1988, Nucl. Acids Res. 11, 5521-5540
Simon M.I., Strathmann, M.P. und Gautam, N., 1991, Science 252, 802-808
Southern, P. und Berg, P., 1982, J. Mol. Appl. Gen. 1, 327
Stinski, M.F. und Roehr, T.J., 1985, J. Virology 55, 431-441
Subramani, S. und DeLuca, M., 1987, Genetic Engineering, Principles and Methods, J.K. Sedlow - ed., Plenum Press, New York, Band 10, 75-89
Sugden, B., Marsh, K. und Yates, J., 1985, Mol. Cell. Biol. 5, 410-413
Tanahashi, H., Ito, T., Inouye, S., Tsuji, F.I., Sakaki, Y., 1990, Gene 96, 249-255
Turner R. und Tjian, R., 1989, Science 243, 1689-1694
Ullrich, A. und Schlessinger, J., 1990, Cell 61, 203-212
Urlaub, G. und Chasin, L.A., 1980, Proc.Natl.Acad.Sci. USA 77, 4216-4220
Voraberger, G., et al., 1991, J. Immunol. 147, 2777
Wagner, M.J., Sharp, J.A. und Summers, W.C., 1981, Proc.Natl.Acad.Sci. USA 78, 1441-1445
Wieland, E. et al., 1985, Ärztl. Lab. 31, 203-214
Winnacker, E.L., 1985, Gene und Klone, Eine Einführung in die Gentechnologie, VCH Verlagsges. Weinheim, 264
Zhou, Q.-Y., Grandy, D.K., Thambi, L., Kushner, J.A., Van Tol, H.H.M., Cone, R., Pribnow, D., Salon, J., Bunzow, J.R., und Civelli, O., 1990, Nature 347, 76-80
Cloning Vectors: Chapter VIII, Hsg. Pouwels, Enger-
Human Pharmacology - Molecular-To-Clinical, Chapter 2: Sites of Action: Receptors, Hsg. Wingard., L.B., Brody, T.M., Larner, J. und Schwartz, A., Mosby Year-Book Inc., St.Louis, 1991
TiPS: Receptor Nomenclature Supplement, 1991
Hepler, J.R. et al., G proteins, TIBS 17, Oct. 1992
Sternweis P.C. et al., Regulation of phospholipase C by G proteins, TIBS 17, Dec. 1992
Spiegel A.M., G proteins in cellular control, Current Opinion in Cell Biology 4, 1992
Berridge M.J., Inositol trisphosphate and calcium signalling, Nature Vol. 361, 28. Jan. 1993

### SEQUENZPROTOKOLL

### (1) TITEL DER ERFINDUNG: Verfahren zum Screenen von Substanzen mit modulierender Wirkung auf einen rezeptorabhängigen zellulären Signalübertragungsweg

### ANZAHL DER SEQUENZEN: 37

### (2) INFORMATION FÜR SEQ ID NO:1:

(i) SEQUENZCHARAKTERISIERUNG:
   (A) LÄNGE: 1630 Basenpaare
   (B) TYP: Nukleinsäure
   (C) STRANGFORM: Einzelstrang
   (D) TOPOLOGIE: linear
(ii) ART DES MOLEKÜLS: cDNA
   (xi) SEQUENZBESCHREIBUNG: SEQ ID NO:1:

CGTATCGATA GCATGTCTGA TCAGACAGCA GCTGCGCAGG GCTCGGAATG CTACCCTAAA 60

AGGCCACTCG AAACCCCAGC CCCGGGAGAA CAGCATGTAC ACCAGCCTCA GTGTTACAGA 120

GTGTGGGTAC ATCAAGGTGA ATGGTGAGCA GAAACTATAA CCTGTTAGTC CTTCTACACC 180

TCATCTGCTA CAAGTTCTGG CTTAGAC ATG GAT ATT CTT TGT GAA GAA AAT 231
Met Asp Ile Leu Cys Glu Glu Asn
1               5

ACT TCT TTG AGC TCA ACT ACG AAC TCC CTA ATG CAA TTA AAT GAT GAC 279
Thr Ser Leu Ser Ser Thr Thr Asn Ser Leu Met Gln Leu Asn Asp Asp
    10                  15                  20

ACC AGG CTC TAC AGT AAT GAC TTT AAC TCC GGA GAA GCT AAC ACT TCT 327
Thr Arg Leu Tyr Ser Asn Asp Phe Asn Ser Gly Glu Ala Asn Thr Ser
25                  30                  35                  40

GAT GCA TTT AAC TGG ACA GTC GAC TCT GAA AAT CGA ACC AAC CTT TCC 375
Asp Ala Phe Asn Trp Thr Val Asp Ser Glu Asn Arg Thr Asn Leu Ser
                45                  50                  55

TGT GAA GGG TGC CTC TCA CCG TCG TGT CTC TCC TTA CTT CAT CTC CAG 423
Cys Glu Gly Cys Leu Ser Pro Ser Cys Leu Ser Leu Leu His Leu Gln
            60                  65                  70

GAA AAA AAC TGG TCT GCT TTA CTG ACA GCC GTA GTG ATT ATT CTA ACT 471
Glu Lys Asn Trp Ser Ala Leu Leu Thr Ala Val Val Ile Ile Leu Thr
        75                  80                  85

ATT GCT GGA AAC ATA CTC GTC ATC ATG GCA GTG TCC [?]TA GAG AAA AAG 519
Ile Ala Gly Asn Ile Leu Val Ile Met Ala Val Ser [?]eu Glu Lys Lys
90 95 100

CTG CAG AAT GCC ACC AAC TAT TTC CTG ATG TCA CTT GCC ATA GCT GAT 567
Leu Gln Asn Ala Thr Asn Tyr Phe Leu Met Ser Leu Ala Ile Ala Asp
105 110 115 120

ATG CTG CTG GGT TTC CTT GTC ATG CCC GTG TCC ATG TTA ACC ATC CTG 615
Met Leu Leu Gly Phe Leu Val Met Pro Val Ser Met Leu Thr Ile Leu
125 130 135

TAT GGG TAC CGG TGG CCT CTG CCG AGC AAG CTT TGT GCA GTC TGG ATT 663
Tyr Gly Tyr Arg Trp Pro Leu Pro Ser Lys Leu Cys Ala Val Trp Ile
140 145 150

TAC CTG GAC GTG CTC TTC TCC ACG GCC TCC ATC ATG CAC CTC TGC GCC 711
Tyr Leu Asp Val Leu Phe Ser Thr Ala Ser Ile Met His Leu Cys Ala
155 160 165

ATC TCG CTG GAC CGC TAC GTC GCC ATC CAG AAT CCC ATC CAC CAC AGC 759
Ile Ser Leu Asp Arg Tyr Val Ala Ile Gln Asn Pro Ile His His Ser
170 175 180

CGC TTC AAC TCC AGA ACT AAG GCA TTT CTG AAA ATC ATT GCT GTT TGG 807
Arg Phe Asn Ser Arg Thr Lys Ala Phe Leu Lys Ile Ile Ala Val Trp
185 190 195 200

ACC ATA TCA GTA GGT ATA TCC ATG CCA ATA CCA GTC TTT GGG CTA CAG 855

Thr Ile Ser Val Gly Ile Ser Met Pro Ile Pro Val Phe Gly Leu Gln
205 210 215

GAC GAT TCG AAG GTC TTT AAG GAG GGG AGT TGC TTA CTC GCC GAT GAT 903
Asp Asp Ser Lys Val Phe Lys Glu Gly Ser Cys Leu Leu Ala Asp Asp
220 225 230

AAC TTT GTC CTG ATC GGC TCT TTT GTG TCA TTT TTC ATT CCC TTA ACC 951
Asn Phe Val Leu Ile Gly Ser Phe Val Ser Phe Phe Ile Pro Leu Thr
235 240 245

ATC ATG GTG ATC ACC TAC TTT CTA ACT ATC AAG TCA CTC CAG AAA GAA 999
Ile Met Val Ile Thr Tyr Phe Leu Thr Ile Lys Ser Leu Gln Lys Glu
250 255 260

GCT ACT TTG TGT GTA AGC GAT CTT GGC ACA CGG GCC AAA TTA GCT TCT 1047
Ala Thr Leu Cys Val Ser Asp Leu Gly Thr Arg Ala Lys Leu Ala Ser
265 270 275 280

TTC AGC TTC CTC CCT CAG AGT TCT TTG TCT TCA GAA AAG CTC TTC CAG 1095
Phe Ser Phe Leu Pro Gln Ser Ser Leu Ser Ser Glu Lys Leu Phe Gln
285 290 295

CGG TCG ATC CAT AGG GAG CCT GGG TCC TAC ACA GGC AGG AGG ACT ATG 1143
Arg Ser Ile His Arg Glu Pro Gly Ser Tyr Thr Gly Arg Arg Thr Met
300 305 310

CAG TCC ATC AGC AAT GAG CAA AAG GCA TGC AAG GTG CTG GGC ATC GTC 1191
Gln Ser Ile Ser Asn Glu Gln Lys Ala Cys Lys Val Leu Gly Ile Val
315 320 325

TTC TTC CTG TTT GTG GTG ATG TGG TGC CCT TTC TTC ATC ACA AAC ATC 1239
Phe Phe Leu Phe Val Val Met Trp Cys Pro Phe Phe Ile Thr Asn Ile
330 335 340

ATG GCC GTC ATC TGC AAA GAG TCC TGC AAT GAG GAT GTC ATT GGG GCC 1287

345 350 355 360

CTG CTC AAT GTG TTT GTT TGG ATC GGT TAT CTC TCT TCA GCA GTC AAC 1335


365 370 375

CCA CTA GTC TAC ACA CTG TTC AAC AAG ACC TAT AGG TCA GCC TTT TCA 1383

380 385 390

CGG TAT ATT CAG TGT CAG TAC AAG GAA AAC AAA AAA CCA TTG CAG TTA 1431

395 400 405

ATT TTA GTG AAC ACA ATA CCG GCT TTG GCC TAC AAG TCT AGC CAA CTT 1479

410 415 420

CAA ATG GGA CAA AAA AAG AAT TCA AAG CAA GAT GCC AAG ACA ACA GAT 1527

425 430 435 440

AAT GAC TGC TCA ATG GTT GCT CTA GGA AAG CAG CAT TCT GAA GAG GCT 1575

445 450 455

TCT AAA GAC AAT AGC GAC GGA GTG AAT GAA AAG GTG AGC TGT GTG TGATAGGCTA 1630

460 465 470

### (2) INFORMATION FÜR SEQ ID NO:2:

(i) SEQUENZCHARAKTERISIERUNG:
   (A) LÄNGE: 471 Aminosäuren
   (B) TYP: Aminosäure
   (D) TOPOLOGIE: linear
(ii) ART DES MOLEKÜLS: Protein
   (xi) SEQUENZBESCHREIBUNG: SEQ ID NO:2:

Met Asp Ile Leu Cys Glu Glu Asn Thr Ser Leu Ser Ser Thr Thr Asn
1               5                   10                  15

Ser Leu Met Gln Leu Asn Asp Asp Thr Arg Leu Tyr Ser Asn Asp Phe
            20                  25                  30

Asn Ser Gly Glu Ala Asn Thr Ser Asp Ala Phe Asn Trp Thr Val Asp
        35                  40                  45

Ser Glu Asn Arg Thr Asn Leu Ser Cys Glu Gly Cys Leu Ser Pro Ser
    50                  55                  60

Cys Leu Ser Leu Leu His Leu Gln Glu Lys Asn Trp Ser Ala Leu Leu
65                  70                  75                  80

Thr Ala Val Val Ile Ile Leu Thr Ile Ala Gly Asn Ile Leu Val Ile
                85                  90                  95

Met Ala Val Ser Leu Glu Lys Lys Leu Gln Asn Ala Thr Asn Tyr Phe
            100                 105                 110

Leu Met Ser Leu Ala Ile Ala Asp Met Leu Leu Gly Phe Leu Val Met
        115                 120                 125

Pro Val Ser Met Leu Thr Ile Leu Tyr Gly Tyr Arg Trp Pro Leu Pro
    130                 135                 140

Ser Lys Leu Cys Ala Val Trp Ile Tyr Leu Asp Val Leu Phe Ser Thr
145                 150                 155                 160

Ala Ser Ile Met His Leu Cys Ala Ile Ser Leu Asp Arg Tyr Val Ala
                165                 170                 175

Ile Gln Asn Pro Ile His His Ser Arg Phe Asn Ser Arg Thr Lys Ala
            180                 185                 190

Phe Leu Lys Ile Ile Ala Val Trp Thr Ile Ser Val Gly Ile Ser Met
        195                 200                 205

Pro Ile Pro Val Phe Gly Leu Gln Asp Asp Ser Lys Val Phe Lys Glu
    210                 215                 220

Gly Ser Cys Leu Leu Ala Asp Asp Asn Phe Val Leu Ile Gly Ser Phe
225                 230                 235                 240

Val Ser Phe Phe Ile Pro Leu Thr Ile Met Val Ile Thr Tyr Phe Leu
                245                 250                 255

Thr Ile Lys Ser Leu Gln Lys Glu Ala Thr Leu Cys Val Ser Asp Leu
            260                 265                 270

Gly Thr Arg Ala Lys Leu Ala Ser Phe Ser Phe Leu Pro Gln Ser Ser
        275                 280                 285

Leu Ser Ser Glu Lys Leu Phe Gln Arg Ser Ile His Arg Glu Pro Gly
    290                 295                 300

Ser Tyr Thr Gly Arg Arg Thr Met Gln Ser Ile Ser Asn Glu Gln Lys
305                 310                 315                 320

Ala Cys Lys Val Leu Gly Ile Val Phe Phe Leu Phe Val Val Met Trp
                325                 330                 335

Cys Pro Phe Phe Ile Thr Asn Ile Met Ala Val Ile Cys Lys Glu Ser
            340                 345                 350

Cys Asn Glu Asp Val Ile Gly Ala Leu Leu Asn Val Phe Val Trp Ile
        355                 360                 365

Gly Tyr Leu Ser Ser Ala Val Asn Pro Leu Val Tyr Thr Leu Phe Asn
    370                 375                 380

Lys Thr Tyr Arg Ser Ala Phe Ser Arg Tyr Ile Gln Cys Gln Tyr Lys
385                 390                 395                 400

Glu Asn Lys Lys Pro Leu Gln Leu Ile Leu Val Asn Thr Ile Pro Ala
                405                 410                 415

Leu Ala Tyr Lys Ser Ser Gln Leu Gln Met Gly Gln Lys Lys Asn Ser
            420                 425                 430

Lys Gln Asp Ala Lys Thr Thr Asp Asn Asp Cys Ser Met Val Ala Leu
        435                 440                 445

Gly Lys Gln His Ser Glu Glu Ala Ser Lys Asp Asn Ser Asp Gly Val

    450                 455                 460

Asn Glu Lys Val Ser Cys Val
465                 470

### (2) INFORMATION FÜR SEQ ID NO:3:

(i) SEQUENZCHARAKTERISIERUNG:
   (A) LÄNGE: 23 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:3:

GGAATTCGCG CCCTGTAGCG GCG 23

### (2) INFORMATION FÜR SEQ ID NO:4:

(i) SEQUENZCHARAKTERISIERUNG:
   (A) LÄNGE: 37 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:4:

CACTGAACTC GAGCAGCTGC GTTGCTGGCG TTTTTCC 37

### (2) INFORMATION FÜR SEQ ID NO:5:

(i) SEQUENZCHARAKTERISIERUNG:
   (A) LÄNGE: 64 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:5:

GACTTCAGAT CTGCGGCCGC CTCGAGGGTA CCGTTAACGT CGACAAACCC CGCCCAGCGT 60

CTTG 64

### (2) INFORMATION FÜR SEQ ID NO:6:

(i) SEQUENZCHARAKTERISIERUNG:
   (A) LÄNGE: 56 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:6:

GACTTCGGAT CCGAGCTCAC TAGTTCTAGA AAGCTTGACG CTGTTAAGCG GGTCGC 56

### (2) INFORMATION FÜR SEQ ID NO:7:

(i) SEQUENZCHARAKTERISIERUNG:
   (A) LÄNGE: 56 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:7:

GACTTCGGAT CCGAGCTCAC TAGTTCTAGA AAGCTTGA[?]G CTGTTAAGCG GGTCGC 56

### (2) INFORMATION FÜR SEQ ID NO:8:

(i) SEQUENZCHARAKTERISIERUNG:
   (A) LÄNGE: 45 Basenpaare
   (B) TYP: Nukleinsaure
   (C) STRANGFORM: Einzelstrang
   (D) TOPOLOGIE: linear
(ii) ART DES MOLEKÜLS: synchetisches Oligodesoxyribonukleotid
   (xi) SEQUENZBESCHREIBUNG: SEQ ID NO:8:

AGCTTGTAAG CAGCAGCTGC AGTGCTCTGC CTTTTATGCC CAAGG 45

### (2) INFORMATION FÜR SEQ ID NO:9:

(i) SEQUENZBESCHREIBUNG:
   (A) LÄNGE: 21 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:9:

TCGATGCGGC CGCGACTTCA G 21

### (2) INFORMATION FÜR SEQ ID NO:10:

(i) SEQUENZBESCHREIBUNG:
   (A) LÄNGE: 17 Basenpaare
   (B) TYP: Nukleinsäure
   (C) STRANGFORM: Einzelstrang
   (D) TOPOLOGIE: linear
(ii) ART DES MOLEKÜLS: synthetiscnes Oligodesoxyribonukleotid
   (xi) SEQUENZBESCHREIBUNG: SEQ ID NO:10:

CTGAAGTCGC GGCCGCA 17

### (2) INFORMATION FÜR SEQ ID NO:11:

(i) SEQUENZCHARAKTERISIERUNG:
   (A) LÄNGE: 25 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:11:

GGCAGCTGAC GTCACTGTCT GGTGC 25

### (2) INFORMATION FÜR SEQ ID NO:12:

(i) SEQUENZCHARAKTERISIERUNG:
   (A) LÄNGE: 35 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:12:

CTCCTTGGCT GACGTCAGTA GAGAGATCCC ATGGC 35

### (2) INFORMATION FÜR SEQ ID NO:13:

(i) SEQUENZCHARAKTERISIERUNG:
   (A) LÄNGE: 27 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:13:

CTCTACTGAC GTCAGCCAAG GAGGTAC 27

### (2) INFORMATION FÜR SEQ ID NO:14:

(i) SEQUENZCHARAKTERISIERUNG:
   (A) LÄNGE: 47 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:14:

CGTCATACTG TGACGTCTTT CAGACACCCC ATTGACGTCA ATGGGAG 47

### (2) INFORMATION FÜR SEQ ID NO:15:

(i) SEQUENZCHARAKTERISIERUNG:
   (A) LÄNGE: 41 Basenpaare
   (3) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:15:

GGCCGCACCA GACAGTGACG TCAGCTGCCA GATCCCATGG C 41

### (2) INFORMATION FÜR SEQ ID NO:16:

(i) SEQUENZCHARAKTERISIERUNG:
   (A) LÄNGE: 35 Basenpaare
   (B) TYP: Nukleinsäure
   (C) STRANGFORM: Einzelstrang
   (D) TOPOLOGIE: linear
(ii) ART DES MOLEKÜLS: synthetishes Oligodesoxyribonukleotid
   (xi) SEQUENZBESCHREIBUNG: SEQ ID NO:16:

CTCCTTGGCT GACGTCAGTA GAGAGATCCC ATGGC 35

### (2) INFORMATION FÜR SEQ ID NO:17:

(i) SEQUENZCHARAKTERISIERUNG:
   (A) LÄNGE: 63 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:17:

TCGACTCCCA TTGACGTCAA TGGGGTGTCT GAAAGACGTC ACAGTATGAC GGCCATGGGA 60

TCT 63

### (2) INFORMATION FÜR SEQ ID NO:18:

(i) SEQUENZCHARAKTERISIERUNG:
   (A) LÄNGE: 57 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:18:

GGCCGCAGGT ACCAGATCTA CTCGAGTGTA GACCGTGATT CAAGCTTAGC TGTAGAC 57

### (2) INFORMATION FÜR SEQ ID NO:19:

(i) SEQUENZCHARAKTERISIERUNG:
   (A) LÄNGE: 41 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:19:

GCTTGAATCA CGGTCTACAC TCGAGTAGAT CTGGTACCTG C 41

### (2) INFORMATION FÜR SEQ ID NO:20:

(i) SEQUENZCHARAKTERISIERUNG:
   (A) LÄNGE: 56 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:20:

TCGACTAAGC TTGAATCACG GTCTACAGCT AAGCTTGAAT CACGGTCTAC AGCTAA 56

### (2) INFORMATION FÜR SEQ ID NO:21:

(i) SEQUENZCHARAKTERISIERUNG:
   (A) LÄNGE: 40 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:21:

CGTGATTCAA GCTTAGCTGT AGACCGTGAT TCAAGCTTAG 40

### (2) INFORMATION FÜR SEQ ID NO:22:

(i) SEQUENZCHARAKTERISIERUNG:
   (A) LÄNGE: 56 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:22:

GGCCGCAGGT ACCAGATCTA CTCGAGTGTA GACCGTGATT CAAGCTTAGT GTAGAC 56

### (2) INFORMATION FÜR SEQ ID NO:23:

(i) SEQUENZCHARAKTERISIERUNG:
   (A) LÄNGE: 50 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:23:

TCGACCTTGA ATCACGGTCT ACACTAAGCT TGAATCACGG TCTACACTAA 50

### (2) INFORMATION FÜR SEQ ID NO:24:

(i) SEQUENZCHARAKTERISIERUNG:
   (A) LÄNGE: 35 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:24:

CGTGATTCAA GCTTAGTGTA GACCGTGATT CAAGG 35

### (2) INFORMATION FÜR SEQ ID NO:25:

(i) SEQUENZCHARAKTERISIERUNG:
   (A) LÄNGE: 53 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:25:

GGCCGCAGGT ACCAGATCTA CTCGAGTGTA GACCGTGATT CAAGCTTAGC CTG 53

### (2) INFORMATION FÜR SEQ ID NO:26:

(i) SEQUENZCHARAKTERISIERUNG:
   (A) LÄNGE: 64 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:26:

TCGACTAAGC TTGAATCACG GTCTACACCA GGCTAAGCTT GAATCACGGT CTACACCAGG 60

CTAA 64

### (2) INFORMATION FÜR SEQ ID NO:27:

(i) SEQUENZCHARAKTERISIERUNG:
   (A) LÄNGE: 52 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:27:

GTGTAGACCG TGATTCAAGC TTAGCCTGGT GTAGACCGTG ATTCAAGCTT AG 52

### (2) INFORMATION FÜR SEQ ID NO:28:

(i) SEQUENZCHARAKTERISIERUNG:
   (A) LÄNGE: 64 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:28:

GGCCGCAGGT ACCAGATCTA CTCGAGTGTA GACCGTGATT CAAGCTTAGC CTGGCGGTGT 60

AGAC 64

### (2) INFORMATION FÜR SEQ ID NO:29:

(i) SEQUENZCHARAKTERISIERUNG:
   (A) LÄNGE: 50 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:29:

CCAGGCTAAG CTTGAATCAC GGTCTACACT CGAGTAGATC TGGTACCTGC 50

### (2) INFORMATION FÜR SEQ ID NO:30:

(i) SEQUENZCHARAKTERISIERUNG:
   (A) LÄNGE: 51 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:30:

CGTGATTCAA GCTTAGCCTG GCGGTGTAGA CCGTGATTCA AGCTTAGCCT G 51

### (2) INFORMATION FÜR SEQ ID NO:31:

(i) SEQUENZCHARAKTERISIERUNG:
   (A) LÄNGE: 65 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:31:

TCGACAGGCT AAGCTTGAAT CACGGTCTAC ACCGCCAGGC TAAGCTTGAA TCACGGTCTA 60

CACCG 65

### (2) INFORMATION FÜR SEQ ID NO:32:

(i) SEQUENZCHARAKTERISIERUNG:
   (A) LÄNGE: 74 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:32:

GGCCGCAGGT ACCAGATCTA CTCGAGTGTA GACCGTGATT CAAGCTTAGC CTGGCCGGTT 60

AGCGCGGTGT AGAC 74

### (2) INFORMATION FÜR SEQ ID NO:33:

(i) SEQUENZCHARAKTERISIERUNG:
   (A) LÄNGE: 62 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:33:

CGCGCTAACC GGCCAGGCTA AGCTTGAATC ACGGTCTACA CTCGAGTAGA TCTGGTACCT 60

GC 62

### (2) INFORMATION FÜR SEQ ID NO:34:

(i) SEQUENZCHARAKTERISIERUNG:
   (A) LÄNGE: 73 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:34:

TCGACAGGCT AAGCTTGAAT CACGGTCTAC ACCGCGCTAA CCGGCCAGGC TAAGCTTGAA 60

TCACGGTCTA CAC 73

### (2) INFORMATION FÜR SEQ ID NO:35:

(i) SEQUENZCHARAKTERISIERUNG:
   (A) LÄNGE: 61 Basenpaare
   (B) TYP: Nukleinsäure
   (C) STRANGFORM: Einzelstrang
   (D) TOPOLOGIE: linear
(ii) ART DES MOLEKÜLS: synthetisches Oligodesoxyribonukleotid
   (xi) SEQUENZBESCHREIBUNG: SEQ ID NO:35:

CGTGATTCAA GCTTAGCCTG GCCGGTTAGC GCGGTGTAGA CCGTGATTCA AGCTTAGCCT 60

G 61

### (2) INFORMATION FÜR SEQ ID NO:36:

(i) SEQUENZCHARAKTERISIERUNG:
   (A) LÄNGE: 6623 Basenpaare
   (B) TYP: Nukleinsäure
   (C) STRANGFORM: Einzelstrang
   (D) TOPOLOGIE: zirkulär
(ii) ART DES MOLEKÜLS: Plasmid-DNA
   (xi) SEQUENZBESCHREIBUNG: SEQ ID NO:36:

AATCAATATT GGCAATTAGC CATATTAGTC ATTGGTTATA TAGCATGAAT CAATATTGGC 60

TATTGGCCAT TGCATACGTT GTATCTATAT CATAATATGT ACATTTATAT TGGCTCATGT 120

CCAATATGAC CGCCATGTTG ACATTGATTA TTGACTAGTT ATTAATAGTA ATCAATTACG 180

GGGTCATTAG TTCATAGCCC ATATATGGAG TTCCGCGTTA CATAACTTAC GGTAAATGGC 240

CCGCCTGGCT GACCGCCCAA CGACCCCCGC CCATTGACGT CAATAATGAC GTATGTTCCC 300

ATAGTAACGC CAATAGGGAC TTTCCATTGA CGTCAATGGG TGGAGTATTT ACGGTAAACT 360

GCCCACTTGG CAGTACATCA AGTGTATCAT ATGCCAAGTA CGCCCCCTAT TGACGTCAAT 420

GACGGTAAAT GGCCCGCCTG GCATTAT[?]CC CAGTACATGA CCTTATGGGA CTTTCCTACT 480

TGGCAGTACA TCTACGTATT AGTCATCGCT ATTACCATGG TGATGCGGTT TTGGCAGTAC 540

ATCAATGGGC GTGGATAGCG GTTTGACTCA CGGGGATTTC CAAGTCTCCA CCCCATTGAC 600

GTCAATGGGA GTTTGTTTTG GCACCAAAAT CAACGGGACT TTCCAAAATG TCGTAACAAC 660

TCCGCCCCAT TGACGCAAAT GGGCGGTAGG CGTGTACGGT GGGAGGTCTA TATAAGCAGA 720

GCTCTCTGGC TAACTAGAGA ACCCACTGCT TACTGGCTTA TCGAAATTAA TACGACTCAC 780

TATAGGGAGA CCCAAGCTTC TGCAGGTCGA CATCGATGGA TCCGGTACCT CGAGCGGCCG 840

CGAATTCTCT AGAGGATCTT TGTGAAGGAA CCTTACTTCT GTGGTGTGAC ATAATTGGAC 900

AAACTACCTA CAGAGATTTA AAGCTCTAAG GTAAATATAA AATTTTTAAG TGTATAATGT 960

GTTAAACTAC TGATTCTAAT TGTTTGTGTA TTTTAGATTC CAACCTATGG AACTGATGAA 1020

TGGGAGCAGT GGTGGAATGC CTTTAATGAG GAAAACCTGT TTTGCTCAGA AGAAATGCCA 1080

TCTAGTGATG ATGAGGCTAC TGCTGACTCT CAACATTCTA CTCCTCCAAA AAAGAAGAGA 1140

AAGGTAGAAG ACCCCAAGGA CTTTCCTTCA GAATTGCTAA GTTTTTTGAG TCATGCTGTG 1200

TTTAGTAATA GAACTCTTGC TTGCTTTGCT ATTTACACCA CAAAGGAAAA AGCTGCACTG 1260

CTATACAAGA AAATTATGGA AAAATATTTG ATGTATAGTG CCTTGACTAG AGATCATAAT 1320

CAGCCATACC ACATTTGTAG AGGTTTTACT TGCTTTAAAA AACCTCCCAC ACCTCCCCCT 1380

GAACCTGAAA CATAAAATGA ATGCAATTGT TGTTGTTAAC TTGTTTATTG CAGCTTATAA 1440

TGGTTACAAA TAAAGCAATA GCATCACAAA TTTCACAAAT AAAGCATTTT TTTCACTGCA 1500

TTCTAGTTGT GGTTTGTCCA AACTCATCAA TGTATCTTAT CATGTCTGGA TCAATTCTGA 1560

AAAACTAGCC TTAAAGACAG ACAGCTTTGT TCTAGTCAGC CAGGCAAGCA TATGTAAATA 1620

AAGTTCCTCA GGGAACTGAG GTTAAAAGAT GTATCCTGGA CCTGCCAGAC CTGGCCATTC 1680

ACGTAAACAG AAGATTCCGC CTCAAGTTCC GGTTAACAAC AGGAGGCAAC GAGATCATCG 1740

CTGTTCCTTA GGACCCTTTT ACTAACCCTA ATTCGATAGC ATATGCTTCC CGTTGGGTAA 1800

CATATGCTAT TGAATTAGGG TTAGTCTGGA TAGTATATAC TACTACCCGG GAAGCATATG 1860

CTACCCGTTT AGGGTTAACA AGGGGGCCTT ATAAACACTA TTGCTAATGC CCTCTTGAGG 1920

GTCCGCTTAT CGGTAGCTAC ACAGGCCCCT CTGATTGACG TTGGTGTAGC CTCCCGTAGT 1980

CTTCCTGGGC CCCTGGGAGG TACATGTCCC CCAGCATTGG TGTAAGAGCT TCAGCCAAGA 2040

GTTACACATA AAGGCAATGT TGTGTTGCAG TCCACAGACT GCAAAGTCTG [?]TCCAGGATG 2100

AAAGCCACTC AGTGTTGGCA AATGTGCACA TCCATTTATA AGGATGTCAA CTACAGTCAG 2160

AGAACCCCTT TGTGTTTGGT CCCCCCCCG[?] GTCACATGTG GAACAGGGCC CAGTTGGCAA 2220

GTTGTACCAA CCAACTGAAG GGATTACATG CACTGCCCCG CGTGAGCAAT ACAAAACAAA 2280

AGCGCTCCTC GTACCAGCGA AGAAGGGGCA GAGATGCCGT AGTCAGGTTT AGTTCGTCCG 2340

GCGGCGCCAG AAATCCGCGC GGTGGTTTTT GGGGGTCGGG GGTGTTTGGC AGCCACAGAC 2400

GCCCGGTGTT CGTGTCGCGC CAGTACATGC GGTCCATGCC CAGGCCATCC AAAAACCATG 2460

GGTCTGTCTG CTCAGTCCAG TCGTGGACCT GACCCCACGC AACGCCCAAA AGAATAACCC 2520

CCACGAACCA TAAACCATTC CCCATGGGGG ACCCCGTCCC TAACCCACGG GGCCCGTGGC 2580

TATGGCGGGC TTGCCGCCCC GACGTTGGCT GCGAGCCCTG GGCCTTCACC CGAACTTGGG 2640

GGTTGGGGTG GGGAAAAGGA AGAAACGCGG GCGTATTGGC CCCAATGGGG TCTCGGTGGG 2700

GTATCGACAG AGTGCCAGCC CTGGGACCGA ACCCCGCGTT TATGAACAAA CGACCCAACA 2760

CCCGTGCGTT TTATTCTGTC TTTTTATTGC CGTCATAGCG CGGGTTCCTT CCGGTATTGT 2820

CTCCTTCCGT GTTTCAGTTA GCCTCCCCCA TCTCCCGATC CCTATTCCTT TGCCCTCGGA 2880

CGAGTGCTGG GGCGTCGGTT TCCACTATCG GCGAGTACTT CTACACAGCC ATCGGTCCAG 2940

ACGGCCGCGC TTCTGCGGGC GATTTGTGTA CGCCCGACAG TCCCGGCTCC GGATCGGACG 3000

ATTGCGTCGC ATCGACCCTG CGCCCAAGCT GCATCATCGA AATTGCCGTC AACCAAGCTC 3060

TGATAGACTT GGTCAAGACC AATGCGG[?][?]C ATATACGCCC GGAGCCGCGG CGATCCTGCA 3120

AGCTCCGGAT GCCTCCGCTC GAAGTAG[?][?][?] GTCTGCTGCT CCATACAAGC CAACCACGGC 3180

CTCCAGAAGA AGATGTTGGC GACCTCGTAT TGGGAATCCC CGAACATCGC CTCGCTCCAG 3240

TCAATGACCG CTGTTATGCG GCCATTGTCC GTCAGGACAT TGTTGGAGCC GAAATCCGCG 3300

TGCACGAGGT GCCGGACTTC GGGGCAGTCC TCGGCCCAAA GCATCAGCTC ATCGAGAGCC 3360

TGCGCGACGG ACGCACTGAC GGTGTCGTCC ATCACAGTTT GCCAGTGATA CACATGGGGA 3420

TCAGCAATCG CGCATATGAA ATCACGCCAT GTAGTGTATT GACCGATTCC TTGCGGTCCG 3480

AATGGGCCGA ACCCGCTCGT CTGGCTAAGA TCGGCCGCAG CGATCGCATC CATGGCCTCC 3540

GCGACCGGCT GCAGAACAGC GGGCAGTTCG GTTTCAGGCA GGTCTTGCAA CGTGACACCC 3600

TGTGCACGGC GGGAGATGCA ATAGGTCAGG CTCTCGCTGA ATTCCCCAAT GTCAAGCACT 3660

TCCGGAATCG GGAGCGCGGC CGATGCAAAG TGCCGATAAA CATAACGATC TTTGTAGAAA 3720

CCATCGGCGC AGCTATTTAC CCGCAGGACA TATCCACGCC CTCCTACATC GAAGCTGAAA 3780

GCACGAGATT CTTCGCCCTC CGAGAGCTGC ATCAGGTCGG AGACGCTGTC GAACTTTTCG 3840

ATCAGAAACT TCTCGACAGA CGTCGCGGTG AGTTCAGGCT TTTTCATATC TCATTGCCCC 3900

CGGACGAGGA TCTGCGGCAC GCTGTTGACG CTGTTAAGCG GGTCGCTGCA GGGTCGCTCG 3960

GTGTTCGAGG CCACACGCGT CACCTTAATA TGCGAAGTGG ACCTGGGACC GCGCCGCCCC 4020

GACTGCATCT GCGTGTTCGA ATTCGCCAAT GACAAGACGC TGGGCGGGGT TTGTGTCATC 4080

ATAGAACTAA AGACATGCAA ATATATTTCT TCCGGGGACA CCGCCAGCAA ACGCGAGCAA 4140

CGGGCCACGG GGATGAAGCA GGGCGGCACC TCGCTAACGG ATTCACCACT CCAAGAATTG 4200

GAGCCAATCA ATTCTTGCGG AGAACTGTGA ATGCGCAAAC CAACCCTTGG CAGAACATAT 4260

CCATCGCGTC CGCCATCTCC AGCAGCCGCA CGCGGCGCAT CTCGGGCAGC GTTGGGTCCT 4320

GGCCACGGGT GCGCATGATC GTGCTCCTGT CGTTGAGGAC CCGGCTAGGC TGGCGGGGTT 4380

GCCTTACTGG TTAGCAGAAT GAATCACCGA TACGCGAGCG AACGTGAAGC GACTGCTGCT 4440

GCAAAACGTC TGCGACCTGA GCAACAACAT GAATGGTCTT CGGTTTCCGT GTTTCGTAAA 4500

GTCTGGAAAC GCGGAAGTCA GCGCTCTTCC GCTTCCTCGC TCACTGACTC GCTGCGCTCG 4560

GTCGTTCGGC TGCGGCGAGC GGTATCAGCT CACTCAAAGG CGGTAATACG GTTATCCACA 4620

GAATCAGGGG ATAACGCAGG AAAGAACATG TGAGCAAAAG GCCAGCAAAA GGCCAGGAAC 4680

CGTAAAAAGG CCGCGTTGCT GGCGTTTTTC CATAGGCTCC GCCCCCCTGA CGAGCATCAC 4740

AAAAATCGAC GCTCAAGTCA GAGGTGGCGA AACCCGACAG GACTATAAAG ATACCAGGCG 4800

TTTCCCCCTG GAAGCTCCCT CGTGCGCTCT CCTGTTCCGA CCCTGCCGCT TACCGGATAC 4860

CTGTCCGCCT TTCTCCCTTC GGGAAGCGTG GCGCTTTCTC ATAGCTCACG CTGTAGGTAT 4920

CTCAGTTCGG TGTAGGTCGT TCGCTCCAAG CTGGGCTGTG TGCACGAACC CCCCGTTCAG 4980

CCCGACCGCT GCGCCTTATC CGGTAACTAT CGTCTTGAGT CCAACCCGGT AAGACACGAC 5040

TTATCGCCAC TGGCAGCAGC CACTGGTAAC AGGATTAGCA GAGCGAGGTA TGTAGGCGGT 5100

GCTACAGAGT TCTTGAAGTG GTGGCCTAAC TACGGCTACA CTAGAAGGAC AGTATTTGGT 5160

ATCTGCGCTC TGCTGAAGCC AGTTACCTTC GGAAAAAGAG TTGGTAGCTC TTGATCCGGC 5220

AAACAAACCA CCGCTGGTAG CGGTGGTTTT TTTGTTTGCA AGCAGCAGAT TACGCGCAGA 5280

AAAAAAGGAT CTCAAGAAGA TCCTTTGATC TTTTCTACGG GGTCTGACGC TCAGTGGAAC 5340

GAAAACTCAC GTTAAGGGAT TTTGGTCATG AGATTATCAA AAAGGATCTT CACCTAGATC 5400

CTTTTAAATT AAAAATGAAG TTTTAAATCA ATCTAAAGTA TATATGAGTA AACTTGGTCT 5460

GACAGTTACC AATGCTTAAT CAGTGAGGCA CCTATCTCAG CGATCTGTCT ATTTCGTTCA 5520

TCCATAGTTG CCTGACTCCC CGTCGTGTAG ATAACTACGA TACGGGAGGG CTTACCATCT 5580

GGCCCCAGTG CTGCAATGAT ACCGCGAGAC CCACGCTCAC CGGCTCCAGA TTTATCAGCA 5640

ATAAACCAGC CAGCCGGAAG GGCCGAGCGC AGAAGTGGTC CTGCAACTTT ATCCGCCTCC 5700

ATCCAGTCTA TTAATTGTTG CCGGGAAGCT AGAGTAAGTA GTTCGCCAGT TAATAGTTTG 5760

CGCAACGTTG TTGCCATTGC TGCAGGCATC GTGGTGTCAC GCTCGTCGTT TGGTATGGCT 5820

TCATTCAGCT CCGGTTCCCA ACGATCAAGG CGAGTTACAT GATCCCCCAT GTTGTGCAAA 5880

AAAGCGGTTA GCTCCTTCGG TCCTCCGATC GTTGTCAGAA GTAAGTTGGC CGCAGTGTTA 5940

TCACTCATGG TTATGGCAGC ACTGCATAAT TCTCTTACTG TCATGCCATC CGTAAGATGC 6000

TTTTCTGTGA CTGGTGAGTA CTCAACCAAG TCATTCTGAG AATAGTGTAT GCGGCGACCG 6060

AGTTGCTCTT GCCCGGCGTC AACACGGGAT AATACCGCGC CACATA[?][?]AG AACTTTAAAA 6120

GTGCTCATCA TTGGAAAACG TTCTTCGGGG CGAAAACTCT CAAGGA[?][?][?]T ACCGCTGTTG 6180

AGATCCAGTT CGATGTAACC CACTCGTGCA CCCAACTGAT CTTCAGCATC TTTTACTTTC 6240

ACCAGCGTTT CTGGGTGAGC AAAAACAGGA AGGCAAAATG CCGCAAAAAA GGGAATAAGG 6300

GCGACACGGA AATGTTGAAT ACTCATACTC TTCCTTTTTC AATATTATTG AAGCATTTAT 6360

CAGGGTTATT GTCTCATGAG CGGATACATA TTTGAATGTA TTTAGAAAAA TAAACAAATA 6420

GGGGTTCCGC GCACATTTCC CCGAAAAGTG CCACCTGACG TCTAAGAAAC CATTATTATC 6480

ATGACATTAA CCTATAAAAA TAGGCGTATC ACGAGGCCCT TTCGTCTTCA AGAATTCTCA 6540

TGTTTGACAG CTTATCGATC CGGCCAACGG TGTTGCCATT GCTGCAGGCG CAGAGCTGGT 6600

AGGTATGGAA GATCTATACA GTG 6623

### (2) INFORMATION FÜR SEQ ID NO:37:

(i) SEQUENZCHARAKTERISIERUNG:
   (A) LÄNGE: 6630 Basenpaare
   (B) TYP: Nukleinsäure
   (C) STRANGFORM: Einzelstrang
   (D) TOPOLOGIE: zirkulär
(ii) ART DES MOLEKÜLS: Plasmid-DNA
   (xi) SEQUENZBESCHREIBUNG: SEQ ID NO:37:

AATCAATATT GGCAATTAGC CATATTAGTC ATTGGTTATA TAGCATGAAT CAATATTGGC 60

TATTGGCCAT TGCATACGTT GTATCTATAT CATAATATGT ACATTTATAT TGGCTCATGT 120

CCAATATGAC CGCCATGTTG ACATTGATTA TTGACTAGTT ATTAATAGTA ATCAATTACG 180

GGGTCATTAG TTCATAGCCC ATATATGGAG TTCCGCGTTA CATAACTTAC GGTAAATGGC 240

CCGCCTGGCT GACCGCCCAA CGACCCCCGC CCATTGACGT CAATAATGAC GTATGTTCCC 300

ATAGTAACGC CAATAGGGAC TTTCCATTGA CGTCAATGGG TGGAGTATTT ACGGTAAACT 360

GCCCACTTGG CAGTACATCA AGTGTATCAT ATGCCAAGTA CGCCCCCTAT TGACGTCAAT 420

GACGGTAAAT GGCCCGCCTG GCATTATGCC CAGTACATGA CCTTATGGGA CTTTCCTACT 480

TGGCAGTACA TCTACGTATT AGTCATCGCT ATTACCATGG TGATGCGGTT TTGGCAGTAC 540

ATCAATGGGC GTGGATAGCG GTTTGACTCA CGGGGATTTC CAAGTCTCCA CCCCATTGAC 600

GTCAATGGGA GTTTGTTTTG GCACCAAAAT CAACGGGACT TTCCAAAATG TCGTAACAAC 660

TCCGCCCCAT TGACGCAAAT GGGCGGTAGG CGTGTACGGT GGGAGGTCTA TATAAGCAGA 720

GCTCTCTGGC TAACTAGAGA ACCCACTGCT TACTGGCTTA TCGAAATTAA TACGACTCAC 780

TATAGGGAGA CCCAAGCTCT AGAGAATTCG CGGCTCGAGG TACCGGATCC ATCGATGTCG 840

ACCTGCAGAA GCTTGCTAGA GGATCTTTGT GAAGGAACCT TACTTCTGTG GTGTGACATA 900

ATTGGACAAA CTACCTACAG AGATTTAAAG CTCTAAGGTA AATATAAAAT TTTTAAGTGT 960

ATAATGTGTT AAACTACTGA TTCTAATTGT TTGTGTATTT TAGATTCCAA CCTATGGAAC 1020

TGATGAATGG GAGCAGTGGT GGAATGCCTT TAATGAGGAA AACCTGTTTT GCTCAGAAGA 1080

AATGCCATCT AGTGATGATG AGGCTACTGC TGACTCTCAA CATTCTACTC CTCCAAAAAA 1140

GAAGAGAAAG GTAGAAGACC CCAAGGACTT TCCTTCAGAA TTGCTAAGTT TTTTGAGTCA 1200

TGCTGTGTTT AGTAATAGAA CTCTTGCTTG CTTTGCTATT TACACCACAA AGGAAAAAGC 1260

TGCACTGCTA TACAAGAAAA TTATGGAAAA ATATTTGATG TATAGTGCCT TGACTAGAGA 1320

TCATAATCAG CCATACCACA TTTGTAGAGG TTTTACTTGC TTTAAAAAAC CTCCCACACC 1380

TCCCCCTGAA CCTGAAACAT AAAATGAATG CAATTGTTGT TGTTAACTTG TTTATTGCAG 1440

CTTATAATGG TTACAAATAA AGCAATAGCA TCACAAATTT CACAAATAAA GCATTTTTTT 1500

CACTGCATTC TAGTTGTGGT TTGTCCAAAC TCATCAATGT ATCTTATCAT GTCTGGATCA 1560

ATTCTGAGAA ACTAGCCTTA AAGACAGACA GCTTTGTTCT AGTCAGCCAG GCAAGCATAT 1620

GTAAATAAAG TTCCTCAGGG AACTGAGGTT AAAAGATGTA TCCTGGACCT GCCAGACCTG 1680

GCCATTCACG TAAACAGAAG ATTCCGCCTC AAGTTCCGGT TAACAACAGG AGGCAACGAG 1740

ATCATCGCTG TTCCTTAGGA CCCTTTTACT AACCCTAATT CGATAGCATA TGCTTCCCGT 1800

TGGGTAACAT ATGCTATTGA ATTAGGGTTA GTCTGGATAG TATATACTAC TACCCGGGAA 1860

GCATATGCTA CCCGTTTAGG GTTAACAAGG GGGCCTTATA AACACTATTG CTAATGCCCT 1920

CTTGAGGGTC CGCTTATCGG TAGCTACACA [?][?]CCCCTCTG ATTGACGTTG GTGTAGCCTC 1980

CCGTAGTCTT CCTGGGCCCC TGGGAGGTAC ATGTCCCCCA GCATTGGTGT AAGAGCTTCA 2040

GCCAAGAGTT ACACATAAAG GCAATGTTGT GTTGCAGTCC ACAGACTGCA AAGTCTGCTC 2100

CAGGATGAAA GCCACTCAGT GTTGGCAAAT GTGCACATCC ATTTATAAGG ATGTCAACTA 2160

CAGTCAGAGA ACCCCTTTGT GTTTGGTCCC CCCCCGTGTC ACATGTGGAA CAGGGCCCAG 2220

TTGGCAAGTT GTACCAACCA ACTGAAGGGA TTACATGCAC TGCCCCGCGT GAGCAATACA 2280

AAACAAAAGC GCTCCTCGTA CCAGCGAAGA AGGGGCAGAG ATGCCGTAGT CAGGTTTAGT 2340

TCGTCCGGCG GCGCCAGAAA TCCGCGCGGT GGTTTTTGGG GGTCGGGGGT GTTTGGCAGC 2400

CACAGACGCC CGGTGTTCGT GTCGCGCCAG TACATGCGGT CCATGCCCAG GCCATCCAAA 2460

AACCATGGGT CTGTCTGCTC AGTCCAGTCG TGGACCTGAC CCCACGCAAC GCCCAAAAGA 2520

ATAACCCCCA CGAACCATAA ACCATTCCCC ATGGGGGACC CCGTCCCTAA CCCACGGGGC 2580

CCGTGGCTAT GGCGGGCTTG CCGCCCCGAC GTTGGCTGCG AGCCCTGGGC CTTCACCCGA 2640

ACTTGGGGGT TGGGGTGGGG AAAAGGAAGA AACGCGGGCG TATTGGCCCC AATGGGGTCT 2700

CGGTGGGGTA TCGACAGAGT GCCAGCCCTG GGACCGAACC CCGCGTTTAT GAACAAACGA 2760

CCCAACACCC GTGCGTTTTA TTCTGTCTTT TTATTGCCGT CATAGCGCGG GTTCCTTCCG 2820

GTATTGTCTC CTTCCGTGTT T[?]AGTTAGCC TCCCCCATCT CCCGATCCCT ATTCCTTTGC 2880

CCTCGGACGA GTGCTGGGGC GTCGGTTTCC ACTATCGGCG AGTACTTCTA CACAGCCATC 2940

GGTCCAGACG GCCGCGCTTC TGCGGGCGAT TTGTGTACGC CCGACAGTCC CGGCTCCGGA 3000

TCGGACGATT GCGTCGCATC GACCCTGCGC CCAAGCTGCA TCATCGAAAT TGCCGTCAAC 3060

CAAGCTCTGA TAGAGTTGGT CAAGACCAAT GCGGAGCATA TACGCCCGGA GCCGCGGCGA 3120

TCCTGCAAGC TCCGGATGCC TCCGCTCGAA GTAGCGCGTC TGCTGCTCCA TACAAGCCAA 3180

CCACGGCCTC CAGAAGAAGA TGTTGGCGAC CTCGTATTGG GAATCCCCGA ACATCGCCTC 3240

GCTCCAGTCA ATGACCGCTG TTATGCGGCC ATTGTCCGTC AGGACATTGT TGGAGCCGAA 3300

ATCCGCGTGC ACGAGGTGCC GGACTTCGGG GCAGTCCTCG GCCCAAAGCA TCAGCTCATC 3360

GAGAGCCTGC GCGACGGACG CACTGACGGT GTCGTCCATC ACAGTTTGCC AGTGATACAC 3420

ATGGGGATCA GCAATCGCGC ATATGAAATC ACGCCATGTA GTGTATTGAC CGATTCCTTG 3480

CGGTCCGAAT GGGCCGAACC CGCTCGTCTG GCTAAGATCG GCCGCAGCGA TCGCATCCAT 3540

GGCCTCCGCG ACCGGCTGCA GAACAGCGGG CAGTTCGGTT TCAGGCAGGT CTTGCAACGT 3600

GACACCCTGT GCACGGCGGG AGATGCAATA GGTCAGGCTC TCGCTGAATT CCCCAATGTC 3660

AAGCACTTCC GGAATCGGGA GCGCGGCCGA TGCAAAGTGC CGATAAACAT AACGATCTTT 3720

GTAGAAACCA TCGGCGCAGC TATTTACCCG CAGGACATAT CCACGCCCTC CTACATCGAA 3780

GCTGAAAGCA CGAGATTCTT CGCCCTCCGA GAGCTGCATC AGGTCGGAGA CGCTGTCGAA 3840

CTTTTCGATC AGAAACTTCT CGACAGACGT CGCGGTGAGT TCAGGCTTTT TCATATCTCA 3900

TTGCCCCCGG ACGAGGATCT GCGGCACGCT GTTGACGCTG TTAAGCGGGT CGCTGCAGGG 3960

TCGCTCGGTG TTCGAGGCCA CACGCGTCAC CTTAATATGC GAAGTGGACC TGGGACCGCG 4020

CCGCCCCGAC TGCATCTGCG TGTTCGAATT CGCCAATGAC AAGACGCTGG GCGGGGTTTG 4080

TGTCATCATA GAACTAAAGA CATGCAAATA TATTTCTTCC GGGGACACCG CCAGCAAACG 4140

CGAGCAACGG GCCACGGGGA TGAAGCAGGG CGGCACCTCG CTAACGGATT CACCACTCCA 4200

AGAATTGGAG CCAATCAATT CTTGCGGAGA ACTGTGAATG CGCAAACCAA CCCTTGGCAG 4260

AACATATCCA TCGCGTCCGC CATCTCCAGC AGCCGCACGC GGCGCATCTC GGGCAGCGTT 4320

GGGTCCTGGC CACGGGTGCG CATGATCGTG CTCCTGTCGT TGAGGACCCG GCTAGGCTGG 4380

CGGGGTTGCC TTACTGGTTA GCAGAATGAA TCACCGATAC GCGAGCGAAC GTGAAGCGAC 4440

TGCTGCTGCA AAACGTCTGC GACCTGAGCA ACAACATGAA TGGTCTTCGG TTTCCGTGTT 4500

TCGTAAAGTC TGGAAACGCG GAAGTCAGCG CTCTTCCGCT TCCTCGCTCA CTGACTCGCT 4560

GCGCTCGGTC GTTCGGCTGC GGCGAGCGGT ATCAGCTCAC TCAAAGGCGG TAATACGGTT 4620

ATCCACAGAA TCAGGGGATA ACGCAGGAAA GAACATGTGA GCAAAAGGCC AGCAAAAGGC 4680

CAGGAACCGT AAAAAGGCCG CGTTGCTGGC GTTTTTCCAT AGGCTCCGCC CCCCTGACGA 4740

GCATCACAAA AATCGACGCT CAAGTCAGAG GTGGCGAAAC CCGACAGGAC TATAAAGATA 4800

CCAGGCGTTT CCCCCTGGAA GCTCCCTCGT GCGCTCTCCT GTTCCGACCC TGCCGCTTAC 4860

CGGATACCTG TCCGCCTTTC TCCCTTCGGG AAGCGTGGCG CTTTCTCATA GCTCACGCTG 4920

TAGGTATCTC AGTTCGGTGT AGGTCGTTCG CTCCAAGCTG GGCTGTGTGC ACGAACCCCC 4980

CGTTCAGCCC GACCGCTGCG CCTTATCCGG TAACTATCGT CTTGAGTCCA ACCCGGTAAG 5040

ACACGACTTA TCGCCACTGG CAGCAGCCAC TGGTAACAGG ATTAGCAGAG CGAGGTATGT 5100

AGGCGGTGCT ACAGAGTTCT TGAAGTGGTG GCCTAACTAC GGCTACACTA GAAGGACAGT 5160

ATTTGGTATC TGCGCTCTGC TGAAGCCAGT TACCTTCGGA AAAAGAGTTG GTAGCTCTTG 5220

ATCCGGCAAA CAAACCACCG CTGGTAGCGG TGGTTTTTTT GTTTGCAAGC AGCAGATTAC 5280

GCGCAGAAAA AAAGGATCTC AAGAAGATCC TTTGATCTTT TCTACGGGGT CTGACGCTCA 5340

GTGGAACGAA AACTCACGTT AAGGGATTTT GGTCATGAGA TTATCAAAAA GGATCTTCAC 5400

CTA[?][?] [?]CTT TTAAATTAAA AATGAAGTTT TAAATCAATC TAAAGTATAT ATGAGTAAAC 5460

TTGGTCTGAC AGTTACCAAT GCTTAATCAG TGAGGCACCT ATCTCAGCGA TCTGTCTATT 5520

TCGTTCATCC ATAGTTGCCT GACTCCCCGT CGTGTAGATA ACTACGATAC GGGAGGGCTT 5580

ACCATCTGGC CCCAGTGCTG CAATGATACC GCGAGACCCA CGCTCACCGG CTCCAGATTT 5640

ATCAGCAATA AACCAGCCAG CCGGAAGGGC CGAGCGCAGA AGTGGTCCTG CAACTTTATC 5700

CGCCTCCATC CAGTCTATTA ATTGTTGCCG GGAAGCTAGA GTAAGTAGTT CGCCAGTTAA 5760

TAGTTTGCGC AACGTTGTTG CCATTGCTGC AGGCATCGTG GTGTCACGCT CGTCGTTTGG 5820

TATGGCTTCA TTCAGCTCCG GTTCCCAACG ATCAAGGCGA GTTACATGAT CCCCCATGTT 5880

GTGCAAAAAA GCGGTTAGCT CCTTCGGTCC TCCGATCGTT GTCAGAAGTA AGTTGGCCGC 5940

AGTGTTATCA CTCATGGTTA TGGCAGCACT GCATAATTCT CTTACTGTCA TGCCATCCGT 6000

AAGATGCTTT TCTGTGACTG GTGAGTACTC AACCAAGTCA TTCTGAGAAT AGTGTATGCG 6060

GCGACCGAGT TGCTCTTGCC CGGCGTCAAC ACGGGATAAT ACCGCGCCAC ATAGCAGAAC 6120

TTTAAAAGTG CTCATCATTG GAAAACGTTC TTCGGGGCGA AAACTCTCAA GGATCTTACC 6180

GCTGTTGAGA TCCAGTTCGA TGTAACCCAC TCGTGCACCC AACTGATCTT CAGCATCTTT 6240

TACTTTCACC AGCGTTTCTG GGTGAGCAAA AACAGGAAGG CAAAATGCCG CAAAAAAGGG 6300

AATAAGGGCG ACACGGAAAT GTTGAATACT CATACTCTTC CTTTTTCAAT ATTATTGAAG 6360

CATTTATCAG GGTTATTGTC TCATGAGCGG ATACATATTT GAATGTATTT AGAAAAATAA 6420

ACAAATAGGG GTTCCGCGCA CATTTCCCCG AAAAGTGCCA CCTGACGTCT AAGAAACCAT 6480

TATTATCATG ACATTAACCT ATAAAAATAG GCGTATCACG AGGCCCTTTC GTCTTCAAGA 6540

ATTCTCATGT TTGACAGCTT ATCGATCCGG CCAACGGTGT TGCCATTGCT GCAGGCGCAG 6600

AGCTGGTAGG TATGGAAGAT CTATACAGTG 6630

## Patentansprüche

1. Verfahren zur Bestimmung der modulierenden Wirkung einer Substanz auf einen rezeptorabhängigen Signalübertragungsweg in der menschlichen oder tierischen Zelle, dadurch gekennzeichnet, daß man die modulierende Wirkung der Substanz auf die Aktivität einer Phospholipase oder auf einen im Signalübertragungsweg der Phospholipaseaktivierung vorgeschalteten oder nachgeschalteten Mechanismus, ausgelöst durch einen an den Signalübertragungsweg gekoppelten Rezeptor, bestimmt, indem man Säugetierzellen, die
a) transformiert sind mit einer rekombinanten DNA, enthaltend ein Reportergen und eine regulatorische Sequenz, die auf die durch die Modulation der Phospholipase-Aktivität hervorgerufene Konzentrationsänderung von Inositol-1,4,5-Triphosphat (IP₃) und eines Diacylglycerins (DAG) anspricht, so daß die Expression des Reportergens durch eine Konzentrationsänderung von IP₃/DAG moduliert wird, und die weiters
b) transformiert sind mit einer rekombinanten DNA, enthaltend eine für einen Rezeptor, der mit dem Phospholipase-Effektorsystem gekoppelt ist, kodierende Sequenz derart, daß die Zellen den Rezeptor exprimieren,
mit der zu untersuchenden Substanz inkubiert und die Konzentration des Reportergenproduktes mißt.

2. Verfahren nach Anspruch 1, dadurch gekennzeichnet, daß die in b) definierte rekombinante DNA eine Sequenz enthält, die für einen humanen Rezeptor kodiert.

3. Verfahren nach Anspruch 1 oder 2, dadurch gekennzeichnet, daß die in a) definierte rekombinante DNA eine regulatorische Sequenz enthält, die auf die durch Modulation von Phospholipase C hervorgerufene Konzentrationsänderung von IP₃ und DAG anspricht, und daß die in b) definierte rekombinante DNA eine Sequenz enthält, die für einen G-Protein gekoppelten Rezeptor kodiert.

4. Verfahren nach einem der Ansprüche 1 bis 3, dadurch gekennzeichnet, daß man außerdem Säugetierzellen, die nur mit der in a) definierten rekombinanten DNA transformiert sind, unter identischen Bedingungen mit der zu untersuchenden Substanz inkubiert und die Konzentration des Reportergenproduktes mißt.

5. Verfahren nach einem der Ansprüche 1 bis 4, dadurch gekennzeichnet, daß man außerdem Säugetierzellen, die transformiert sind mit rekombinanter DNA, enthaltend das Reportergen und eine regulatorische Sequenz, die auf die durch die Modulation von Adenylatzyklase hervorgerufene Konzentrationsänderung von cAMP anspricht, so daß die Expression des Reportergens durch eine Konzentrationsänderung von cAMP moduliert wird, mit der zu untersuchenden Substanz inkubiert und die Konzentration des Reportergenproduktes mißt.

6. Verfahren nach Anspruch 5, dadurch gekennzeichnet, daß man außerdem Zellen, die mit einer auf cAMP ansprechenden rekombinanten DNA und mit einer in b) definierten rekombinanten DNA, enthaltend die für denselben Rezeptor kodierende Sequenz wie die Zellen, die mit der auf IP₃/DAG ansprechender rekombinanter DNA transformiert sind, mit der zu untersuchenden Substanz inkubiert und die Konzentration des Reportergenproduktes mißt.

7. Verfahren nach Anspruch 5, dadurch gekennzeichnet, daß man außerdem Zellen, die transformiert sind mit einer rekombinanten DNA, enthaltend eine für einen Rezeptor, der mit dem Adenylatzyklase-Effektorsystem gekoppelt ist, kodierende Sequenz derart, daß die Zellen den Rezeptor exprimieren, mit der zu untersuchenden Substanz inkubiert und die Konzentration des Reportergenproduktes mißt.

8. Verfahren nach einem der Ansprüche 1 bis 7, dadurch gekennzeichnet, daß es als Screening-Assay eingesetzt wird, in dem die zu untersuchende Substanz eine von zahlreichen Substanzen ist, mit denen eine vorbestimmte Zahl von Säugetierzellen unter vorbestimmten Bedingungen inkubiert und die Konzentration des Reportergenproduktes gemessen wird.

9. Verfahren nach Anspruch 8, dadurch gekennzeichnet, daß man als Reportergenprodukt Luciferase in Gegenwart eines Reagens mißt, das ein zur Lyse der Zellen geeignetes Detergens, einen Puffer mit pH-Wert 6 bis 9, vorzugsweise pH 7 bis 8, ein Magnesiumsalz, vorzugsweise Magnesiumsulfat, Adenosintriphosphat, Luciferin, ein mildes organisches Reduktionsmittel wie Dithiothreitol und/oder β-Mercaptoethanol sowie gegebenenfalls Natriumtripolyphosphat und/oder Natriumpyrophosphat enthält.

10. Rekombinante DNA, enthaltend ein Reportergen und eine damit funktionell verbundene Expressionskontrollsequenz für die Expression in Säugetierzellen, dadurch gekennzeichnet, daß die Expressionskontrollsequenz eine regulatorische Sequenz enthält, die auf die durch die Modulation von Phospholipase hervorgerufene Konzentrationsänderung von IP₃ und DAG anspricht, zur Verwendung in einem Verfahren nach einem der vorhergehenden Ansprüche.

11. Rekombinante DNA nach Anspruch 10, dadurch gekennzeichnet, daß sie eine regulatorische Sequenz natürlichen Ursprungs enthält.

12. Rekombinante DNA nach Anspruch 11, dadurch gekennzeichnet, daß sie die 5'-regulatorische Sequenz eines durch IP₃/DAG induzierbaren Gens enthält.

13. Rekombinante DNA nach Anspruch 12, dadurch gekennzeichnet, daß sie die 5'-regulatorische Sequenz des ICAM-1-Gens enthält.

14. Rekombinante DNA nach Anspruch 10, dadurch gekennzeichnet, daß die regulatorische Sequenz synthetisch hergestellt ist.

15. Rekombinante DNA nach Anspruch 14, dadurch gekennzeichnet, daß die regulatorische Sequenz mehrere auf die Modulation von IP₃/DAG ansprechende regulatorische Elemente in Abstand zueinander enthält.

16. Rekombinante DNA nach Anspruch 15, dadurch gekennzeichnet, daß sie drei bis zwölf regulatorische Elemente enthält.

17. Rekombinante DNA nach Anspruch 15 oder 16, dadurch gekennzeichnet, daß zumindest ein Teil der regulatorischen Elemente und/oder der zwischen ihnen gelegenen Sequenzabschnitte sich voneinander unterscheidet.

18. Rekombinante DNA nach Anspruch 16 oder 17, dadurch gekennzeichnet, daß sie drei TRE-Elemente des ICAM-1-Gens enthält.

19. Rekombinante DNA nach Anspruch 16 oder 17, dadurch gekennzeichnet, daß sie sechs TRE-Elemente des ICAM-1-Gens enthält.

20. Rekombinante DNA nach einem der Ansprüche 10 bis 19, dadurch gekennzeichnet, daß sie als Reportergen ein Luciferasegen enthält.

21. Rekombinante DNA nach einem der Ansprüche 10 bis 20, dadurch gekennzeichnet, daß sie ein Markergen enthält.

22. Säugetierzellen, transformiert mit einer rekombinanten DNA gemäß einem der Ansprüche 10 bis 21.

23. Säugetierzellen nach Anspruch 22, dadurch gekennzeichnet, daß sie außerdem transformiert sind mit einer rekombinanten DNA, enthaltend eine für einen Rezeptor, der mit dem Phosopholipase-Effektorsystem gekoppelt ist, kodierende Sequenz derart, daß die Zellen den Rezeptor exprimieren.

24. Säugetierzellen nach Anspruch 23, dadurch gekennzeichnet, daß die rekombinante DNA eine für einen humanen Rezeptor kodierende Sequenz enthält.

25. Säugetierzellen nach Anspruch 23 oder 24, dadurch gekennzeichnet, daß die rekombinante DNA eine für einen G-Protein gekoppelten Rezeptor kodierende Sequenz enthält.

26. Säugetierzellen nach Anspruch 25, dadurch gekennzeichnet, daß die Expressionskontrollsequenz eine regulatorische Sequenz enthält, die auf die durch die Modulation von Phospholipase C hervorgerufene Konzentrationsänderung von IP₃ und DAG anspricht.

27. Säugetierzellen nach Anspruch 26, dadurch gekennzeichnet, daß sie transformiert sind mit rekombinanter DNA, enthaltend eine Sequenz, kodierend für einen Rezeptor, ausgewählt aus der Gruppe Serotonin-Rezeptoren vom 5-HT_{1C}- und vom 5-HT₂-Typ, Thrombin-Rezeptor, Neuropeptid Y-Rezeptoren, Neurokinin-Rezeptoren, PAF-Rezeptoren, Angiotensin-Rezeptoren, muskarinische Acetylcholin-Rezeptoren vom M₁-, M₃- und M₅-Typ.

28. Rekombinante DNA, enthaltend ein Reportergen und eine damit funktionell verbundene Expressionskontrollsequenz für die Expression in Säugetierzellen, wobei die Expressionskontrollsequenz eine synthetisch hergestellte regulatorische Sequenz enthält, die auf die durch Modulation von Adenylatzyklase hervorgerufene Konzentrationsänderung von cAMP anspricht, dadurch gekennzeichnet, daß die regulatorische Sequenz mehrere auf die Modulation von cAMP ansprechende regulatorische Elemente in Abstand zueinander enthält, zur Verwendung in einem Verfahren gemäß einem der Ansprüche 5 bis 9.

29. Rekombinante DNA nach Anspruch 28, dadurch gekennzeichnet, daß sie drei bis zwölf regulatorische Elemente enthält.

30. Rekombinante DNA nach Anspruch 28 oder 29, dadurch gekennzeichnet, daß zumindest ein Teil der regulatorischen Elemente und/oder der zwischen ihnen gelegenen Sequenzabschnitte sich voneinander unterscheidet.

31. Rekombinante DNA nach einem der Ansprüche 28 bis 30, dadurch gekennzeichnet, daß sie als Reportergen ein Luciferasegen enthält.

32. Rekombinante DNA nach einem der Ansprüche 28 bis 31, dadurch gekennzeichnet, daß sie ein Markergen enthält.

33. Säugetierzellen, transformiert mit einer rekombinanten DNA nach einem der Ansprüche 28 bis 32.

34. Säugetierzellen nach Anspruch 33, dadurch gekennzeichnet, daß sie außerdem transformiert sind mit einer rekombinanten DNA, enthaltend eine für einen Rezeptor, der mit dem Phospholipase-Effektorsystem gekoppelt ist, kodierende Sequenz derart, daß die Zellen den Rezeptor exprimieren.

35. Säugetierzellen nach Anspruch 34, dadurch gekennzeichnet, daß sie transformiert sind mit rekombinanter DNA, enthaltend eine Sequenz, kodierend für einen Rezeptor, ausgewählt aus der Gruppe Serotonin-Rezeptoren vom 5-HT_{1C}- und vom 5-HT₂-Typ, Thrombin-Rezeptor, Neuropeptid Y-Rezeptoren, Neurokinin-Rezeptoren, PAF-Rezeptoren, Angiotensin-Rezeptoren, muskarinische Acetylcholin-Rezeptoren vom M₁-, M₃- und M₅-Typ.

36. Säugetierzellen nach Anspruch 33, dadurch gekennzeichnet, daß sie außerdem transformiert sind mit einer rekombinanten DNA, enthaltend eine für einen Rezeptor, der mit dem Adenylatzyklase-Effektorsystem gekoppelt ist, kodierende Sequenz derart, daß die Zellen den Rezeptor exprimieren.

37. Säugetierzellen nach Anspruch 36, dadurch gekennzeichnet, daß sie transformiert sind mit rekombinanter DNA, enthaltend eine Sequenz, kodierend für einen Rezeptor, ausgewählt aus der Gruppe muskarinischer Acetylcholin-Rezeptoren vom M₂- und M₄-Typ, Dopamin-Rezeptoren vom D₁-, D₂₁-, D₂ₛ- und D₅-Typ, Serotonin-Rezeptoren vom 5-HT_{1A}- und 5-BT_{1D}-Typ, Adenosin-Rezeptoren vom A₁- und A₂-Typ.

## Claims

1. Process for determining the modulating effect of a substance on a receptor-dependent signal transduction pathway in a human or animal cell, characterised in that the modulating effect of the substance on the activity of a phospholipase or on a mechanism which precedes or succeeds the phospholipase activation in the signal transduction pathway initiated by a receptor coupled to the signal transduction pathway, is determined by incubating mammalian cells which
a) are transformed with a recombinant DNA containing a reporter gene and a regulatory sequence which responds to the change in concentration of inositol-1,4,5-triphosphate (IP₃) and a diacylglycerol (DAG) brought about by the modulation of the phospholipase activity, so that the expression of the reporter gene is modulated by a change in concentration of IP₃/DAG, and which are furthermore
b) transformed with a recombinant DNA, containing a sequence which codes for a receptor which is coupled to the phospholipase effector system so that the cells express the receptor,
with the test substance and measuring the concentration of the reporter gene product.

2. Process according to claim 1, characterised in that the recombinant DNA defined in b) contains a sequence which codes for a human receptor.

3. Process according to claim 1 or 2, characterised in that the recombinant DNA defined in a) contains a regulatory sequence which responds to the change in concentration of IP₃ and DAG brought about by modulation of phospholipase C, and that the recombinant DNA defined in b) contains a sequence which codes for a G-protein coupled receptor.

4. Process according to one of claims 1 to 3, characterised in that, furthermore, mammalian cells which are transformed only with the recombinant DNA defined in a) are incubated with the test substance under identical conditions and the concentration of the reporter gene product is measured.

5. Process according to one of claims 1 to 4, characterised in that, moreover, mammalian cells which are transformed with recombinant DNA containing the reporter gene and a regulatory sequence which responds to the change in concentration of cAMP brought about by the modulation of adenylate cyclase so that the expression of the reporter gene is modulated by the change in concentration of cAMP are incubated with the test substance and the concentration of the reporter gene product is measured.

6. Process according to claim 5, characterised in that, moreover, cells which are transformed with the recombinant DNA responding to cAMP and with a recombinant DNA defined in b), containing the sequence coding for the same receptor as the cells which are transformed with recombinant DNA responding to IP₃/DAG, are incubated with the test substance and the concentration of the reporter gene product is measured.

7. Process according to claim 5, characterised in that, moreover, cells which are transformed with a recombinant DNA, containing a sequence coding for a receptor which is coupled to the adenylate cyclase effector system in such a way that the cells express the receptor, are incubated with the test substance and the concentration of the reporter gene product is measured.

8. Process according to one of claims 1 to 7, characterised in that it is used as a screening assay in which the test substance is one of a number of substances with which a predetermined number of mammalian cells are incubated under predetermined conditions and the concentration of the reporter gene product is measured.

9. Process according to claim 8, characterised in that, as the reporter gene product, luciferase is measured in the presence of a reagent which contains a detergent suitable for lysing the cells, a buffer having a pH from 6 to 9, preferably pH 7 to 8, a magnesium salt, preferably magnesium sulphate, adenosine triphosphate, luciferin, a mild organic reducing agent such as dithiothreitol and/or β-mercaptoethanol and optionally sodium tripolyphosphate and/or sodium pyrophosphate.

10. Recombinant DNA containing a reporter gene and an expression control sequence functionally connected therewith for the expression in mammalian cells, characterised in that the expression control sequence contains a regulatory sequence which responds to the change in concentration of IP₃ and DAG brought about by the modulation of phospholipase, for use in a process according to one of the preceding claims.

11. Recombinant DNA according to claim 10, characterised in that it contains a regulatory sequence of natural origin.

12. Recombinant DNA according to claim 11, characterised in that it contains the 5'-regulatory sequence of a gene which can be induced by IP₃/DAG.

13. Recombinant DNA according to claim 12, characterised in that it contains the 5'-regulatory sequence of the ICAM-1 gene.

14. Recombinant DNA according to claim 10, characterised in that the regulatory sequence is synthetically produced.

15. Recombinant DNA according to claim 14, characterised in that the regulatory sequence contains a plurality of regulatory elements responding to the modulation of IP₃/DAG spaced from one another.

16. Recombinant DNA according to claim 15, characterised in that it contains three to twelve regulatory elements.

17. Recombinant DNA according to claim 15 or 16, characterised in that at least some of the regulatory elements and/or the sequence sections located between them differ from one another.

18. Recombinant DNA according to claim 16 or 17, characterised in that it contains three TRE-elements of the ICAM-1 gene.

19. Recombinant DNA according to claim 16 or 17, characterised in that it contains six TRE-elements of the ICAM-1 gene.

20. Recombinant DNA according to one of claims 10 to 19, characterised in that it contains a luciferase gene as reporter gene.

21. Recombinant DNA according to one of claims 10 to 20, characterised in that it contains a marker gene.

22. Mammalian cells transformed with a recombinant DNA according to one of claims 10 to 21.

23. Mammalian cells according to claim 22, characterised in that they are moreover transformed with a recombinant DNA containing a sequence coding for a receptor which is coupled to the phospholipase effector system in such a way that the cells express the receptor.

24. Mammalian cells according to claim 23, characterised in that the recombinant DNA contains a sequence coding for a human receptor.

25. Mammalian cells according to claim 23 or 24, characterised in that the recombinant DNA contains a sequence coding for a G-protein-coupled receptor.

26. Mammalian cells according to claim 25, characterised in that the expression control sequence contains a regulatory sequence which responds to the change in concentration of IP₃ and DAG caused by the modulation of phospholipase C.

27. Mammalian cells according to claim 26, characterised in that they are transformed with recombinant DNA containing a sequence coding for a receptor, selected from the group comprising serotonin receptors of the 5-HT_{1C}- and 5-HT₂-type, thrombin receptor, neuropeptide Y-receptors, neurokinin receptors, PAF-receptors, angiotensin receptors, muscarinic acetylcholine receptors of the M₁-, M₃- and M₅-type.

28. Recombinant DNA, containing a reporter gene and an expression control sequence functionally connected therewith for expression in mammalian cells, wherein the expression control sequence contains a synthetically produced regulatory sequence which responds to the change in concentration of cAMP brought about by modulation of adenylate cyclase, characterised in that the regulatory sequence contains a plurality of regulatory elements responding to the modulation of cAMP, spaced apart from one another, for use in a process according to one of claims 5 to 9.

29. Recombinant DNA according to claim 28, characterised in that it contains three to twelve regulatory elements.

30. Recombinant DNA according to claim 28 or 29, characterised in that at least some of the regulatory elements and/or the sequence sections located between them differ from one another.

31. Recombinant DNA according to one of claims 28 to 30, characterised in that it contains a luciferase gene as the reporter gene.

32. Recombinant DNA according to one of claims 28 to 31, characterised in that it contains a marker gene.

33. Mammalian cells transformed with a recombinant DNA according to one of claims 28 to 32.

34. Mammalian cells according to claim 33, characterised in that they are furthermore transformed with a recombinant DNA containing a sequence coding for a receptor which is coupled to the phospholipase effector system, so that the cells express the receptor.

35. Mammalian cells according to claim 34, characterised in that they are transformed with recombinant DNA containing a sequence coding for a receptor selected from the group comprising serotonin receptors of the 5-HT_{1C}- and 5-HT₂-type, thrombin receptor, neuropeptide Y-receptors, neurokinin receptors, PAF-receptors, angiotensin receptors and muscarinic acetylcholine receptors of the M₁-, M₃- and M₅-type.

36. Mammalian cells according to claim 33, characterised in that they are also transformed with a recombinant DNA containing a sequence coding for a receptor which is coupled to the adenylate cyclase effector system, in such a way that the cells express the receptor.

37. Mammalian cells according to claim 36, characterised in that they are transformed with recombinant DNA containing a sequence coding for a receptor, selected from the group comprising muscarinic acetylcholine receptors of the M₂- and M₄-type, dopamine receptors of the D₁-, D₂₁-, D₂ₛ- and D₅-type, serotonin receptors of the 5-HT_{1A}- and 5-HT_{1D}-type and adenosine receptors of the A₁- and A₂-type.

## Revendications

1. Procédé de détermination de l'effet modulateur d'une substance sur une voie de transmission de signaux dépendant d'un récepteur dans la cellule humaine ou animale, caractérisé en ce que l'on détermine l'effet modulateur de la substance sur l'activité d'une phospholipase ou sur un mécanisme précédant ou suivant l'activation d'une phospholipase dans la voie de transmission de signaux, déclenché par un récepteur couplé à la voie de transmission de signaux, en incubant avec la substance à étudier des cellules de mammifère qui
a) sont transformées avec un ADN recombiné contenant un gène reporter et une séquence régulatrice qui réagit à la variation de concentration de l'inositol-1,4,5-triphosphate (IP₃) et d'une diacylglycérine (DAG) qui est provoquée par la modulation de l'activité de phospholipase, de sorte que l'expression du gène reporter est modulée par une variation de concentration de IP₃/DAG et qui, en outre,
b) sont transformées avec un ADN recombiné contenant une séquence codant un récepteur qui est couplé au système effecteur de phospholipase, de sorte que les cellules expriment le récepteur,
et en mesurant la concentration du produit du gène reporter.

2. Procédé selon la revendication 1 caractérisé en ce que l'ADN recombiné défini en b) contient une séquence qui code un récepteur humain.

3. Procédé selon la revendication 1 ou 2 caractérisé en ce que l'ADN recombiné défini en a) contient une séquence régulatrice qui réagit à la variation de concentration de IP₃ et DAG provoquée par la modulation de la phospholipase C, et en ce que l'ADN recombiné défini en b) contient une séquence qui code un récepteur couplé à une protéine G.

4. Procédé selon l'une des revendications 1 à 3 caractérisé en ce que l'on incube en outre avec la substance à étudier des cellules de mammifère qui ne sont transformées qu'avec l'ADN recombiné défini en a) dans des conditions identiques et on mesure la concentration du produit du gène reporter.

5. Procédé selon l'une des revendications 1 à 4 caractérisé en ce que l'on incube en outre avec la substance à étudier des cellules de mammifère qui sont transformées avec un ADN recombiné contenant le gène reporter et une séquence régulatrice qui réagit à la variation de concentration de AMPc provoquée par la modulation de l'adénylate cyclase, de sorte que l'expression du gène reporter est modulée par une variation de concentration de AMPc, et on mesure la concentration du produit du gène reporter.

6. Procédé selon la revendication 5 caractérisé en ce que l'on incube en outre avec la substance à étudier des cellules qui sont transformées avec un ADN recombiné réagissant à AMPc et avec un ADN recombiné défini en b) contenant la séquence codant le même récepteur que les cellules qui sont transformées avec l'ADN recombiné réagissant à IP₃/DAG, et on mesure la concentration du produit du gène reporter.

7. Procédé selon la revendication 5 caractérisé en ce que l'on incube en outre avec la substance à étudier des cellules qui sont transformées avec un ADN recombiné contenant une séquence codant un récepteur qui est couplé au système effecteur de l'adénylate cyclase de telle manière que les cellules expriment le récepteur, et on mesure la concentration du produit du gène reporter.

8. Procédé selon l'une des revendications 1 à 7 caractérisé en ce qu'il est utilisé comme test de triage par le fait que la substance à étudier est l'une des nombreuses substances avec lesquelles un nombre prédéterminé de cellules de mammifère sont incubées dans des conditions prédéterminées et la concentration du produit du gène reporter est mesurée.

9. Procédé selon la revendication 8 caractérisé en ce que l'on mesure comme produit du gène reporter la luciférase en présence d'un réactif qui contient un détergent approprié à la lyse des cellules, un tampon d'un pH de 6 à 9, de préférence d'un pH de 7 à 8, un sel de magnésium, de préférence le sulfate de magnésium, de l'adénosinetriphosphate, de la luciférine, un réducteur organique doux comme le dithiothréitol et/ou le β-mercaptoéthanol ainsi éventuellement que du tripolyphosphate de sodium et/ou du pyrophosphate de sodium.

10. ADN recombiné contenant un gène reporter et une séquence de contrôle de l'expression liée à lui de manière fonctionnelle pour l'expression dans des cellules de mammifère caractérisé en ce que la séquence de contrôle de l'expression contient une séquence régulatrice qui réagit à la variation de concentration de IP₃ et DAG provoquée par la modulation de phospholipase, destiné à être utilisé dans un procédé selon l'une des revendications précédentes.

11. ADN recombiné selon la revendication 10 caractérisé en ce qu'il contient une séquence régulatrice d'origine naturelle.

12. ADN recombiné selon la revendication 11 caractérisé en ce qu'il contient la séquence régulatrice en 5' d'un gène inductible par IP₃/DAG.

13. ADN recombiné selon la revendication 12 caractérisé en ce qu'il contient la séquence régulatrice en 5' du gène d'ICAM-1.

14. ADN recombiné selon la revendication 10 caractérisé en ce que la séquence régulatrice est préparée par synthèse.

15. ADN recombiné selon la revendication 14 caractérisé en ce que la séquence régulatrice contient à distance les uns des autres plusieurs éléments régulateurs réagissant à la modulation de IP₃/DAG.

16. ADN recombiné selon la revendication 15 caractérisé en ce qu'il contient trois à douze éléments régulateurs.

17. ADN recombiné selon la revendication 15 ou 16 caractérisé en ce qu'au moins une partie des éléments régulateurs et/ou des segments de séquence disposés entre eux diffèrent les uns des autres.

18. ADN recombiné selon la revendication 16 ou 17 caractérisé en ce qu'il contient trois éléments TRE du gène d'ICAM-1.

19. ADN recombiné selon la revendication 16 ou 17 caractérisé en ce qu'il contient six éléments TRE du gène d'ICAM-1.

20. ADN recombiné selon l'une des revendications 10 à 19 caractérisé en ce qu'il contient comme gène reporter un gène de luciférase.

21. ADN recombiné selon l'une des revendications 10 à 20 caractérisé en ce qu'il contient un gène marqueur.

22. Cellules de mammifère transformées avec un ADN recombiné selon l'une des revendications 10 à 21.

23. Cellules de mammifère selon la revendication 22 caractérisées en ce qu'elles sont transformées en outre avec un ADN recombiné contenant une séquence codant un récepteur qui est couplé au système effecteur de phospholipase de telle manière que les cellules expriment le récepteur.

24. Cellules de mammifère selon la revendication 23 caractérisées en ce que l'ADN recombiné contient une séquence codant un récepteur humain.

25. Cellules de mammifère selon la revendication 23 ou 24 caractérisées en ce que l'ADN recombiné contient une séquence codant un récepteur couplé à une protéine G.

26. Cellules de mammifère selon la revendication 25 caractérisées en ce que la séquence de contrôle de l'expression contient une séquence régulatrice qui réagit à la variation de concentration de IP₃ et DAG provoquée par la modulation de la phospholipase C.

27. Cellules de mammifère selon la revendication 26 caractérisées en ce qu'elles sont transformées avec un ADN recombiné contenant une séquence codant un récepteur choisi dans le groupe des récepteurs à la sérotonine du type 5-HT_{1C} et du type 5-HT₂, du récepteur à la thrombine, des récepteurs au neuropeptide Y, des récepteurs aux neurokinines, des récepteurs à PAF, des récepteurs à l'angiotensine, des récepteurs à l'acétylcholine muscariniques du type M₁, M₃ et M₅.

28. ADN recombiné contenant un gène reporter et une séquence de contrôle de l'expression liée à lui de manière fonctionnelle pour l'expression dans des cellules de mammifère, où la séquence de contrôle de l'expression contient une séquence régulatrice préparée par synthèse qui réagit à la variation de concentration de AMPc provoquée par la modulation de l'adénylate cyclase, caractérisé en ce que la séquence régulatrice contient à distance les uns des autres plusieurs éléments régulateurs réagissant à la modulation de AMPc, destiné à être utilisé dans un procédé selon l'une des revendications 5 à 9.

29. ADN recombiné selon la revendication 28 caractérisé en ce qu'il contient trois à douze éléments régulateurs.

30. ADN recombiné selon la revendication 28 ou 29 caractérisé en ce qu'au moins une partie des éléments régulateurs et/ou des segments de séquence disposés entre eux diffèrent les uns des autres.

31. ADN recombiné selon l'une des revendications 28 à 30 caractérisé en ce qu'il contient comme gène reporter un gène de luciférase.

32. ADN recombiné selon l'une des revendications 28 à 31 caractérisé en ce qu'il contient un gène marqueur.

33. Cellules de mammifère transformées avec un ADN recombiné selon l'une des revendications 28 à 32.

34. Cellules de mammifère selon la revendication 33 caractérisées en ce qu'elles sont transformées en outre avec un ADN recombiné contenant une séquence codant un récepteur qui est couplé au système effecteur de phospholipase de telle manière que les cellules expriment le récepteur.

35. Cellules de mammifère selon la revendication 34 caractérisées en ce qu'elles sont transformées avec un ADN recombiné contenant une séquence codant un récepteur choisi dans le groupe des récepteurs à la sérotonine du type 5-HT_{1C} et du type 5-HT₂, du récepteur à la thrombine, des récepteurs au neuropeptide Y, des récepteurs aux neurokinines, des récepteurs à PAF, des récepteurs à l'angiotensine, des récepteurs à l'acétylcholine muscariniques du type M₁, M₃ et M₅.

36. Cellules de mammifère selon la revendication 33 caractérisées en ce qu'elles sont transformées en outre avec un ADN recombiné contenant une séquence codant un récepteur qui est couplé au système effecteur de l'adénylate cyclase de telle manière que les cellules expriment le récepteur.

37. Cellules de mammifère selon la revendication 36 caractérisées en ce qu'elles sont transformées avec un ADN recombiné contenant une séquence codant un récepteur choisi dans le groupe des récepteurs à l'acétylcholine muscariniques du type M₂ et M₄, des récepteurs à la dopamine du type D₁, D₂₁, D₂ₛ et D₅, des récepteurs à la sérotonine du type 5-HT_{1A} et 5-HT_{1D}, des récepteurs à l'adénosine du type A₁ et A₂.
